# EUROPEAN PATENT APPLICATION

(11) **EP 4 095 162 A1**
(43) Date of publication of application: **30.11.2022**
(21) Application number: 20915498.8
(22) Date of filing: 23.12.2020
(51) Int. Cl.: C08B 37/08, A61K 47/36

(54) **APPLICATION OF AND PREPARATION METHOD FOR CATIONIC POLYMER MODIFIED BY FLUORINE-CONTAINING COMPOUND AS DRUG CARRIER**

(30) Priority: 20.01.2020 CN 202010061971
(71) Applicant: Soochow University, Industrial Park Suzhou Jiangsu 215000 (CN)
(72) Inventor: LIU, Zhuang, Suzhou, Jiangsu 215000 (CN); CHEN, Qian, Suzhou, Jiangsu 215000 (CN); JIN, Qiutong, Suzhou, Jiangsu 215000 (CN); ZHAO, Qi, Suzhou, Jiangsu 215000 (CN); XIAO, Zhisheng, Suzhou, Jiangsu 215000 (CN); WEI, Ting, Suzhou, Jiangsu 215000 (CN); SHEN, Jingjing, Suzhou, Jiangsu 215000 (CN)
(74) Representative: RGTH
(86) International application number: PCT/CN2020/138465
(87) International publication number: WO 2021/147598

(57) **Abstract**

The invention provides a fluorinated chitosan derivative as a drug carrier, which has the following structure: a fluorine-containing compound covalently attached to the backbone of chitosan. The chitosan has a molecular weight in the range of 1000-5000000, a degree of deacetylation of not less than 55%, and a viscosity in the range of 25-1000 cps. The fluorine-containing compound is a fluorine-containing aliphatic chain shown by the following chemical formula (I) 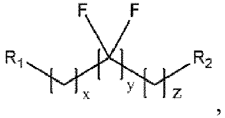 or an aromatic ring with functional groups shown by the following formula (II) 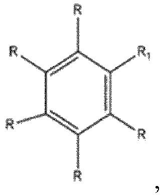 wherein Ri is halogen (fluorine, chlorine, bromine, iodine), or a halogen-substituted alkane, cycloalkane, aldehyde group, carboxyl group, alkenyl group, alkynyl group, hydroxyl group, sulfonyl chloride, sulfonic acid bond or mercapto group, for interacting with a primary amino group. The compounds of the present invention can be universally bound to a variety of drugs to promote drug absorption and bioavailability, and reduce toxicity.

## Description

### FIELD OF THE INVENTION

The present invention relates to the technical field of polymer chemistry and medical biomaterials, andmore particularly to a fluoride modification-based cationic polymer as a drug carrier and a preparation method and use thereof.

### DESCRIPTION OF THE RELATED ART

In recent years, hydrophilic cationic polymer materials such as polyethyleneimine (PEI), polylysine, due to their cationic properties, can combine with nucleic acids, polypeptides and protein molecules to form nanocomposites, which not only can promote the entry of these macromolecular compounds into cells, but also can protect the drugs from degradation by hydrolytic enzymes in a microenvironment. The internal tertiary amino structure of the hydrophilic cationic polymer materials promotes the escape of the drugs in the cell endosome through proton sponge effect. Also, the cationic polymer materials can weaken the tight junction of epithelial cells to increase the permeability of the epithelium, and thus improve the absorption efficiency of drug macromolecules in epithelial cells. However, the high cytotoxicity of the cationic polymer materials during use ultimately limits their clinical applications.

Chitosan is a cationic polysaccharide after deacetylation of chitin. It has good biosafety properties and excellent mucosal adhesion properties, and has been widely used in the design of transmucosal dosage forms. It is reported in the literature that chitosan can produce mucosal adhesion through the interaction of its own positive charges and negative charges on the skin and mucosal surface and the hydrophobic effect of hydrophobic groups, effectively extending the residence time of biologically active substances, such as drugs, polypeptides and proteins, in the chitosan solution at a lesion site. Driven by the later effects of diffusion or chitosan degradation, the active substances are allowed to be slowly released from the chitosan solution, so as to achieve a long-term sustained release effect on the local skin and mucous membranes. Although chitosan can significantly improve the bioavailability of perfusion drugs, high concentrations of chitosan may cause severe mucosal and epithelial damage, limiting its clinical application as a drug carrier.

Transdermal administration refers to an administration mode in which a drug penetrates the skin, is absorbed through the capillaries, and enters the blood circulation to achieve an effective blood drug concentration and then to take effect. Transdermal administration can avoid the liver first-pass effect of oral administration and the inactivation of the drug in the gastrointestinal tract. Especially for patients who need long-term administration, transdermal administration is a convenient and quick treatment mode. However, as the first barrier of the human body, the skin can prevent the intrusion of most foreign substances. The rate of drug penetration through the skin is often slow, and the penetration amount is difficult to reach a concentration required for effective treatment. As a result, the optimal therapeutic effect cannot be exerted. Also, the dosage of a pharmaceutical preparation for transdermal administration is usually related to an effective contact area between an administration system and the skin. The dosage could be increased by enlarging the contact area, but a general administration area is not greater than 60 cm², so the drug is required to have a certain rate of transdermal penetration. Except for several drugs with small dosage requirements and suitable dissolution characteristics, most drugs are difficult to meet the treatment requirements.

Currently commonly used clinical transdermal dosage forms include patches or gels containing chemical penetration-enhancing ingredients and patches or gels with physical penetration promotion. Since the launch of Transderm Scop, a transdermal patch for the treatment of motion sickness in 1981, patches and gels containing chemical penetration-enhancing ingredients have been widely used in the treatment of various diseases including dementia, Parkinson's disease and acute pain. So far, more than 20 transdermal patches have been approved by the US FDA for marketing. However, the existing technologies or products usually have one or more defects such as limited transdermal effect, low drug bioavailability, inability to universally combine well with a variety of drugs, and greater toxicity.

Seeking non-injection administration modes has always been a research hotspot in the fields of pharmaceutics, biochemistry, etc., which involves administration routes including oral administration, pulmonary administration, nasal administration, rectal administration, vaginal administration, etc. These non-injection administration modes have the advantages of convenient medication, reducing the pain of patients with medication, and improving patient compliance, and have become a hot topic in the study of topical preparations. However, in practical applications, there is still the problem of low delivery efficiency of macromolecular drugs. One of the important reasons is that various mucous membranes of human organs (including nasal mucosa, lung mucosa, vaginal mucosa, oral mucosa, gastrointestinal mucosa, etc.) are one of the barriers that various drugs are difficult to cross. Therefore, the development of transmucosal carriers to improve the delivery efficiency of drugs and realize the efficient transmucosal delivery of drugs in the body has very important scientific significance and huge practical application value.

Mucosa refers to a membranous structure lining the luminal surface of tubular or sac-like organs in living organisms (digestive, respiratory, urinary, reproductive, etc.). It is composed of epithelial tissue and loose connective tissue, and some organs also contain muscularis mucosa. The connective tissue part is called the lamina propria, and the epithelial tissue part is called the epithelium, which contains blood vessels and nerves and can secrete mucus. The normal mucosa can vary from light red to bright red in color due to the degree of blood filling, and is moist and somewhat stretchable, and folds are often formed in an empty state. It has the functions of protection, secretion and absorption, and the epithelium is the main part of tubular organs for functional activities. The type of epithelium varies with its location and function. There are different names depending on the location, such as nasal mucosa, lung mucosa, vaginal mucosa, oral mucosa, gastrointestinal mucosa, etc.

Although in recent years, a series of hydrophilic cationic polymer materials such as polyethyleneimine (PEI), polyamide-amine dendrimer, chitosan, β-cyclodextrin, gelatin, and cationic polypeptides/amino acids such as polylysine (PLL), cationic polyester, cationic polyphosphate, polyvinylpyridinium, poly(dimethylamino)ethyl methacrylate, can weaken the tight junction of epithelial cells to increase the permeability of the epithelium, thereby promoting the mucosal penetration of the drug, and improving the bioavailability of the drug. However, such a delivery system has poor biocompatibility and certain toxic and side effects on the body, and easily causes mucosal and epithelial damage at high concentrations, so that its clinical application is facing a bottleneck. Therefore, such cationic polymer materials still need to be further improved to achieve a better drug absorption promoting effect and lower toxicity.

Therefore, how to invent a novel drug carrier material that has obvious drug absorption promoting effect and low cytotoxicity is a challenging direction.

An objec of the present invention is to provide a novel drug carrier material with obvious drug absorption promoting effect and low toxicity. A fluorine-containing compound-modified chitosan proposed by the present invention has a mature synthesis process, simple operation, high synthesis efficiency, short cycle time, and a high yield without complicated purification steps, and this simple synthesis method provides a good basis for commercialization. The fluorine-containing compound-modified chitosan of the present invention is useful as a variety of drug carriers.

The present invention adopts the following technical solutions. A fluorinated chitosan derivative for use as a drug carrier, having the following structure: a fluorine-containing compound is covalently attached to the backbone of chitosan, wherein

the chitosan has a molecular weight in the range of 1000-5000000, a degree of deacetylation of not less than 55%, and a viscosity in the range of 25-1000 cps,

the fluorine-containing compound is a fluorine-containing aliphatic chain shown by the following chemical formula (I) 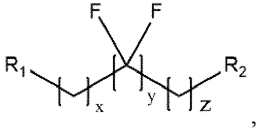 or an aromatic ring functional group shown by the following formula (II) 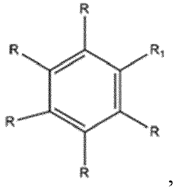 wherein R1 is an active group capable of reacting with a primary amino group, selected from halogen (fluorine, chlorine, bromine, iodine), or a halogen-substituted alkane, cycloalkane, aldehyde group, carboxyl group, double bond, alkyne bond, hydroxyl group, sulfonyl chloride, sulfonic acid bond or mercapto group.

Further, a fluorinated chitosan derivative for use as a drug carrier, having a molecular skeleton of chitosan which contains a primary amino group, as shown in formula (IV): 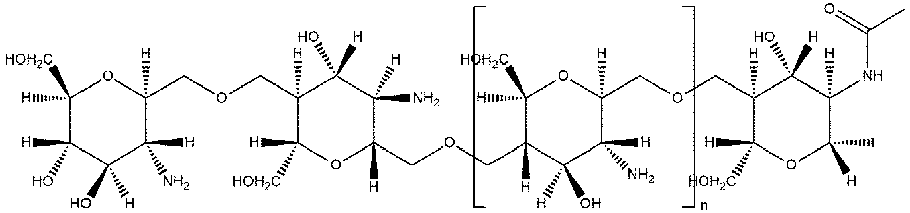
formula (IV);
wherein a linking group formed between the primary amino group of the chitosan and a fluorine-containing functional group is: -NH-, -N=C-, -NHCH₂CH(OH)-, -NHCH₂CH(OH)CH₂O-, 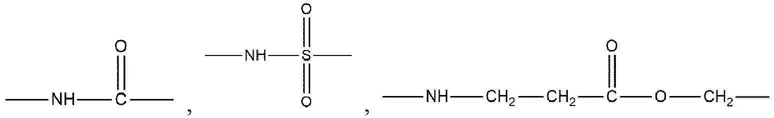 and a derivative group thereof; the chitosan has a molecular weight in the range of 1000-5000000, a degree of deacetylation of not less than 55%, and a viscosity in the range of 25-1000 cps,
the fluorine-containing functional group is a fluorine-containing aliphatic chain or an aromatic ring functional group.

Further, the fluorinated chitosan derivative for use as a drug carrier, in the formula (I), x is an integer of 0-3, y is an integer of 0-20, and z is an integer of 0-8, R₂ is CF₃ , CHF₂, CH₂F, or CH₃ (when y is not 0);
the fluorine-containing aliphatic chain compound refers to a fluorine-containing hydrocarbon compound and derivatives thereof, and includes trifluoroacetic acid, pentafluoropropionic acid, heptafluorobutyric acid, nonafluorovaleric acid, undecafluorohexanoic acid, tridecafluoroheptanoic acid, pentadecafluorooctanoic acid, heptadecafluorononanoic acid, nonadecafluorodecanoic acid, heptafluorobutyric anhydride, perfluoroheptanoic anhydride, nonadecafluorodecanoic anhydride, 2,2,3,3,4,4,4-heptafluorobutyl acrylate, 3-(1H, 1H, 5H octafluoropentyloxy)-1,2-epoxypropane, nonafluorobuanesulphonic anhydride and derivatives thereof..

Further, the fluorinated chitosan derivative for use as a drug carrier, in the formula (II), R is H, CH₃, OH, NO₂, O, CF₃, F, CH₂OH, CN, NCO, or (CF₂)ₐCF₃ (a is an integer of 1-20), or the like, and at least one R is F;
the fluorine-containing aromatic ring compound includes 3-fluorobenzoic acid, 3,5-difluorobenzoic acid, 2,3,5,6-tetrafluoro-4-methylbenzoic acid, pentafluorobenzoic acid, 2-fluoro-3-(trifluoromethyl)benzoic acid and derivatives thereof.

Further, the fluorinated chitosan derivative for use as a drug carrier, the chitosan and the fluorine-containing compound are covalently linked and the chitosan is surface-modified, to form a drug carrier having a structure as shown in formula (V) below, wherein b and c are both an integer of 20-500: 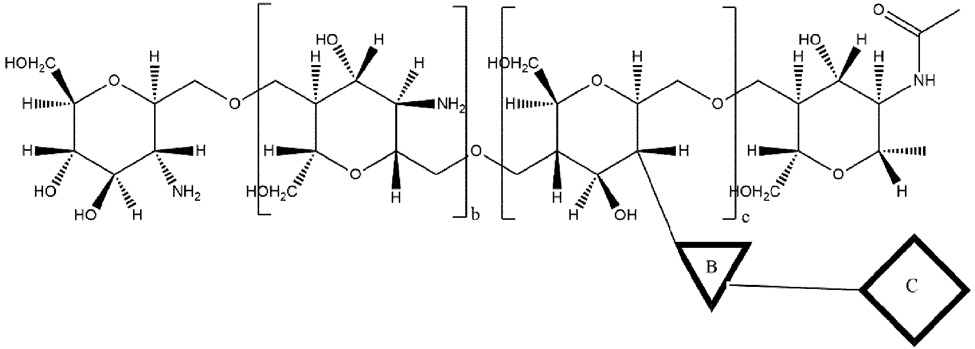 wherein B is a linking group formed by a fluorine-containing functional group and a primary amino group of the chitosan, and C is a fluorine-containing aliphatic chain or an aromatic ring functional group.

Further, the fluorinated chitosan derivative for use as a drug carrier, the fluorine-containing aliphatic chain is a class of fluorine-containing compounds with an active group capable of reacting with an amino group, and includes those as shown in formula (VI): 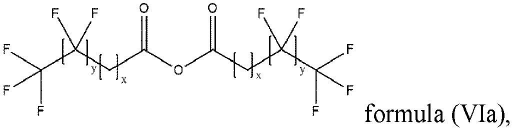 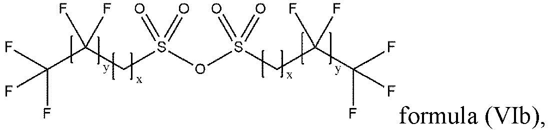 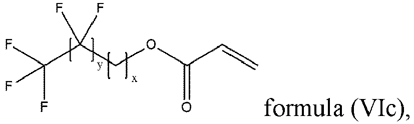 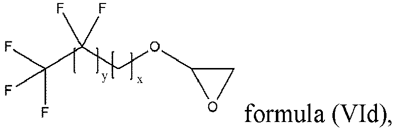 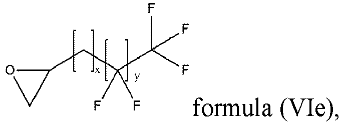 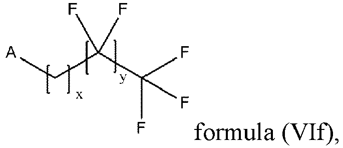 wherein A is an active group capable of reacting with a primary amino group, such as -COOH or 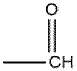 , x is an integer of 0-3, and y is an integer of 0-8.

Further, the fluorinated chitosan derivative for use as a drug carrier, the fluorine-containing aromatic ring compound is a class of fluorine-containing compounds with an active group capable of reacting with an amino group, and includes those as shown in formula (VII):

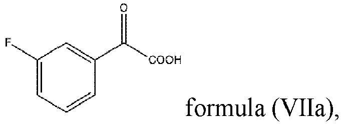

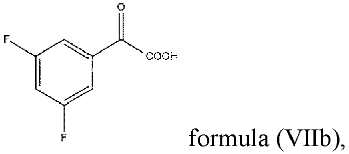

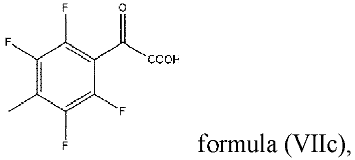

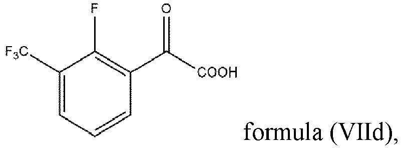

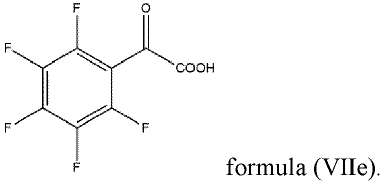

Further, the fluorinated chitosan derivative for use as a drug carrier serves as a drug carrier of a drug, and the drug is selected from a small molecule drug, a polypeptide, a protein drug, a combined drug of different drugs, and a combined drug of a drug and other pharmaceutical excipients.

Preferably, the invention also provides use of a fluorine-containing compound-modified chitosan as a drug carrier, the fluorinated chitosan derivative is used as a drug carrier of a small molecule drug, a polypeptide, a protein drug, a combined drug of different drugs, and a combined drug of a drug and other pharmaceutical excipients.

In an embodiment, the invention provides a drug composite, including the fluorinated chitosan derivative for use as a drug carrier and a drug, wherein the drug is selected from a small molecule drug, a polypeptide, a protein drug, a combined drug of different drugs, and a combined drug of a drug and other pharmaceutical excipients.

In an embodiment, the invention provides a transdermal administration preparation prepared from the fluorinated chitosan derivative for use as a drug carrier, including a transdermal preparation component (a), wherein the component (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps.

In an embodiment, the invention provides a transmucosal administration preparation prepared from the fluorinated chitosan derivative for use as a drug carrier, including a transmucosal preparation component (a), wherein the component (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps; the mucosa includes nasal mucosa, lung mucosa, vaginal mucosa, oral mucosa, and gastrointestinal mucosa.

In an embodiment, the invention provides an ocular barrier-penetrating administration preparation prepared from a fluorinated chitosan derivative for use as a drug carrier, including an ocular barrier-penetrating preparation component (a), wherein the component (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps; the ocular barrier is a tear barrier, a corneal/conjunctival barrier, and a blood-aqueous barrier, or a blood-retinal barrier.

In an embodiment, the invention provides a transdermal vaccine carrier prepared from a fluorinated chitosan derivative for use as a drug carrier, including a transdermal vaccine carrier (a), wherein the transdermal vaccine carrier (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps; the transdermal vaccine carrier has three antigen penetration pathways: transcellular penetration, paracellular penetration and transappendgeal penetration.

In an embodiment, the invention provides a carrier for cosmetic and health care products prepared from a fluorinated chitosan derivative for use as a drug carrier, including a carrier for cosmetic and health care products (a), wherein the carrier for cosmetic and health care products (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps; the carrier for cosmetic and health care products is suitable for hair growth drugs and hair care drugs, cosmetic drugs, and health care drugs.

A method for preparing a fluorinated chitosan derivative includes the following steps: preparation of a solution of chitosan in an aqueous acetic acid solution: chitosan is weighed and added into an aqueous acetic acid solution and stirred to be fully dissolved, and then sodium hydroxide is added dropwise and stirred until a clear solution is obtained and the pH is between 6.2-6.8;
activation of a fluorine-containing compound: a fluorine-containing compound is weighed and dissolved in an appropriate amount of anhydrous dimethyl sulfoxide, and the reaction amounts of EDC and NHS are sequentially added, and stirred under protection from light;
the activated fluorine-containing compound solution is added dropwise to the chitosan solution under rapid stirring, and stirred under protection from light, until fully reacted.

The method for preparing a fluorinated chitosan derivative, further includes the following steps: the fully reacted solution is slowly added dropwise to a solution of potassium hydroxide in ethanol and stirred, a precipitate is filtered and washed with a large amount of absolute ethanol until a filtrate is neutral, the precipitate is dehydrated by washing with methanol and diethyl ether and dried in vacuum, and the dried precipitate is dissolved in a hydrochloric acid solution and lyophilized to obtain a fluorinated chitosan hydrochloride.

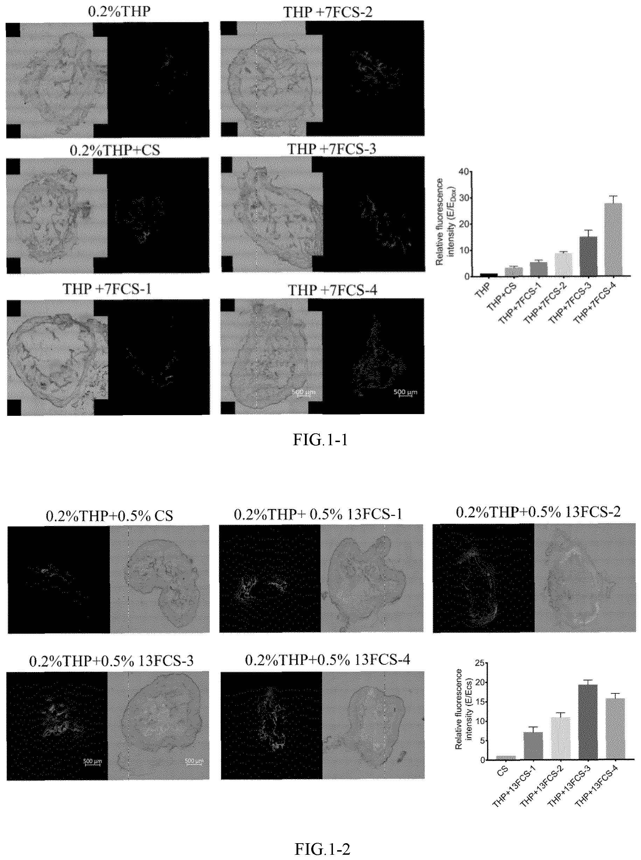

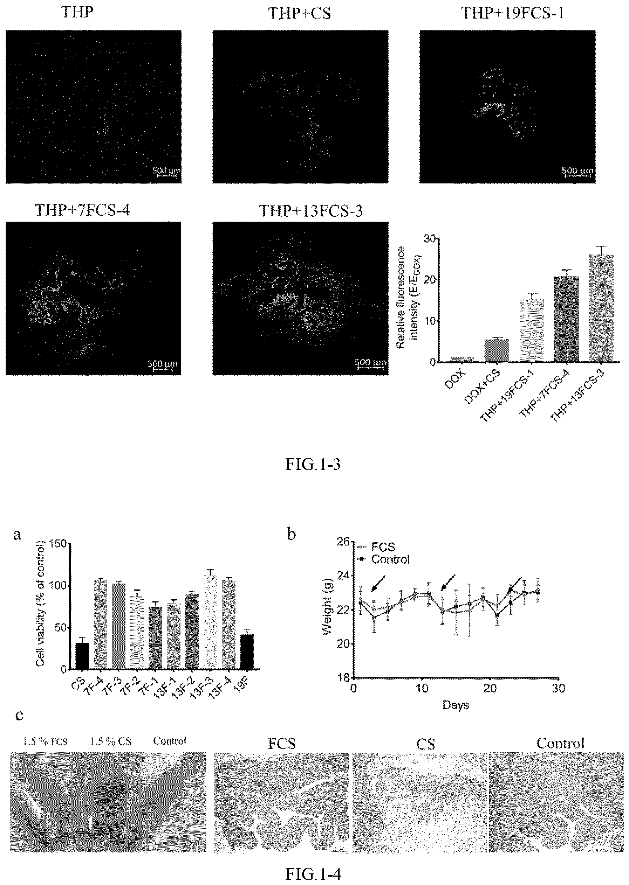

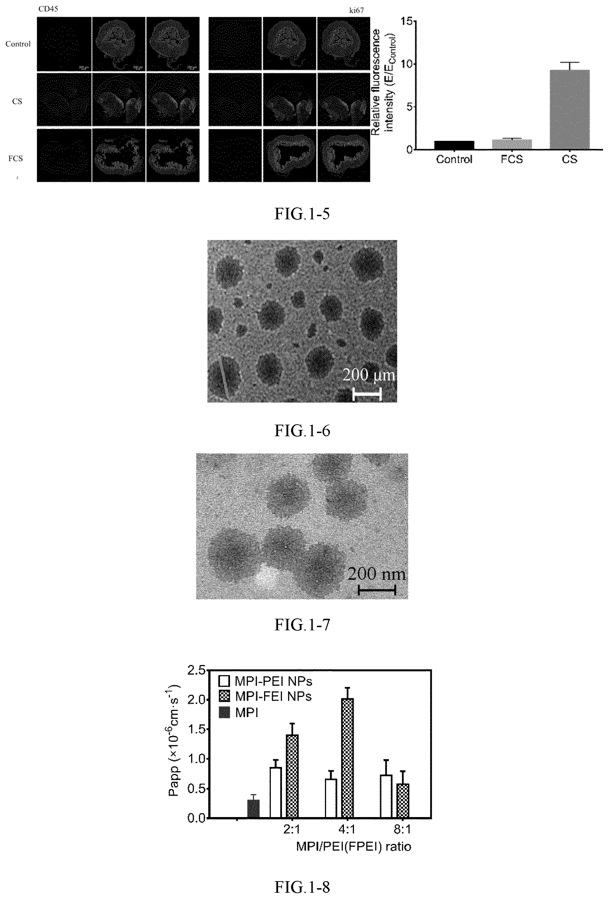

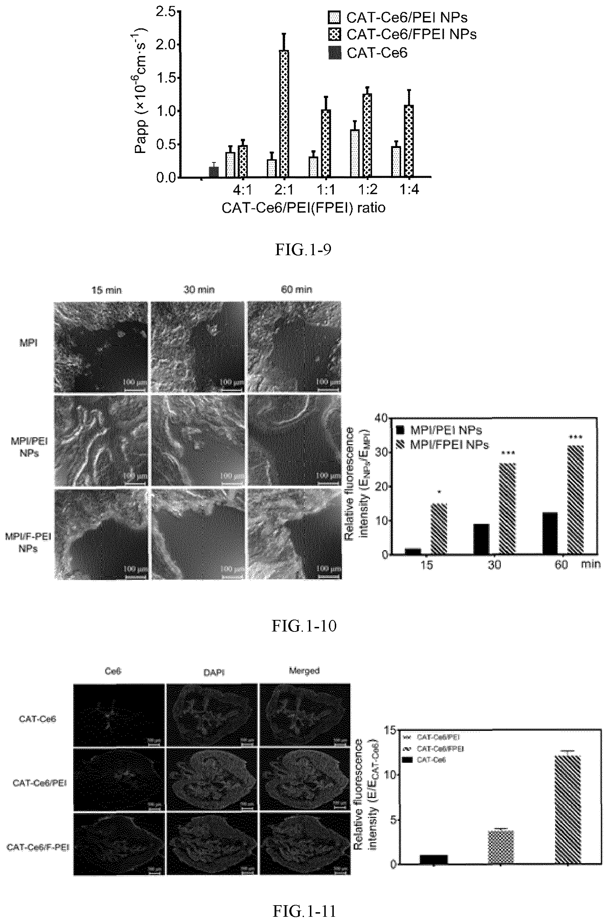

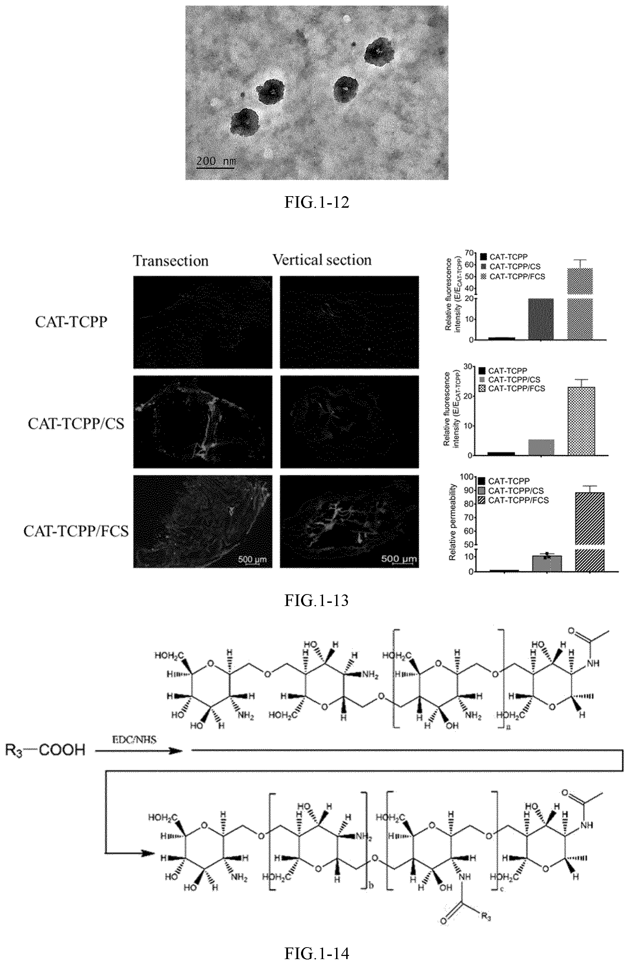

Refering to FIG. 1- FIG.14, which show a synthetic route diagram of a fluorine-containing carboxylic acid-modified chitosan as a bladder perfusion drug carrier.

A method for preparing 3-fluorobenzoic acid fluorinated chitosan includes the following steps:
(1) preparation of a solution of chitosan in an aqueous acetic acid solution: fully dried chitosan is weighed and added into an aqueous acetic acid solution and stirred to be fully dissolved, and then sodium hydroxide is slowly added dropwise and stirred until a clear solution is obtained and the pH is between 6.2-6.8;
(2) activation of 3-fluorobenzoic acid: 3-fluorobenzoic acid is weighed and dissolved in an appropriate amount of anhydrous dimethyl sulfoxide, and the reaction amounts of EDC and NHS are sequentially added, and fully stirred under protection from light;
(3) preparation of 3-fluorobenzoyl chitosan: the above-mentioned activated 3-fluorobenzoic acid solution is slowly added dropwise to the chitosan solution under rapid stirring, and stirred under protection from light, until fully reacted.

The method for preparing 3-fluorobenzoic acid fluorinated chitosanfurther includes the following steps:
the fully reacted solution is slowly added dropwise to a solution of potassium hydroxide in ethanol and stirred, a precipitate is filtered and washed with a large amount of absolute ethanol until a filtrate is neutral, the precipitate is dehydrated by washing with methanol and diethyl ether and dried in vacuum;
the dried precipitate is dissolved in a hydrochloric acid solution and lyophilized to obtain a 3-fluorobenzoic acid fluorinated chitosan hydrochloride.

A method for preparing perfluoroheptanoic acid fluorinated chitosan includes the following steps:
(1) preparation of a solution of chitosan in an aqueous acetic acid solution: fully dried chitosan is weighed and added into an aqueous acetic acid solution and stirred to be fully dissolved, and then sodium hydroxide is slowly added dropwise and stirred until a clear solution is obtained and the pH is between 6.2-6.8;
(2) activation of perfluoroheptanoic acid: perfluoroheptanoic acid is weighed and dissolved in an appropriate amount of anhydrous dimethyl sulfoxide, and appropriate amounts of EDC and NHS are sequentially added, and fully stirred under protection from light;
(3) preparation of perfluoroheptanoic acid-modified chitosan: the above-mentioned activated perfluoric acid solution is slowly added dropwise to the chitosan solution under rapid stirring, and stirred under protection from light, until fully reacted.

The method for preparing perfluoroheptanoic acid fluorinated chitosan further includes the following steps:
the fully reacted solution is slowly added dropwise to a solution of potassium hydroxide in ethanol and fully stirred, a precipitate is filtered and washed with a large amount of absolute ethanol until a filtrate is neutral, the precipitate is dehydrated by washing with methanol and diethyl ether and dried in vacuum, and the dried precipitate is dissolved in a hydrochloric acid solution and lyophilized to obtain perfluoroheptanoic acid fluorinated chitosan hydrochloride.

In the fluorinated chitosan derivative for use as a drug carrier, the fluorinated chitosan derivative is a perfluoroheptanoic acid fluorinated chitosan hydrochloride, and the degree of fluorination modification of the perfluoroheptanoic acid fluorinated chitosan hydrochloride is 18%-25% or 20%-22%.

The invention also provides a drug composite, which includes the fluorinated chitosan derivative for use as a drug carrier and a drug, wherein the drug is selected from a small molecule drug, a polypeptide, a protein drug, a combined drug of different drugs, and a combined drug of a drug and other pharmaceutical excipients.

Given that the high cytotoxicity of cationic polymer materials during use ultimately limits their clinical applications, and that although chitosan can significantly improve the bioavailability of perfusion drugs, high concentrations of chitosan may cause severe mucosal and epithelial damage, limiting its clinical application as a drug carrier. In recent years, the inventors have tried various technical solutions to improve biological safety. For example, various modification groups such as polyethylene glycol, cyclodextrin, amino acids, glycosyl group, fluorination modification are attached to the surface of these cationic polymers. However, these have little effect. In accidental experiments, it has been found that fluorinated cationic polymer materials can be significantly improved in terms of biocompatibility and macromolecule transport effect, and has a stronger binding and protective effect on nucleic acids, polypeptides and protein drugs.

In an embodiment, the inventors have designed and synthesized a series of fluorinated chitosan derivatives. The experimental results show that fluorinated chitosan (FCS) has a more significant performance in promoting drug penetration and absorption than chitosan. Also, the results of a safety evaluation test in cells and mice *in vivo* show that FCS has good biological safety, and even with high concentrations, FCS has no obvious cytotoxicity and mucosal and epithelial damage, and its biological toxicity is significantly lower than that of unmodified chitosan.

The inventors have established an *in vitro* bladder mucosal barrier model by selecting SV-HUC-1 human normal bladder cancer commercial cells, and briefly explained the mechanism of FCS in promoting the drug penetration and absorption through bladder mucosa by investigating the influence of FCS on the membrane resistance of SV-HUC-1 monolayer cells, permeability of fluorescent yellow, tight junction ultrastructure of cells and tight junction proteins. Experimental results show that FCS can significantly reduce the membrane resistance value of SV-HUC-1 monolayer cells, increase the permeation efficiency of fluorescent yellow, and regulate the tight junctions of cells by changing the structure and spatial distribution of tight junction proteins and E-cadherin, and increase the uptake efficiency of drug molecules through paracellular pathway. In other words, FCS can effectively increase the gap between cells in biological tissue barriers (such as mucosal epithelial tissue, etc.), so that free drug molecules or drugs carried by FCS can more effectively traverse these biological tissue barriers.

In addition, a large amount of experimental data shows that as the length of the fluorine-containing aliphatic chain and the degree of substitution on the chitosan backbone increase, the drug penetration and absorption promoting capacity of the modified product first increases and then decreases, indicating that the modification of chitosan cannot be over-fluorinated, and the effect of fluorinated chitosan on promoting mucosal permeation and absorption of perfusion drugs may be the result of the combined effect of the positively charged molecular skeleton of chitosan and the fluorine-containing aliphatic chain.

Therefore, in the present invention FCS is selected as a novel drug carrier for further research. In the present invention, a series of fluorinated chitosan derivatives are designed and synthesized, and their applications in the pharmaceutical field include but are not limited to the following disease models, such as bladder cancer perfusion administration (or other intracavitary perfusion), lung inhalation administration, transdermal administration, oral administration, ocular administration, vaccine administration, nasal administration.

Bladder cancer is one of the most common urinary tumors. Clinically, more than 75% of bladder cancers are non-muscular invasive bladder cancer (NMIBC), where 30% to 80% of NMIBC patients relapse within 5 years after transurethral resection of bladder tumor (TURBT), and 10%-20% of NMIBC patients progress to muscular invasive bladder cancer. Therefore, post-TURBT adjuvant perfusion chemotherapy or immunotherapy to inhibit or delay tumor recurrence has become the first choice for bladder cancer according to clinical treatment guidelines. Although post-TURBT adjuvant chemotherapy drugs can delay tumor recurrence, due to the physiological characteristics of the bladder and the physiological barrier effect of its mucosa, the conventional bladder perfusion of liquid drugs results in limited residence time in the bladder, short action time, and low bioavailability, and thus the time- and concentration-dependent perfusion drugs cannot exert significant anti-tumor effects, so that the absolute risk of recurrence and progression of bladder cancer cannot be effectively reduced and the prognosis cannot be effectively improved. The technical solution of the present invention is based on the use of FCS as a new transmucosal drug carrier, which can improve the bioavailability of perfusion drugs, and increase the efficiency of drugs entering the internal structure of bladder tumors after intracavitary perfusion, thereby improving the efficacy of bladder perfusion therapy. This technical solution can also be applied to other intracavitary perfusion therapies (such as peritoneal cavity, pelvic cavity, thoracic cavity).

Compared with intravenous administration therapy, pulmonary inhalation administration therapy locally delivers a drug to a tumor tissue, so the required drug dosage is significantly reduced, and the toxic and side effects are slight. The special physiological structure of the lungs determines the characteristics and advantages of lung inhalation administration: the lungs have a large surface area, abundant capillaries, and a thin layer of alveolar epithelial cells, so that pulmonary administration takes effect quickly; biological metabolic enzymes in the lungs are concentrated, with low biological activity, which reduces the hydrolysis of proteins, so that proteins and polypeptides are easily quickly absorbed through the surface of the alveoli, maintaining their biological activity; the liver first-pass effect is avoided. However, there are still some shortcomings that limit its clinical application. For example, the inhaled drug is quickly cleared from the lungs, and the effective deposition of the drug in the lungs cannot be guaranteed. For lung cancer, although the drug inhaled from the lungs can reach the alveoli, the efficiency of entering the interior of the lung tumor is generally very low, which seriously affects the efficacy of the lung inhalation administration mode for the treatment of lung cancer. The technical solution of the present invention is based on the use of FCS as a new transmucosal drug carrier, which can improve the bioavailability of drugs for lung inhalation, and increase the efficiency of drugs entering the internal structure of lung tumors after inhalation, thereby improving the efficacy of lung inhalation administration therapy.

The transdermal administration system refers to a preparation where a drug is administered on the surface of the skin and the drug passes through each layer of the skin at a certain rate and enters the systemic circulation to produce a systemic or local therapeutic effect. As a non-invasive topical administration mode, transdermal administration has many advantages such as simple operation and strong patient adaptability. However, transdermal administration is usually restricted by the stratum corneum lipid lamellae barrier of the skin and the physical and chemical properties of the drug. How to improve the ability of the drug to enter the blood circulation or enter subcutaneous lesions (such as skin cancer) through the transdermal administration mode is a major challenge for this technology. The technical solution of the present invention is based on the use of FCS as a new transmucosal drug carrier, which can improve the bioavailability of transdermal drugs, greatly increase the ability of drugs to penetrate the skin barrier, and enable drugs to enter the blood circulation or enter subcutaneous lesions (such as skin cancer) through the transdermal administration mode more effectively, thereby improving the efficacy of transdermal administration therapy.

The present invention provides a composite including a fluorine-containing compound-modified chitosan and a drug, and a use thereof in promoting drug absorption, wherein the drug includes a small molecule drug, a polypeptide, a protein drug, a combined drug of different drugs, and a combined drug of a drug and other pharmaceutical excipients.

For example, epirubicin (THP) is used as a bladder perfusion drug, and the fluorine-containing compound-modified chitosan prepared by the present invention is used as a drug transport carrier to promote the drug to enter the bladder tissue. Experiments show that the present invention has the following advantages: the present invention maintains good biocompatibility while maintaining significant promotion on absorption efficiency of the perfusion drug in the bladder mucosa. Through *in vivo* perfusion experiments in mice, it is found that the efficiency of the fluorine-containing compound-modified chitosan in improving the absorption of THP in the bladder mucosa is significantly higher than that of an aqueous solution of THP and that of a solution of chitosan; and also, the fluorine-containing compound-modified chitosan achieves high efficiency in promoting bladder mucosal absorption of the drug at lower concentrations. The bladder perfusion drug carrier provided by the present invention has the advantages of high efficiency, low toxicity, low price, simple synthesis and the like.

In an embodiment, the invention provides a pharmaceutical composition using the transdermal administration preparation, including a transdermal preparation component (a) and a drug component (b), wherein the component (b) is a diabetes drug, an anti-tumor drug, an immunomodulator, an antiviral drug, an anti-inflammatory drug, or an analgesic drug.

In an embodiment, a drug composite for the treatment of diabetes using the transdermal administration preparationincludes fluorinated chitosan and insulin, wherein the weight ratio of the fluorinated chitosan to the insulin is 1:0.25-4.

In an embodiment, in the drug composite for the treatment of diabetes using the transdermal administration preparation, the fluorinated chitosan and the insulin are mutually adsorbed to form a composite, and the particle size of the composite is less than 10 microns, or the particle size of the composite is not greater than 500 nanometers, and the weight ratio of the fluorinated chitosan to the insulin is 1:0.5-2.

In an embodiment, the invention provides a method for preparing the drug composite for the treatment of diabetes using the transdermal administration mode, wherein:
fluorinated chitosan and insulin are separately dissolved in a weak acid solution environment until uniformly dissolved;
the fluorinated chitosan and the insulin are uniformly mixed at a reaction weight ratio of 1:0.25-4, a weak base solution is dropwise added during stirring after uniform mixing, the pH is adjusted to 6-7, and under neutral conditions, the fluorinated chitosan and the insulin are adsorbed together to form stable nanoparticles.

In an embodiment, the method for preparing the drug composite for the treatment of diabetes utilizes the transdermal administration mode:
the reaction weight ratio of the fluorinated chitosan to the insulin is 1:0.25-4 or 1:0.5-2;
after the reaction is complete, the pharmaceutical composition is removed, pre-added with a cryoprotectant and then lyophilized to obtain a fluorinated chitosan-insulin lyophilized powder.

In an embodiment, the invention also provides a transdermal patch of a pharmaceutical composition for the treatment of diabetes using the transdermal administration preparation, wherein the fluorinated chitosan and the insulin are mutually adsorbed to form a composite, the weight ratio of the fluorinated chitosan to the insulin is 1: 0.25-4, and the composite is uniformly mixed with a hydrogel to obtain a transdermal patch.

In an embodiment, the invention also provides a drug composite for the treatment of melanoma using the transdermal administration preparation, wherein the fluorinated chitosan and programmed cell death-ligand 1 antibody form a composite, and the particle size range of the composite is less than 10 microns, or the particle size range of the composite is not greater than 500 nanometers, and the reaction weight ratio of the fluorinated chitosan to the programmed cell death-ligand 1 antibody is 1:0.25- 4.

In an embodiment, in the drug composite for treating melanoma, the reaction weight ratio of the fluorinated chitosan and the programmed cell death-ligand 1 antibody is 1:0.25-4 or 1:1, and an aqueous solution of the fluorinated chitosan-programmed cell death-ligand 1 antibody is mixed with petrolatum ointment to form a fluorinated chitosan-programmed cell death-ligand 1 antibody transdermal ointment.

The invention also provides a transdermal administration preparation including a component (a), wherein the component (a) is a fluorine-containing compound-modified cationic polymer, and the fluorine-containing compound-modified cationic polymer can be used as a transdermal administration preparation of a drug for treating diabetes, a drug for treating tumor diseases, or an anti-inflammatory drug.

The invention also provides a transdermal administration preparation including a component (a), wherein the component (a) is a fluorine-containing compound-modified cationic polymer, and the fluorine-containing compound-modified cationic polymer is used as a transdermal administration preparation in the preparation of a medical cosmetic drug, a topical drug preparation, a topical preparation for medical devices, and a cosmetic skin care product.

Conventional transdermal patches usually need to add a penetration enhancer into the patch to promote the penetration of the drug through the skin. Commonly used penetration enhancers include alcohols such as ethanol and butanol, dimethyl sulfoxide, laurocapram, pyrrolone and their derivatives, surfactants and fatty acid compounds. Among them, the penetration-promoting mechanism of alcohols and pyrrolone derivatives is mainly to swell lipids in the stratum corneum to increase the solubility of the drug; laurocaprams are to change the compactness of a lipid bilayer and increase the fluidity of lipids to promote the skin penetration of the drug; when fatty acid compounds are inserted into a hydrophobic structure of a lipid bilayer through the cis structure of an unsaturated hydrophobic chain thereof, the lipid bilayer is twisted, forming a very fine pore channel, allowing the drug to diffuse into it. The physical penetration enhancement methods mainly include iontophoresis method, ultrasonic method, electroporation method and microneedling method. The physical penetration enhancement method is mainly used for drugs for which chemical penetration enhancers are difficult to work, for example, macromolecular drugs such as polypeptides and proteins, and ionic drugs. The iontophoresis method is to apply an appropriate electric field on the skin surface to increase the skin penetration rate of the drug. Due to the existence of the electric field, the interaction of ions in the electric field, the convective movement of the solvent under the electric field and the increase in skin permeability caused by the electric current will all promote the transdermal absorption of the drug. The ultrasound method is to promote the entry of drug molecules into the skin under the action of ultrasound. The possible mechanisms are that: 1. the local thermal effect results in an increase in the drug permeability; 2. the local radiation pressure effect causes the drug to move in the direction of sound waves to promote drug penetration; 3. the local micro-streaming effect enables the drug to enter the skin through hair follicles and sweat glands; and 4. the cavitation effect causes disordered arrangement of the stratum corneum of the skin to promote drug penetration. The electroporation method is to promote the transdermal absorption of the drug by means of a pulsed electric field. The transdermal mechanism of the currently reported electroporation method is that under a pulsed electric field, the lipid molecules in the skin are re-arranged in an orderly manner to form a new channel to promote drug penetration. After the pulsed electric field is over, the lipid molecules recover the previous disorderly arrangement, thereby closing the channel. The microneedling method is to use micron-level microneedles to form very small wounds on the skin to efficiently promote skin penetration of the drug.

The fluorine-containing compound-modified cationic polymer, especially fluorinated chitosan, described in the present invention can increase the penetration capacity of the drug in the skin, while decreasing the administration area, thereby reducing the toxic and side effects that the drug may have on the normal skin. As a chemical penetration enhancing polymer, compared with other chemical penetration enhancers, the technical solution described in the present invention does not have strong volatility and irritation as an aqueous solution, has a fast action speed and a long duration, and can promote the skin penetration of a series of drug molecules, including small molecule drugs, macromolecular drugs such as polypeptides and proteins, ionic drugs, etc. Compared with the physical penetration enhancement methods, the technical solution of the present invention does not require an external electric field, ultrasound, etc., which greatly reduces the trauma, pain, inconvenience and safety hazards for patients. Therefore, the fluorinated chitosans disclosed in the examples of the present invention can all be used as transdermal preparations and used in combination with other drugs. Also, the fluorine-containing compound-modified cationic polymers can also be used as transdermal preparations and used in combination with other drugs due to modification with the fluorine-containing compound.

The fluorinated chitosan drug carrier provided by the present invention has the advantages of obvious drug absorption promoting effect and low toxicity, etc. The fluorine-containing compound-modified chitosan proposed by the present invention has a mature synthesis process, simple operation, high synthesis efficiency, short cycle time, and a high yield without complicated purification steps, and this simple synthesis method provides a good basis for commercialization. The fluorine-containing compound-modified chitosan of the present invention is useful as a variety of drug carriers, can effectively improve the therapeutic effect, and has a wide range of uses and a low cost.

Further, the transdermal effect produced by the technical solution of the present invention is temporary. After the drug is removed, the stratum corneum cells of the skin will close the channel to protect the safety of the human body. The skin consists of epidermis and dermis. The epidermis is divided into stratum corneum, stratum lucidum, stratum granulosum and stratum germinativum in order from superficial to deep. The dermis is composed of dense connective tissues, and is divided into papillary layer and reticular layer in order from superficial to deep. The papillary layer is connected to the stratum germinativum of the epidermis, and contains abundant receptors such as capillaries, lymphatic vessels, nerve endings, and tactile corpuscles. The stratum corneum is the largest rate-limiting barrier for transdermal administration. The stratum corneum in most of the skin consists of 5-25 layers of flat keratinocytes. These cells have no nucleus and organelles, and have thicker cell membranes. They are lifeless and water-tight, and have the functions of preventing tissue fluid from flowing out, resisting friction and preventing infection. The fluorine-containing compound-modified cationic polymer can stimulate a change in the distribution of tight junction proteins in these cells to reduce tight junctions between cells, and further stimulate actin phosphorylation, thereby promoting the paracellular transport and opening the intercellular space to forms channels, so that the drug is carried through the stratum corneum and is further penetrated into the skin, and then into the dermis and into the skin capillaries and lymphatic circulation to exert the drug effect (see FIG. 1).

The technical solution of the present invention can be used in an oral administration system, specifically as follows.

### Intestinal mucosa:

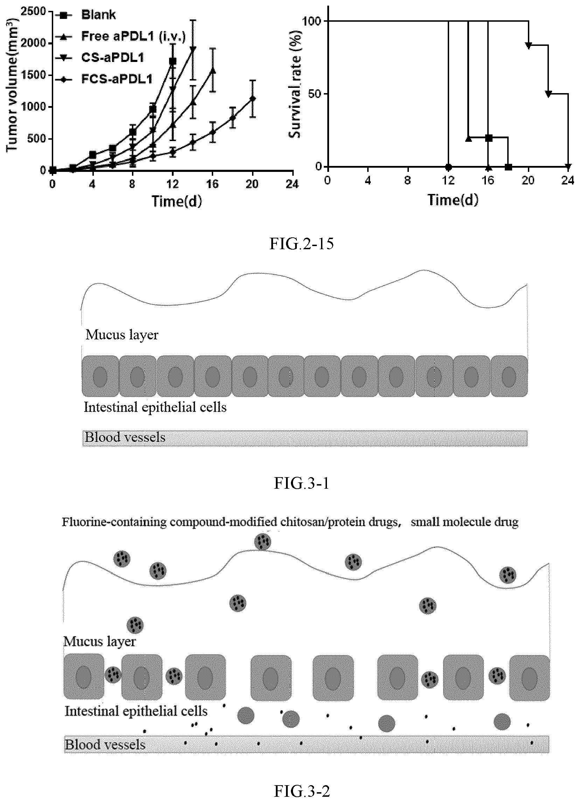

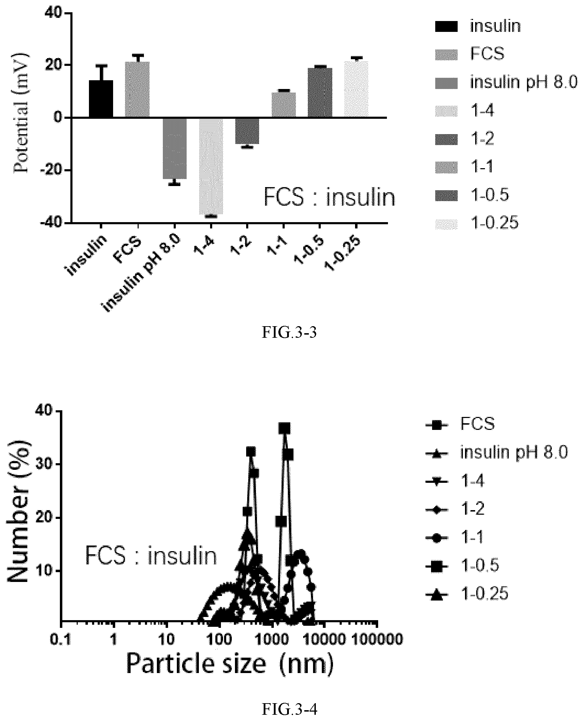

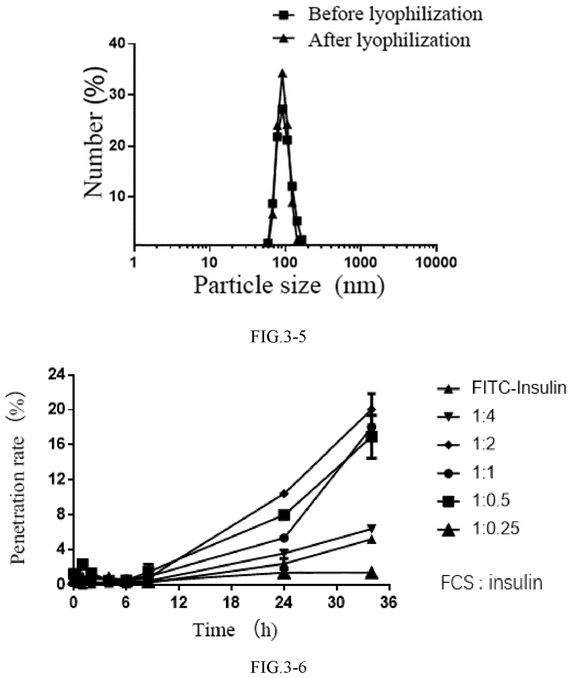

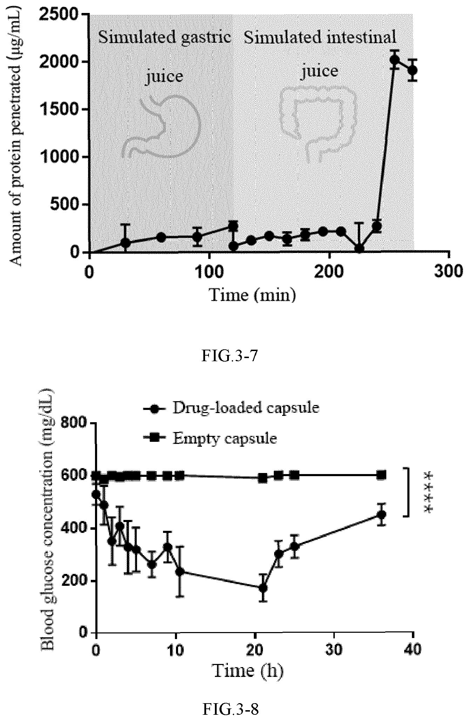

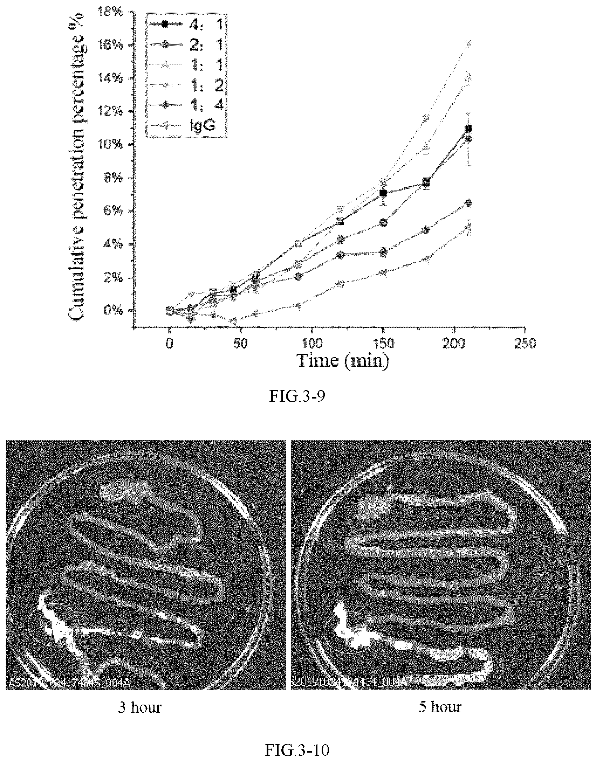

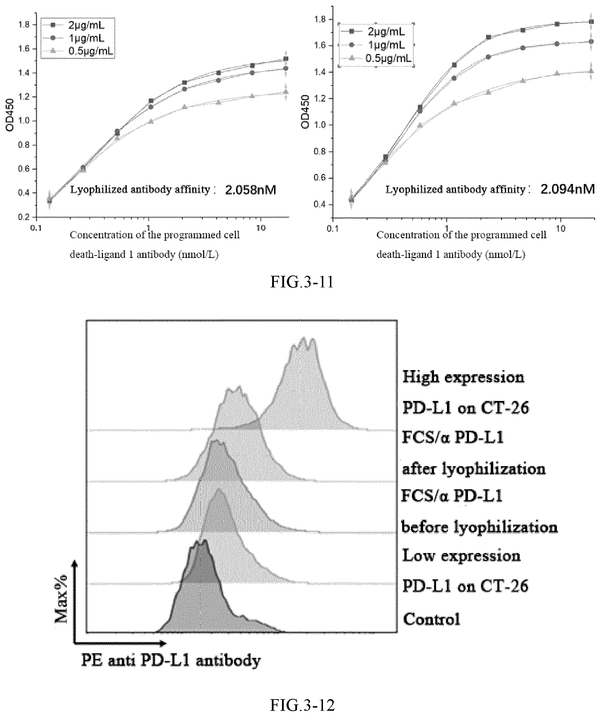

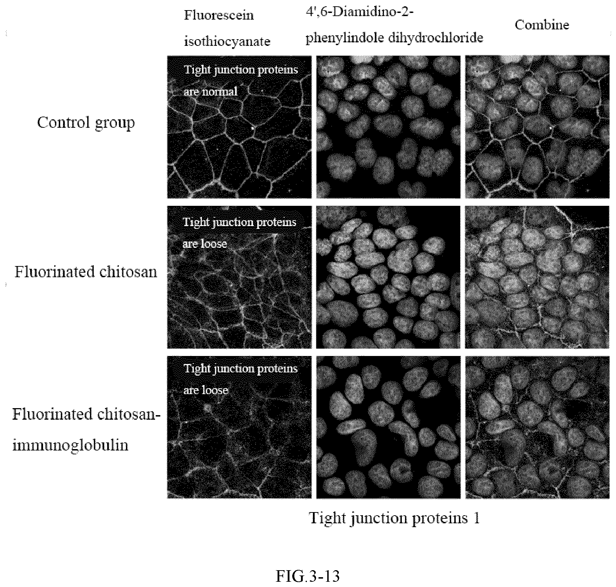

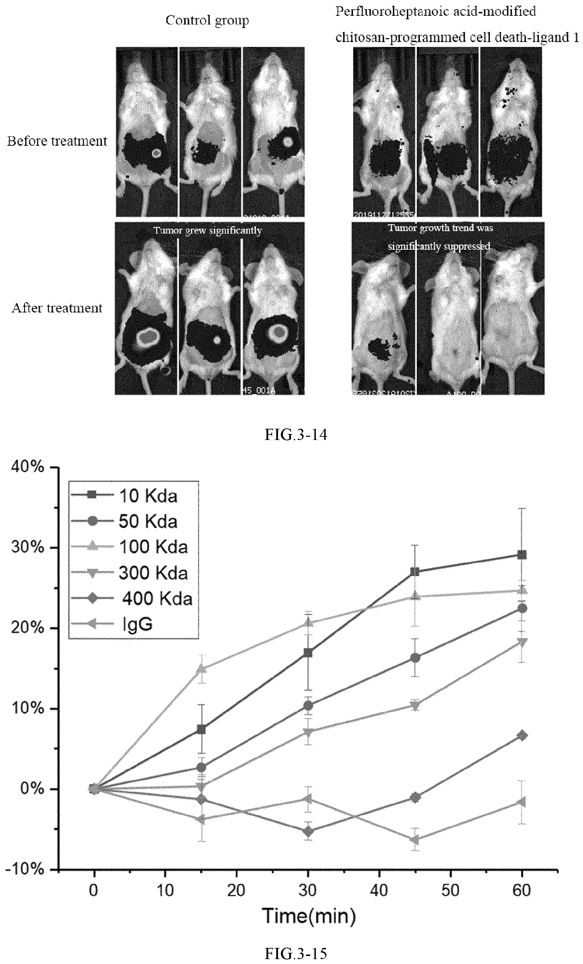

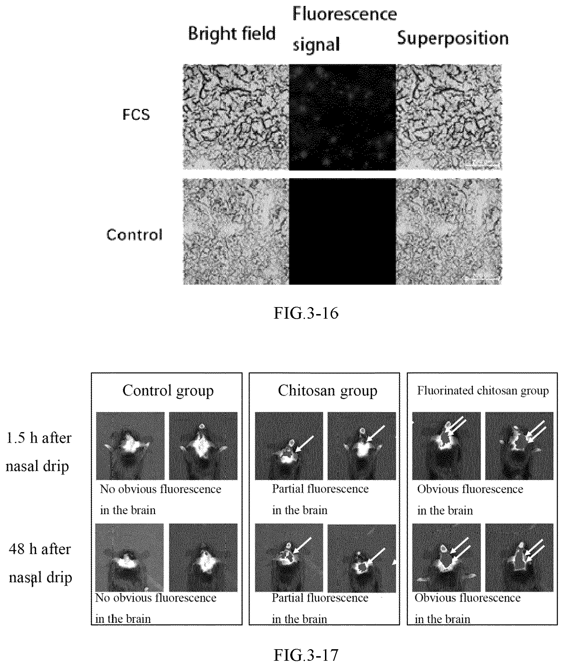

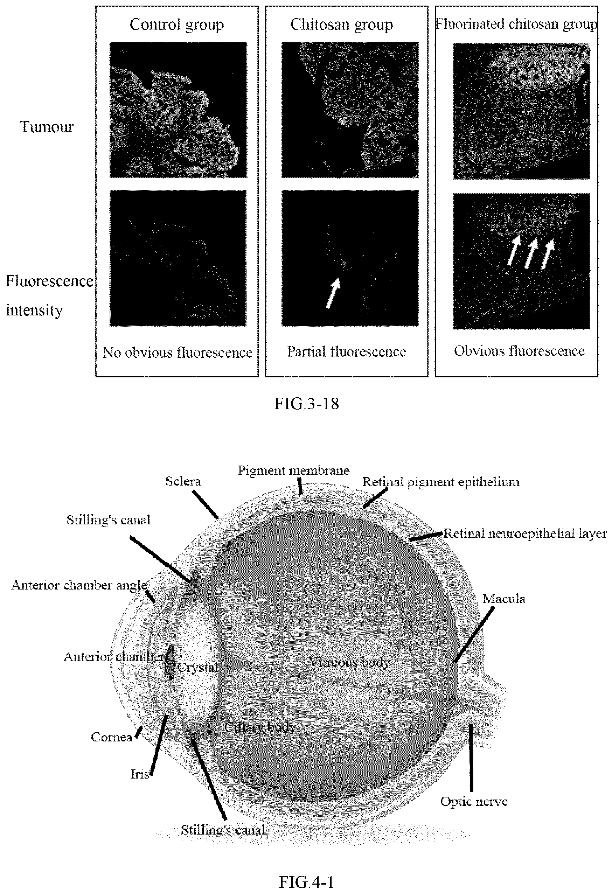

The small intestine is the main part of drug absorption. Before being absorbed from the small intestine into the blood circulation, a drug will inevitably face three main gastrointestinal physiological barriers, as shown in FIG. 3-1, including a protease barrier, a mucus barrier and an intestinal epithelial cell barrier. There are abundant proteases in the small intestine, including trypsin, chymotrypsin, elastase and carboxypeptidase. Protein and polypeptide drugs can be rapidly degraded and inactivated under the action of various proteases. There is also a mucus barrier in the intestine. The surface of the intestinal epithelial cells is covered with a layer of mucus of varying thickness, which is composed of 95% water and 5% electrolytes, lipids, proteins and glycoproteins. The mucus layer has viscoelasticity and plays a protective role. Also, the mucus barrier is also one of the main barriers that affect the oral absorption of protein and polypeptide drugs. In addition to the mucus barrier, there is also an intestinal epithelial cell barrier in the intestine. The intestinal epithelial cell layer mainly includes two types of cells: epithelial cells (intestinal epithelial cells) that can absorb substances and epithelial cells that are difficult to absorb substances. These cells are linked to each other through tight junctions, forming a relatively impermeable barrier that severely limits the absorption of foreign substances.

Using fluorinated chitosan as a carrier can not only solve the above problems, but also increase the penetration of the drug, so that the drug can reach the blood or the treatment site efficiently, and the availability of the drug can be improved.

Next, taking insulin and programmed cell death-ligand 1 antibody as example drugs, the transmucosal mechanism of fluorinated chitosan is explained.

As described in Example 3-1, when fluorinated chitosan is used as a drug carrier to deliver insulin, it can open epithelial channels without disrupting the function of intestinal epithelial cells to secrete tight junction proteins, as shown in FIG. 3-2.

With fluorinated chitosan as the main body, corresponding oral drugs such as solutions, syrups, granules, capsules, powders, pills, and tablets, can be made. After oral administration, the drug is absorbed by the gastrointestinal tract into the blood, and reaches local or systemic tissues through blood circulation, to achieve the purpose of treating diseases. However, protein and polypeptide drugs are easily degraded by digestive enzymes in the gastrointestinal tract. Also, they have a large molecular weight and there is a strong tendency to aggregate between molecules, making it difficult to pass through the barriers in the body to play a sufficient role. If not treated for direct oral absorption, their bioavailability is only 0.5%. Using fluorinated chitosan as a carrier can maintain the activity of protein and polypeptide drugs, and stabilize their configuration; reduce the formation of multimers and facilitate to the absorption in the intestinal mucosa; and reduce the degradation of protein and polypeptide drugs by digestive enzymes; and also, can open the epithelial channels without destroying the function of intestinal epithelial cells to secrete tight junction proteins, which can increase the penetration of the drug in the small intestine, facilitate the drug to reach the blood or the treatment site efficiently, and improve the availability of the drug. Next, taking insulin and programmed cell death-ligand 1 antibody as example drugs, the use of a fluorine-containing compound-modified cationic polymer as a drug carrier in oral administration is realized.

The drug may be a diabetes drug, an anticolitis drug, an anesthetic drug, an anti-inflammatory drug, an antibacterial drug, an antiviral drug, an antiparasitic drug, etc.

The diabetes drug may be sulfonylureas, including, but not limited to, sulfambutamide, tolbutamide, chlorpropamide, acetohexamide, gliclazide, glipyride, glimepiride and the like and derivatives thereof. The diabetes drug may be non-sulfonylureas, including, but not limited to, repaglinide, nateglinide and the like and derivatives thereof. The diabetes drug may be thiazolidinediones, including, but not limited to, rosiglitazone, pioglitazone and the like and derivatives thereof. The diabetes drug may be biguanides, including, but not limited to, phenformin, metformin and the like and derivatives thereof. The diabetes drug may be an α-glucosidase inhibitor, including, but not limited to, acarbose, voglibose, miglitol and the like and derivatives thereof. The diabetes drug may be dipeptidyl peptidase-IV, including, but not limited to, glucagon-like peptide, DPP-IV inhibitors, sitagliptin, vildagliptin, and saxagliptin. In one embodiment of the present invention, the anti-diabetic drug includes, but is not limited to, insulin.

As described in Example 3-1, fluorinated chitosan can be used as a drug carrier to deliver insulin, which is administered as an oral drug for the treatment of diabetes. With the increase in diabetes cases worldwide and the lack of compliance of patients with blood glucose management using injectable insulin, there is an urgent need to develop effective oral insulin preparations. However, the gastrointestinal tract presents a huge obstacle to the oral delivery of biological agents. The present invention can solve the problem of low oral availability of insulin. Oral administration is the most acceptable route of administration for patients. However, insulin is easily degraded by various enzymes in the gastrointestinal tract, and insulin itself has a large molecular weight and is difficult to be absorbed by the epithelial cell membrane of the gastrointestinal tract. The oral administration of insulin through the fluorinated chitosan drug carrier can not only protect the activity of insulin, but also promote the passage of insulin through the small intestinal mucosa to improve the availability of the drug.

Any disease or dysfunction, especially body injury, may cause a person to feel pain. In many cases, analgesia treatment is needed. Analgesics are a class of drugs that act on the central nervous system and selectively inhibit the pain center, reducing or eliminating pain without affecting other sensations. They are mainly used clinically to relieve severe pain caused by trauma, burns, post-operation and cancer. However, many analgesics can not quickly relieve pain and be administered locally. The present invention can solve this problem.

The analgesics may be morphine and derivatives thereof, including, but not limited to, codeine, ethylmorphine, benzylmorphine, iso-codeine, heroin, phenethylmorphine, hydromorphone, oxymorphone, nalorphine, nalbuphine, naloxone, naltrexone and the like and derivatives thereof.

The analgesics may be synthetic analgesics, including, but not limited to, pethidine, anileridine, phenoperidine, piminodine, alphaprodine, betaprodine, fentanyl, Alfentanil, sufentanil, remifentanil, pethidine hydrochloride, methadone, dextromoramide, dextropropoxyphene, methadone hydrochloride, N-methylmorphinan, levorphanol, butorphanol, morphinan, pentazocine, dezocine, sumatriptan, tramadol, pizotifen, nefopam.

Anesthetics refer to drugs that can cause temporary and reversible loss of consciousness and pain in the entire body or a part of the body of a patient. However, most of the anesthetics are intravenously injected and intraspinally injected. These two modes of administration increase the pain of patients compared to oral administration. The present invention can increase patient compliance through oral administration of anesthetics.

The anesthetics may be intravenously injected anesthetics, including, but not limited to, ketamine hydrochloride, propofol, sodium thiopental, etomidate, midazolam and sodium gamma-hydroxybutyrate.

The anesthetics may be local anesthetics, including, but not limited to, aromatic acid esters, aromatic amides, amino ketones, amino ethers, carbamates, hydroxyprocaine, chloroprocaine, tetracaine, butacaine, thiocaine, procainamide, bupivacaine, articaine, etidocaine, ropivacaine, mepivacaine, clonin hydrochloride and the like and derivatives thereof.

Inflammation is closely related to the occurrence and development of many major diseases such as cardiovascular and cerebrovascular diseases, neurodegenerative diseases and even tumors. Therefore, early imaging, diagnosis and anti-inflammatory strategies for inflammation are important means to prevent and treat many diseases. At present, most anti-inflammatory drugs are administered intravenously and orally. However, intravenous injection of anti-inflammatory drugs can have serious side effects, and oral administration of anti-inflammatory drugs results in extremely low bioavailability. The present invention can improve the oral availability of anti-inflammatory drugs, and enable patients to have higher compliance.

The anti-inflammatory drugs may be non-steroidal anti-inflammatory drugs, including, but not limited to, aspirin, acetaminophen, non-specific cyclooxygenase inhibitors such as antipyrine, analgin, butazolidin, oxyphenbutazone, mefenamic acid, indometacin, sulindac, diclofenac sodium, ibuprofen, naproxen, piroxicam, meloxicam and derivatives thereof.

The anti-inflammatory drugs may be steroidal anti-inflammatory drugs, including, but not limited to, hydrocortisone, corticosterone, aldosterone, triamcinolone, prednisolone, dexamethasone acetate, methylprednisolone aceponate and the like and derivatives thereof.

The embodiments of the present invention can be used to treat various inflammations, including, but not limited to, myocarditis, arthritis, colitis, tonsillitis, gastritis, rhinitis, periodontitis, enteritis, pharyngitis, prostatitis, vaginitis, cervicitis, scapulohumeral periarthritis, cervical spondylosis, bursitis, dermatitis, conjunctivitis, otitis media, etc.

### Colonic mucosa:

The colon is an important part of the digestive system, is mainly involved in the absorption of water, vitamins and inorganic salts, and is also an important place for the formation of feces. The intestinal wall of the colon can be divided into serosa, longitudinalis, tunica muscularis, submucosa and mucous layer from outside to inside. As the human body's first line of defense, the mucosal layer can protect the intestine from harmful substances such as bacteria and toxins in the intestinal cavity. The normal colonic mucosal barrier is composed of a mechanical barrier, a chemical barrier, an immune barrier and a biological barrier. Among them, the mechanical barrier includes colonic epithelial cells and the junctions between epithelial cells, and is an important part of the mucosal barrier; the chemical barrier mainly includes mucus secreted by the mucosal epithelium, digestive juice and antibacterial substances produced by the intestinal native flora; the immune barrier is composed of the lymphoid tissue of the intestinal mucosa and the antibodies secreted by the intestine, and the lymphoid tissue can protect the intestine from some pathogenic antigens through cellular immunity and humoral immunity; and the biological barrier is a normal intestinal commensal flora, and an interdependent micro-ecosystem is formed between the intestinal commensal flora and the host. Under normal circumstances, the intestinal flora can form a microbial barrier to protect the body from foreign microorganisms. The colonic epithelial cell barrier is an important part of the intestinal mechanical barrier, and is composed of colonic epithelial cells and cell junctions between epithelial cells. These intercellular junctions include tight junctions, gap junctions, and adhesion junctions, which work together to close the intercellular space. The tight junctions play a vital role and are composed of junction adhesion molecules, Occludin, Claudin, and ZO-1. Cell junctions can prevent macromolecular substances such as endotoxin in the intestinal cavity from entering the intestinal tissue.

Due to the presence of the above-mentioned barriers, it is difficult for drugs to penetrate into the colon for patients with diseases in the colon. Using fluorinated chitosan as a carrier can effectively overcome the colon-related mechanical barrier, chemical barrier, immune barrier and biological barrier, improve the permeability of drugs in the colon, and enhance the availability of drugs. Compared with intravenous injection or intraperitoneal injection, more drugs will penetrate the affected site.

As described in Example 3-2, when fluorinated chitosan is used as a drug carrier to deliver insulin, it can open epithelial channels without disrupting the function of intestinal epithelial cells to secrete tight junction proteins, as shown in FIG. 3-2.

As described in Example 3-2, fluorinated chitosan can be used as a drug carrier to deliver anti-tumor drugs such as immune checkpoint blocking antibodies, which are administered as oral drugs for treating tumors. Colon cancer is one of the most common gastrointestinal tumors, with high morbidity and mortality. Although the programmed cell death-ligand 1 antibodies have been successful in the treatment of colorectal cancer, the programmed cell death-ligand 1 antibodies are all administered by intravenous injection, which may cause serious adverse reactions such as gastrointestinal toxicity. In addition, due to systemic administration, the drug is less enriched in the colorectal region. Oral administration, as the most acceptable mode of administration for patients, can deliver most of the drugs to the affected site and reduce adverse reactions. However, the programmed cell death-ligand 1 antibody is easily inactivated due to the harsh environment in the digestive tract, and the programmed cell death-ligand 1 antibody has a large molecular weight and is difficult to cross the intestinal epithelial cells. Oral administration of the programmed cell death-ligand 1 antibody through the fluorinated chitosan drug carrier can make the programmed cell death-ligand 1 antibody pass through the colorectal mucosa to improve the availability of the drug.

Inflammatory bowel disease (IBD) is a chronic idiopathic inflammation that can affect the entire gastrointestinal tract (GI) and increase the risk of colorectal cancer. IBD consists of two main clinically defined forms, Crohn's disease (CD) and ulcerative colitis (UC). CD usually involves the ileum and colon, but it can discontinuously affect any area of the gastrointestinal tract, and the inflammation is usually transmural. In contrast, UC is confined to the large intestine, extending from the rectum to the proximal end, and may involve the entire colon in an uninterrupted manner, and is accompanied by extensive superficial mucosal ulcers. Both CD and UC are associated with high morbidity and decreased quality of life. The incidence of IBD is increasing globally, bringing a heavy burden to public health care. Oral administration, as the most acceptable mode of administration for patients, can deliver most of the anticolitis drugs to the affected site and reduce the inconvenience caused by anal plugs.

The anti-colitis drugs may be a small molecule drug, including, but not limited to, budesonide, beclomethasone, 5-aminosalicylic acid, superoxide dismutase, 4-aminotempol, catalase, methotrexate, rolipram, adriamycin, vancomycin, colistin sulfate, suromycin, ramoplanin, LFF-571, berberine, bilirubin, gallic acid, catechol and derivatives thereof.

The anti-colitis drugs may be protein drugs, including, but not limited to, leukemia inhibitory factor, transforming growth factor β, ovalbumin and derivatives thereof.

The anti-colitis drugs may be small interfering ribonucleic acid (siRNA) and antisense oligonucleotides including, but not limited to, antisense oligonucleotides against tumor necrosis factor-a (TNF-α), abciximab-TNF messenger ribonucleic acid-15 (miRNA-155) inhibitor, TNF-α siRNA, CD98 siRNA, cyclin D1 siRNA, prolyl hydroxylase 2 siRNA, Map4k4 siRNA, cleavage oligonucleotide, IL10 producing plasmid, NF-kB decoy oligonucleotide, pcDNA3-EGFP, peptide antigen (PeptAg) and derivatives thereof.

The anti-colitis drugs may be probiotics, including, but not limited to, Lactobacillus casei (ATCC 39392), Lactococcus lactis, Bacillus ovale, Vibrio cholerae, probiotic VSL-3 or DNA isolated therefrom, Escherichia coli.

Colorectal cancer is one of the most common malignant tumors in the world. The treatment of colorectal cancer mainly relies on surgery, chemotherapy and immunotherapy. Currently, chemotherapy and immunotherapy are usually administered by intravenous injection. Although oral administration has the highest patient acceptance and compliance among different routes, clinically used chemotherapy products developed in oral preparations are rare. This is due to the limited absorption and instability of drugs in the gastrointestinal tract (GI), and the first-pass effect in the liver on drug metabolism, which are closely related to the reduction of the therapeutic anti-tumor effect of oral preparations. In addition, due to the short residence time and instability at the tumor site, small molecule drugs are poorly absorbed by cancer cells, and the systemic absorption and transport effects of oral preparations for colorectal cancer are negligible, and there is currently no clinically available chemotherapy. The present invention can increase the oral availability of the drugs.

The anti-colorectal cancer drugs may be bioalkylating agents, including, but not limited to, chlormethine hydrochloride, chlorambucil, melphalan, prenimustine, cyclophosphamide, thiotepa, carmustine, busulfan, cisplatin, carboplatin and the like and derivatives thereof. The anti-colorectal cancer drugs may be anti-metabolic drugs, including, but not limited to, fluorouracil, cytarabine, mercaptopurine, methotrexate and the like and derivatives thereof. The anti-colorectal cancer drugs may be anti-tumor antibiotics, including, but not limited to, actinomycin D, doxorubicin, zorubicin, mitoxantrone and the like and derivatives thereof. The anti-colorectal cancer drugs may be active ingredients of traditional Chinese medicines, including, but not limited to, natural medicine active ingredients such as 10-hydroxycamptothecin, vinblastine sulfate, paclitaxel, docetaxel, and derivatives thereof. The anti-colorectal cancer drug can be immune checkpoint inhibitors. See Table 3-1. The anti-colorectal cancer drugs may be cytokines, including, but not limited to, cytokines which are a class of small molecular proteins with a wide range of biological activities synthesized or secreted by immune cells (such as monocytes, macrophages, T cells, B cells, NK cells, etc.) and some non-immune cells (endothelial cells, epidermal cells, fibroblasts, etc.) upon stimulation. The cytokines include, but are not limited to, interleukins (ILs), interferons (IFNs), tumor necrosis factors (TNFs), colony stimulating factors (CSFs), chemokine family, growth factors (GFs), transforming growth factor-β family (TGF-β family). The interleukins include, but are not limited to, IL-1-IL-38. The colony-stimulating factors include, but are not limited to, G (granulocyte)-CSF, M (macrophage)-CSF, GM (granulocyte, macrophage)-CSF, Multi (multiple)-CSF (IL-3), SCF, EPO etc. The interferons include, but are not limited to, IFN-α, IFN-β, and IFN-γ. The tumor necrosis factors include, but are not limited to, TNF-α and TNF-β. The transforming growth factor-β family includes, but is not limited to, TGF-β1, TGF-β2, TGF-β3, TGFβ1β2, and bone morphogenetic proteins (BMPs). The growth factors include, but are not limited to, epidermal growth factor (EGF), platelet-derived growth factor (PDGF), fibroblast growth factor (FGF), hepatocyte growth factor (HGF), insulin-like growth factor-I (IGF-1), IGF-II, leukemia inhibitory factor (LIF), nerve growth factor (NGF), oncostatin M (OSM), platelet-derived endothelial cell growth factor (PDECGF), transforming growth factor-a (TGF-α), vascular endothelial cell growth factor (VEGF). The chemokine family includes, but is not limited to, four subfamilies: (1) CXC/α subfamily, mainly chemotactic neutrophils, the main members of which are IL-8, melanoma growth stimulating activity (GRO/MGSA), platelet factor-4 (PF-4), platelet basic protein, proteolysis-derived products CTAP-III and β-thromboglobulin, inflammatory protein 10 (IP-10), ENA-78. (2) CC/β subfamily, mainly chemotactic monocytes, the members of which include macrophage inflammatory protein 1α (MIP-1α), MIP-1β, RANTES, monocyte chemotactic protein-1 (MCP-1/MCAF), MCP-2, MCP-3 and I-309. (3) Type C subfamily, the representative of which is lymphotactin. (4) CX3C subfamily, Fractalkine, which is a CX3C type chemokine and has a chemotactic effect on monocytes-macrophages, T cells and NK cells. The cytokines include, but are not limited to, cytokines used to treat cancer and cytokines that reduce the side effects of cancer treatment. They play an important role in the normal immune response of the human body and the ability of the immune system to respond to cancer. The cytokines used to treat cancer include, but are not limited to, interferons and interleukins. The cytokines may also be hematopoietic growth factors, which reduce the side effects of cancer treatment by promoting the growth of blood cells destroyed by chemotherapy. The cytokines that reduce the side effects of cancer treatment include, but are not limited to, erythropoietin, IL-11, granulocyte-macrophage colony stimulating factor (GM-CSF) and granulocyte-colony stimulating factor (G-CSF). BCG Vaccine is a live vaccine made from a suspension of attenuated Mycobacterium bovis, which can increase the activity of macrophages, improve the body's cellular immunity, and be used to treat bladder cancer. Immunomodulatory drugs include, but are not limited to, thalidomide (Thalomid^{®}), lenalidomide (Revlimid^{®}), pomalidomide (Pomalyst^{®}) and imiquimod (Aldara^{®}, Zyclara^{®}).

### Oral mucosa

The oral mucosal administration system has many advantages compared with the traditional administration system: 1. a durable, constant and controllable blood drug concentration can be maintained, thereby reducing adverse reactions; 2. the first pass effect in the liver and the interference and degradation from gastrointestinal factors can be avoided to improve the bioavailability of drugs; 3. the pain of injection medication can be alleviated to improve patient compliance; 4. if there is a problem after the administration, drugs can be stopped in time, which is convenient to use; and 5. the number of administration and the dosage are reduced, and the incidence of adverse reactions of drugs is reduced. Therefore, the oral mucosal administration systems have received more and more attention.

However, due to the limited space for absorption of the oral mucosa, the drug delivery system should not be too bulky; involuntary saliva secretion and swallowing affect the efficacy of an oral mucosal pathway; the taste stimulation and foreign body sensation from drugs affect the compliance with the pathway; not all substances can pass through the oral mucosa, and their absorption is affected by fat solubility, pH, molecular weight and the like.

Oral mucosa is a barrier in the process of drug transport through the oral cavity. The properties of the membranes in different areas of the oral cavity are different, and the thickness and composition of the stratum corneum and non-stratum corneum tissues are also different. In addition, the thickness of the membrane at the absorption site, blood flow, blood or lymph perfusion, cell renewal, enzyme content, etc. are all related to the oral mucosal absorption of drugs. The oral mucosal barrier is composed of an epithelial barrier, a basement membrane barrier, and lamina propria. There is an epithelial barrier in the oral mucosal epithelium. The epithelial layer is the main barrier for drugs to pass through the mucosa. The permeability of the keratinized epithelium is greater than that of the non-keratinized epithelium. The main permeation barrier exists at 1/4 to 1/3 of the outermost epithelium, the thickness of the epithelium also has a great influence on the permeability, and the stratum corneum is the main absorption barrier. Although the superficial part of oral epithelial cells can prevent the entry of foreign substances, the basement membrane located at the junction of epithelial cells and lamina propria can also restrict the penetration of drugs. The lipophilicity of a drug should not be too strong, otherwise the drug will be difficult to dissolve in saliva, failing to reach an effective level, and will be difficult to pass through the basement membrane, affecting the transmembrane absorption of the drug. It is generally believed that the lamina propria does not function as a permeation barrier. Blood vessels and nerves are in the lamina propria to provide nutrition and innervation for the epithelium. However, some researchers believe that the lamina propria part will also constitute a certain permeation barrier. Using fluorinated chitosan as a carrier can effectively overcome the epithelial barrier, stratum corneum barrier, and permeation barrier related to the oral mucosa, to effectively increase the penetration rate of drugs, facilitate drugs to actually reach the blood or the treatment site, and improve the availability of drugs.

Partial therapeutic effect of oral mucosal administration after absorption into the blood is similar to the effect of nasal mucosal administration. Preparations include mouthwashs, lozenges, sublingual tablets, films and other dosage forms. For example, it can treat diseases such as herpetic trigeminal/glossopharyngeal neuralgia, inflammations, pharyngitis caused by bacteria and viruses, oral ulcers, local anesthesia, etc. In addition, there are many sublingual venous plexuses, which can cause rapid absorption into the blood after the mucosa penetration, avoiding the liver first-pass effect, and thus, oral mucosal administration is mostly used for heart-related diseases. Drugs related to heart diseases may include, but are not limited to, specific drugs and derivatives thereof in Table 3-2 below.

**Table 3-2 shows drugs related to heart diseases**

| | |
|---|---|
| Anti-angina drugs | nitroglycerin, molsidomine, nitroprusside, isosorbide, propranolol, metoprolol, labetalol and the like and derivatives thereof |
| Antihypertensive drugs | methyldopa, clonidine, mecamylamine, reserpine, phentolamine, trimazosin, prazosin hydrochloride, pinacidil, captopril, enalapril maleate, losartan, nifedipine, amlodipine, verapamil, diltiazem, flunarizine and the like and derivatives thereof |
| Antiarrhythmic drugs | quinidine, procainamide, lidocaine, propafenone, amiodarone, verapamil and the like and derivatives thereof |
| Cardiotonic drugs | digoxin, amrinone, milrinone, pimobendan, dobutamine and the like and derivatives thereof |
| Lipid-regulating drugs | clofibrate, gemfibrozil, hydrochloric acid, mevastatin, lovastatin and the like and derivatives thereof |

Any disease or dysfunction, especially body injury, may cause a person to feel pain. In many cases, analgesia treatment is needed. Analgesics are a class of drugs that act on the central nervous system and selectively inhibit the pain center, reducing or eliminating pain without affecting other sensations. They are mainly used clinically to relieve severe pain caused by trauma, burns, post-operation and cancer. However, many analgesics can not quickly relieve pain and be administered locally. The present invention can solve this problem.

The analgesics may be morphine and derivatives thereof, including, but not limited to, codeine, ethylmorphine, benzylmorphine, iso-codeine, heroin, phenethylmorphine, hydromorphone, oxymorphone, nalorphine, nalbuphine, naloxone, naltrexone and the like and derivatives thereof.

The analgesics may be synthetic analgesics, including, but not limited to, pethidine, anileridine, phenoperidine, piminodine, alphaprodine, betaprodine, fentanyl, Alfentanil, sufentanil, remifentanil, pethidine hydrochloride, methadone, dextromoramide, dextropropoxyphene, methadone hydrochloride, N-methylmorphinan, levorphanol, butorphanol, morphinan, pentazocine, dezocine, sumatriptan, tramadol, pizotifen, nefopam.

Anesthetics refer to drugs that can cause temporary and reversible loss of consciousness and pain in the entire body or a part of the body of a patient. However, most of the anesthetics are intravenously injected and intraspinally injected. These two modes of administration increase the pain of patients compared to oral administration. The present invention can increase patient compliance through oral administration of anesthetics.

The anesthetics may be intravenously injected anesthetics, including, but not limited to, ketamine hydrochloride, propofol, sodium thiopental, etomidate, midazolam and sodium gamma-hydroxybutyrate.

The anesthetics may be local anesthetics, including, but not limited to, aromatic acid esters, aromatic amides, amino ketones, amino ethers, carbamates, hydroxyprocaine, chloroprocaine, tetracaine, butacaine, thiocaine, procainamide, bupivacaine, articaine, etidocaine, ropivacaine, mepivacaine, clonin hydrochloride and the like and derivatives thereof.

Inflammation is closely related to the occurrence and development of many major diseases such as cardiovascular and cerebrovascular diseases, neurodegenerative diseases and even tumors. Therefore, early imaging, diagnosis and anti-inflammatory strategies for inflammation are important means to prevent and treat many diseases. At present, most anti-inflammatory drugs are administered intravenously and orally. However, intravenous injection of anti-inflammatory drugs can have serious side effects, and oral administration of anti-inflammatory drugs results in extremely low bioavailability. The present invention can improve the oral availability of anti-inflammatory drugs, and enable patients to have higher compliance.

The anti-inflammatory drugs may be non-steroidal anti-inflammatory drugs, including, but not limited to, aspirin, acetaminophen, non-specific cyclooxygenase inhibitors such as antipyrine, metamizole sodium, phenylbutazone, oxyphenbutazone, mefenamic acid, indometacin, sulindac, diclofenac sodium, ibuprofen, naproxen, piroxicam, meloxicam and derivatives thereof.

The anti-inflammatory drugs may be steroidal anti-inflammatory drugs, including, but not limited to, hydrocortisone, corticosterone, aldosterone, triamcinolone, prednisolone, dexamethasone acetate, methylprednisolone aceponate and the like and derivatives thereof.

The embodiments of the present invention can be used to treat various inflammations, including, but not limited to, myocarditis, arthritis, colitis, tonsillitis, gastritis, rhinitis, periodontitis, enteritis, pharyngitis, prostatitis, vaginitis, cervicitis, scapulohumeral periarthritis, cervical spondylosis, bursitis, dermatitis, conjunctivitis, otitis media, etc.

Similarly, oral delivery vaccines may be coated with a protective shell to deliver antigens to the intestine, to bind to the intestinal-associated lymphoid tissues, to be captured and recognized by intestinal antigen-presenting cells and migrate to Peyer's lymph nodes, triggering an immune response, or may also be directly contained in the mouth for oral vaccine delivery. Oral vaccines include but are not limited to: live attenuated polio vaccine, rotavirus vaccine, typhoid vaccine, live dysentery vaccine, enteric typhoid vaccine, pertussis vaccine, rabies vaccine, diphtheria toxoid vaccine, tetanus toxoid vaccine, Cholera toxin B subunit vaccine, E. coli enterotoxin vaccine, 3-O deacylated monophosphoryl lipid A vaccine, BCG polysaccharide nucleic acid vaccine, bacterial cell wall skeleton vaccine.

The technical solution of the present invention can be used in an inhalation (pulmonary inhaler/nebulizer) administration system, as follows.

With fluorinated chitosan as the main body, bound drugs can also be prepared into corresponding nebulization preparations/inhalation preparations to promote administration of the drugs across the lung mucosa.

Although intravenous injection is a conventional treatment route, it is still difficult to effectively deliver drugs to the lung tissues. The drugs in intravenous injection cannot be effectively deposited in the lungs and can easily cause side effects in other parts of the body. The absorption area of the alveoli is large, and the permeability of the alveolar epithelial cells is high; direct pulmonary administration by means of nebulization preparations can increase the local drug concentration in the lung tissue, reduce drug loss, accurately quantify drug dosage, and reduce toxic and side effects. However, macromolecules with large molecular weight, hydrophilicity, and negative charge, such as proteins and nucleic acids, are still difficult to penetrate the lung barrier. Some negatively charged hydrophilic macromolecules are easily degraded by nucleases, and are difficult to penetrate the biomembrane to reach the site of action. The drug has a short residence time in the lungs and low bioavailability, and cannot effectively control the progression of lung diseases and improve the prognosis. The technical solution of the present invention takes perfluoroheptanoic acid-modified chitosan as an example to serve as a novel transpulmonary mucosal carrier. Perfluoroheptanoic acid-modified chitosan can effectively protect the activity of protein drugs and increase the retention time of drugs in the lungs, and effectively improve the bioavailability of pulmonary medical mists, thereby improving the therapeutic effect of pulmonary administration. Nebulization preparations include, but are not limited to, aerosols, dry powder inhalers and sprays. Inhalation preparations include liquid (such as inhalation aerosols and nebulized inhalation solutions) or solid preparations (such as dry powder inhalers) delivered to the lungs in the form of a vapor or an aerosol. The drugs used include, but are not limited to, local therapeutic drugs, antibiotic drugs, antiviral drugs, antitumor drugs, protein and polypeptide drugs, and the like.

The drugs used in nebulization preparations may be anti-inflammatory drugs, including, but not limited to non-steroidal drugs and steroidal drugs. Non-steroidal drugs include, but are not limited to, aspirin, acetaminophen, diclofenac, indomethacin, ibuprofen, fenbufen and the like and derivatives thereof in various dosage forms. Steroidal drugs include, but are not limited to, adrenocortical hormone drugs, namely, glucocorticoid drugs and the like and derivatives thereof in various dosage forms.

The drugs used in nebulization preparations may be antibiotics. Antibiotics include but are not limited to β-lactams, aminoglycosides, macrolides, polypeptides, lincosamides, tetracyclines, amide alcohols/chloram phenicols, rifamycins and other antibiotics. β-lactams include, but are not limited to, penicillin drugs (penicillin G, procaine penicillin, penicillin V, benzathine penicillin, oxacillin, cloxacillin, flucloxacillin, amoxicillin, ampicillin, piperacillin, mezlocillin, azlocillin, amoxicillin and clavulanate potassium, piperacillin sodium and tazobactam sodium, ampicillin sodium and sulbactam sodium), cephalosporins (cephalothin, cefazolin, cefathiamidine, cefalexin, cefadroxil, cefradine, cefuroxime, cefamandole, cefotiam, cefonicid, cefuroxime axetil, cefaclor, cefprozil, cefotaxime, ceftazidime, cefoperazone, cefazoxime, ceftriaxone, cefixime, cefodizime , cefmenoxime, cefteram, ceftibuten, cefdinir, cefpiramide, cefpirome, cefepime, cefoperazone sodium and sulbactam sodium) and derivatives thereof in various dosage forms. Atypical β-lactams (cephamycins such as cefoxitin, cefmetazole, cefotetan, cefminox, cefbuperazone, monocyclic β-lactams such as aztreonam, carumonam, carbapenems such as imipenem, meropenem, panipenem, faropenem, ertapenem, biapenem, doripenem, and oxycephalosporins such as latamoxef and flomoxef) and derivatives thereof in various dosage forms. Aminoglycosides include but are not limited to etimicin, streptomycin, gentamicin, kanamycin, amikacin, tobramycin, netilmicin, spectinomycin, isepamicin, neomycin, paromomycin, kasugamycin, micronomicin, sisomicin, ribostamycin and derivatives thereof in various dosage forms. Macrolides include, but are not limited to, erythromycin, azithromycin, clarithromycin, roxithromycin, telithromycin, dirithromycin, midecamycin, acetylmidecamycin, kitasamycin, and acetylkitasamycin, acetylspiramycin, spiramycin, oleandomycin, tylosin, josamycin, rosamycin, erythromycin ethylsuccinate, tilmicosin, kitasamycin and derivatives thereof in various dosage forms. Polypeptides include, but are not limited to, vancomycin, norvancomycin, teicoplanin, bleomycin, polymyxin B, polymyxin E, bacitracin and derivatives thereof in various dosage forms. Lincosamides include, but are not limited to, lincomycin, clindamycin and derivatives thereof in various dosage forms. Tetracyclines include, but are not limited to, doxycycline, minocycline, tetracycline, chlortetracycline, oxytetracycline, demeclocycline, doxycycline, tigecycline and derivatives thereof in various dosage forms. Amide alcohols/chloram phenicols include, but are not limited to, chloramphenicol, thiamphenicol, chloramphenicol palmitate, chloramphenicol succinate, florfenicol and derivatives thereof in various dosage forms. Rifamycins include, but are not limited to, rifampicin, rifapentine, rifabutin, rifamycin sodium and derivatives thereof in various dosage forms, other antibiotics such as fosfomycin, nystatin and derivatives thereof in various dosage forms.

The drugs used in nebulization preparations may be antiviral drugs, including, but not limited to sulfonamides and antibacterial synergists, quinolone antibacterial drugs, antituberculosis drugs, antifungal drugs, antiviral drugs, anti-AIDS drugs and antiparasitic drugs. Sulfonamide drugs include but are not limited to sulfamethoxypyridazine, sulfasalazine, sulphadimethoxine, sulfadimoxine, sulfadimethoxine, sulfadiazine sodium, sulfisoxazole, sulfadimidine, sulfisomidine, sulphadimidine sodium, sulfamethoxydiazine, sulfamonomethoxine, sulfamethoxypyridazine, sulfapiridazin sodium, sulfamethoxypyrazine, sulfamethoxazole, sulfachloropyridazine, sulfachlorpyridazine sodium, sulfaphenazolum, sulfachoropyrazine sodium, sulfaquinoxaline, sulfaquinoxaline sodium, sulfathiazole and the like and derivatives thereof in various dosage forms. Antibacterial synergists include, but are not limited to, trimethoprim (TMP), diaveridine (DVD), ormetoprim (OMP) and the like and derivatives thereof in various dosage forms. Quinolone antibacterial drugs include but are not limited to nalidixic acid, pipemidic acid, norfloxacin, ofloxacin, levofloxacin, pefloxacin, enoxacin, ciprofloxacin, lomefloxacin, fleroxacin, sparfloxacin, enrofloxacin, gatifloxacin, moxifloxacin, pazufloxacin and the like and derivatives thereof in various dosage forms. Antituberculosis drugs include but are not limited to crotoniazide, protionamide, ethionamide, Yunnan Baiyao, rifapentine, rifampicin-isoniazid (Rifinah), isoniazid-rifampicin-pyrazinamide (Rifater), rifampicin and isoniazid, rifampicin (Benemicin, Rifasynt, Syntoren), rifamycin sodium (Feing Li Fu), rifamycin sodium, pyrazinamide, furilazone, Prunella vulgaris, Allicin, Rifasynt, isoniazide para-aminosalicylate (He Qing), sodium para-aminosalicylate, pasiniazid, isoniazid, ftivazide, rifampinand isoniazid, isoniazid rifampicin and pyrazinamide (Dai Fei Lin), thioacetazone, capreomycin sulfate, streptomycin sulfate, para-aminosalicylate sod. (p-aminosalicylic acid, antituberculosis sodium, paramisan sodium), Bletilla striata, ethambutol hydrochloride, Lysionotin (Nevadensin), Mycobacterium Phlei F.U.36 (Utilin "S" injection), acediasulfone sodium, streptomycin (streptomycin sulfate) and the like and derivatives thereof in various dosage forms. Antifungal drugs include but are not limited to the following two types of drugs and derivatives thereof in various dosage forms: the first type is antibiotics, mainly griseofulvin, nystatin and amphotericin B; the other type is synthetic drugs, including imidazole drugs (such as clotrimazole, econazole, miconazole and ketoconazole, etc.), flucytosine and allylamine derivatives. Antiviral drugs include, but are not limited to, idoxuridine (Idexur, IDU), trifluorothymidine (TFT), vidarabine (Ara-A), ribavirin (RBV), aciclovir (ACV), ganciclovir (DHPG), azidothymidine (AZT), dideoxyinosine (DDI), amantadine, rimantadine, moroxydine (Virugon), ftibamzone, foscarnet (PFA), isoprinosine and the like and derivatives thereof in various dosage forms. Anti-AIDS drugs include but are not limited to nucleoside reverse transcriptase inhibitors (NRTIs), non-nucleoside reverse transcriptase inhibitors (NNRTIs), protease inhibitors (PIs), integrase inhibitors (raltegravir), fusion inhibitors (FIs) and CCRS inhibitors (maraviroc) and the like and derivatives thereof in various dosage forms. Antiparasitic drugs include, but are not limited to, antiprotozoal drugs, anthelmintic drugs, insecticides and the like and derivatives thereof in various dosage forms.

The drugs used in nebulization preparations may be anti-tumor drugs, specifically as described in Table 3-3.

As described in Example 3-3, fluorinated chitosan can be used as a drug carrier to deliver antibody drugs such as programmed cell death-ligand 1 antibodies for pulmonary administration in the form of inhalants/sprays.

The technical solution of the present invention can be used in a nasal administration system, specifically as follows.

With fluorinated chitosan as the main body, bound drugs can also be prepared into corresponding nasal drops to promote administration of the drugs across the nasal mucosa.

The nasal mucosa covers the surface of the nasal cavity, and cartilage, bone or skeletal muscle is beneath the mucosa. According to the difference in structure and function, the nasal mucosa is further divided into three regions: vestibular region, respiratory region and olfactory region. The vestibular region is a region adjacent to the anterior naris, which is rich in vibrissa to block the inhalation of larger dust particles in the air. The respiratory region occupies most of the nasal mucosa and has developed epithelial cilia, which can swing to the pharynx, where the mucus with dust particles and bacteria is discharged to the pharynx, eventually expelling them from the body. In addition, this region is rich in blood vessels and glands, which can warm and moisturize the inhaled air. The olfactory region occupies a small area of the mucosa, mainly located at the top of the nasal cavity. It contains olfactory cells and olfactory glands that specialize in the sense of smell, which secrete odorous particles that can dissolve and reach the olfactory region to stimulate the olfactory hairs on the surface of the olfactory cells to produce olfactory. Moreover, because the olfactory cells have different receptors, they can accept the stimulation of different chemical molecules respectively, so different senses of smell can be generated. The human nasal mucosa has a total surface area of about 150 cm², has many microvilli on the epithelial cells thereof, distributed with a large number of capillaries and lymphatic vessels, which enlarges the drug absorption area and accelerates the drug absorption. The existing preparations for nasal administration include drops, odorants, ointments, nasal suppositories, inhalants, sprays, powder aerosols and the like. The mucosa in the upper part of the nasal cavity is thicker than the mucosa in the bottom of the nasal cavity and in the sinuses, has dense blood vessels, and serves as the main place for drug absorption. As a new transmucosal carrier, the product of the present invention can increase the retention time of drugs in the nasal mucosa, reduce the entry of drugs into the oral cavity, increase the availability, and accelerate drugs to penetrate the mucosa into the blood to reach the lesion site for treatment. Herein, bypassing the blood-brain barrier, administering through the nasal mucosa to treat brain diseases is an example of the present invention. The nasal mucosa is rich in olfactory cells. The central processes of olfactory cells form olfactory nerve fibers, from which the olfactory filaments extend. The olfactory filaments terminate in the olfactory bulb of the olfactory neurons and directly reach the brain along the olfactory nerves of the olfactory bulb. After nasal administration, the drug carrier of the present invention can extend the residence time of the drug and increase the bioavailability, and be transfered to the brain by the olfactory cells, bypassing the blood-brain barrier for drug delivery. The trigeminal nerve in the nasal cavity is also considered to be one of the pathways to target the central nervous system for nasal administration. Therefore, by use of this pathway, the present invention enables drugs to penetrate the nasal mucosa to treat brain diseases such as trigeminal neuralgia, and can be used in the fields of preparing vaccines for nasal mucosa, local anesthesia of nasal mucosa and the like.

Drugs for treating cerebral stroke may include, but are not limited to, specific drugs and derivatives thereof in Table 3-4 below.

**Table 3-4**

| | |
|---|---|
| Vasodilators | persantine, sodium nitroprusside, urapidil, phentolamine, chlorpromazine, salbutamol, mecamylamine, captopril, losartan, nifedipine and the like and derivatives thereof |
| Drugs that improve microcirculation and expand blood | improve Hydroxyethyl starch, dextran, gelatin, fluorocarbons and the like and derivatives thereof |
| Thrombolytic drugs | urokinase, streptokinase, lumbrokinase, staphylokinase, anisoylated plasminogen-streptokinase activator complex (APSAC), snake venom antithrombotic enzyme, alteplase, reteplase and the like and derivatives thereof |
| Anticoagulant therapy | heparin, warfarin, activated antithrombin, recombinant hirudin, activated protein C, antithrombin and the like and derivatives thereof |
| Calcium ion antagonists | nimodipine, labetalol, metoprolol and the like and derivatives thereof |
| Drugs to prevent platelet aggregation | aspirin, clopidogrel, ozagrel and the like and derivatives thereof |
| Traditional Chinese medicines | Effective components of traditional Chinese medicines for promoting blood circulation and removing blood stasis and Chinese patent medicines thereof |

Anti-tumor drugs may include, but are not limited to, specific drugs and derivatives thereof in Table 3-1 below.

**Table 3-1**

| | | |
|---|---|---|
| Hormonal drugs | Estrogen drugs | steroid estrogens such as quinestrol, ethinylestradiol, nilestriol, estradiol benzoate, estradiol 3,17-dipropionate, estradiol valerate, estradiol 17-cyclopentanoate, mestranol, and derivatives thereof |
| | | non-steroidal hormones and derivatives thereof such as diethylstilbestrol and derivatives thereof |
| | | anti-estrogens such as fulvestrant, aminoglutethimide, formestane, anastrozole, letrozole, exemestane, clomiphene, tamoxifen, toremifene, and derivatives thereof |
| | Androgen drugs | androgen drugs such as methyltestosterone, testosterone propionate, and derivatives thereof |
| | | anabolic hormones such as oxymetholone, stanozolol, nandrolone phenylpropionate, metandienone, and derivatives thereof |

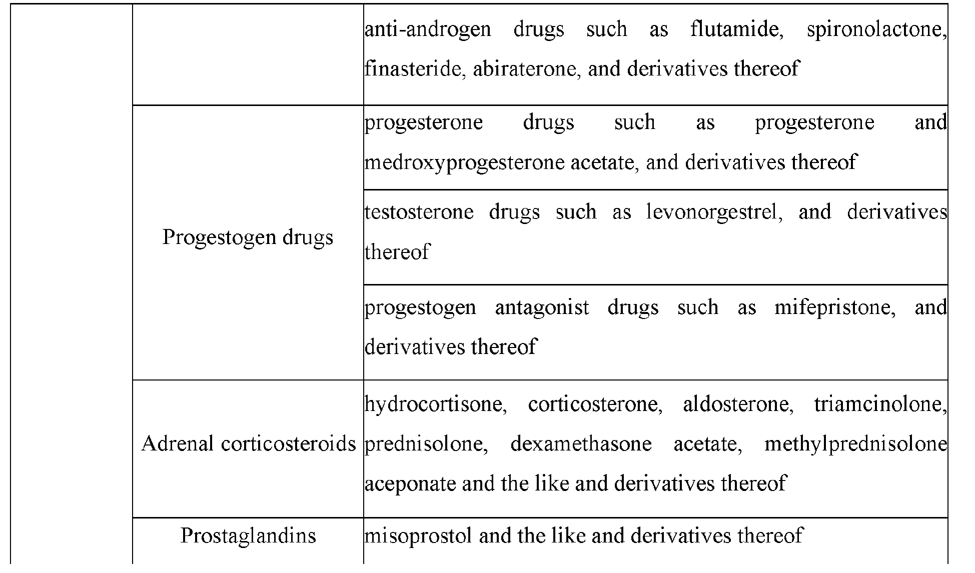

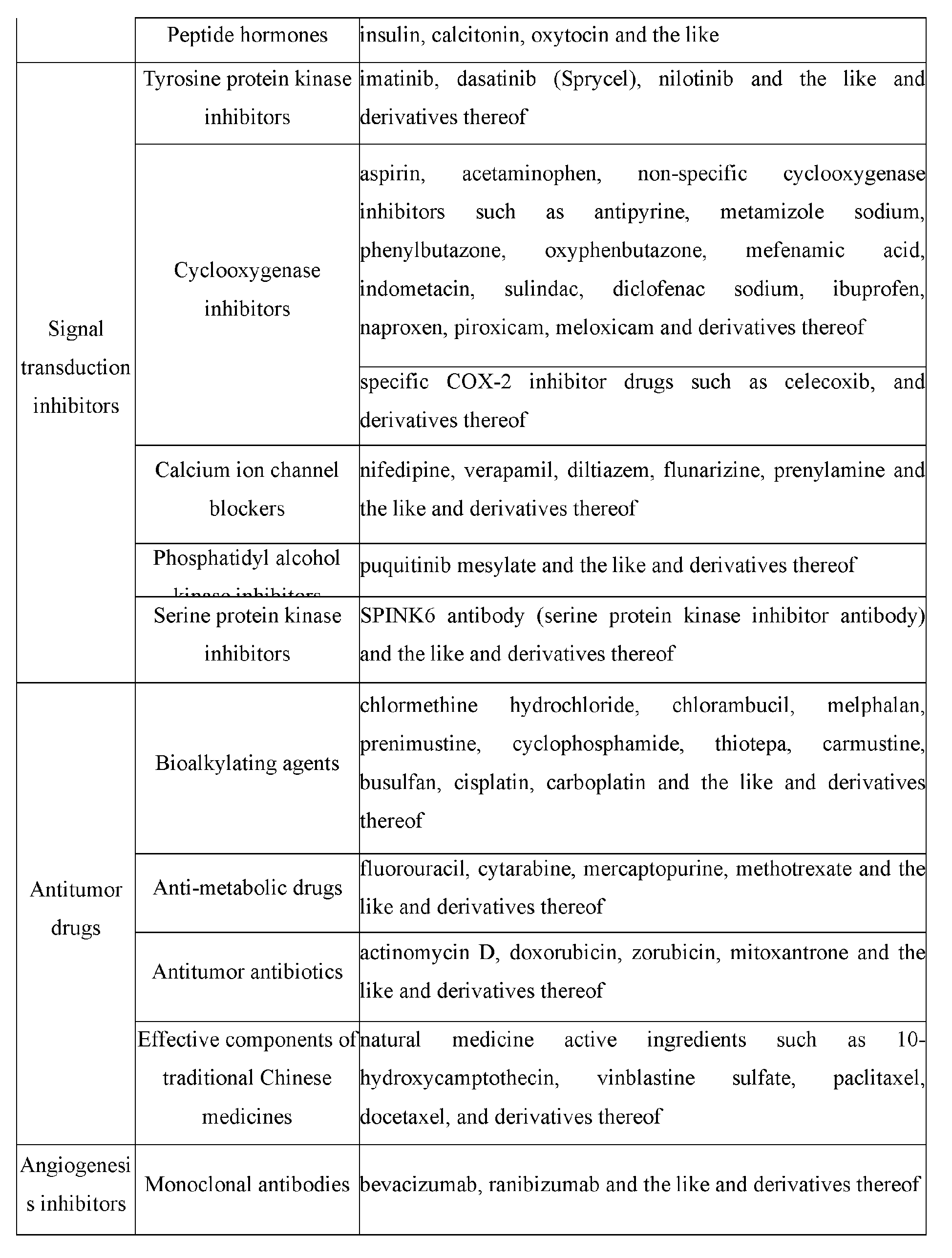

| | | |
|---|---|---|
| | Small molecule inhibitors | sorafenib, sunitinib, vandetanib, vatalanib and the like and derivatives thereof |
| | Angiopoietin signaling pathway inhibitors | Angiopoietin signaling pathway inhibitors and derivatives thereof |
| Immunother apy drugs | Checkpoint inhibitors | CTLA4 monoclonal antibody (Ipilimumab), PD-1 monoclonal antibody (Pembrolizumab, Nivolumab, Arezolizumab, Avelumab, Durvelumab), PD-LI monoclonal antibody (Atezolizumab, Avelumab, Durvalumab), LAG-3 (lymphocyte activation gene 3) monoclonal antibody, TIM-3 (T-cell immunoglobulin and mucin-domain containing-3) monoclonal antibody, TIGIT (T cell immunoglobulin and ITIM domain protein) monoclonal antibody, costimulatory factors B7-H3, B7 -H4 and B7-H5 monoclonal antibodies and the like and derivatives thereof |
| | Cytokines | IFNa2b, IFNa2a, IL-2, etc. |
| | Engineered T cells | CD19-specific CART cells, etc. |
| | Oncolytic viruses | recombinant human papgm-csf activated HSV genes, etc. |
| | Bispecific antibodies | CD19 and CD3b specific antibodies, etc. |

Nasal mucosal administration via the trigeminal nerve route can also be used for the treatment of herpetic trigeminal/glossopharyngeal neuralgia. Examples include, but are not limited to, ketamine, desipramine, carbamazepine and the like and derivatives thereof.

Nasal mucosal administration can also treat other diseases. For example, anti-inflammatory drugs, antibiotics, and antiviral drugs are used to treat rhinitis, inflammation of the throat and lungs. See the nebulization preparation section for example drugs.

Nasal mucosal administration penetrates the mucosa to exert a local effect or enters the blood to exert a systemic effect. For example, when applied to anesthesia, example drugs of general anesthesia include but are not limited to ketamine hydrochloride, propofol, sodium thiopental, etomidate, midazolam and sodium gamma-hydroxybutyrate. Local anesthetics include, but are not limited to, aromatic acid esters, aromatic amides, amino ketones, amino ethers, carbamates, hydroxyprocaine, chloroprocaine, tetracaine, butacaine, thiocaine, procainamide, bupivacaine, articaine, etidocaine, ropivacaine, mepivacaine, clonin and the like and derivatives thereof.

Any disease or dysfunction, especially body injury, may cause a person to feel pain. In many cases, analgesia treatment is needed. Analgesics are a class of drugs that act on the central nervous system and selectively inhibit the pain center, reducing or eliminating pain without affecting other sensations. They are mainly used clinically to relieve severe pain caused by trauma, burns, post-operation and cancer.

The analgesics may be morphine and derivatives thereof, including, but not limited to, codeine, ethylmorphine, benzylmorphine, iso-codeine, heroin, phenethylmorphine, hydromorphone, oxymorphone, nalorphine, nalbuphine, naloxone, naltrexone and the like and derivatives thereof. The analgesics may be synthetic analgesics, including, but not limited to, pethidine, anileridine, phenoperidine, piminodine, alphaprodine, betaprodine, fentanyl, Alfentanil, sufentanil, remifentanil, pethidine hydrochloride, methadone, dextromoramide, dextropropoxyphene, methadone hydrochloride, N-methylmorphinan, levorphanol, butorphanol, morphinan, pentazocine, dezocine, sumatriptan, tramadol, pizotifen, nefopam. The drugs may be diabetes treatment drugs including, but not limited to, sulfonylureas such as carbutamide, tolbutamide, chlorpropamide, acetohexamide, gliclazide, glipyride, glimepiride, and derivatives thereof; non-sulfonylureas such as repaglinide, nateglinide, and derivatives thereof; thiazolidinediones such as rosiglitazone, pioglitazone, and derivatives thereof; biguanides such as phenformin, metformin, and derivatives thereof; α-glucosidase inhibitors such as acarbose, voglibose, miglitol, and derivatives thereof; dipeptidyl peptidase-IV drugs such as glucagon-like peptide, DPP-IV inhibitors, sitagliptin, vildagliptin, and saxagliptin, and insulin and the like and derivatives thereof.

Also, pulmonary vaccine delivery can also be carried out by inhaling the spray vaccine through the nasal cavity. Nasal vaccines include but are not limited to: influenza vaccine, pertussis vaccine, tuberculosis spray vaccine, influenza spray vaccine, measles spray vaccine, Bordetella pertussis spray vaccine, Chlamydia pneumonia spray vaccine, Streptococcus pneumoniae spray vaccine, Bacillus anthracis spray vaccine, pneumococcal surface protein-nasal gel vaccine, botulinum toxin type A BoHc/A gel vaccine, tetanus toxoid gel vaccine.

As described in Example 3-4, fluorinated chitosan can be used as a drug carrier to deliver drugs from the nasal mucosa to treat brain tumor diseases.

Intravaginal administration is a route of administration where drugs are used in the vagina. In terms of pharmacology, compared with other routes of administration, intravaginal administration has the advantage of acting mainly in the vagina or nearby structures (e.g., the vaginal part of the cervix) to reduce systemic adverse reactions. The vagina is an efficient site for drug delivery, especially in terms of women's health. The vagina is usually an ideal route for drug administration, because compared with the oral administration route, the dosage in vaginal administration is lower, the drug level is stable, and the frequency of administration is lower. With vaginal administration, absorption is not affected by gastrointestinal diseases, and there is no first pass effect. Methods of vaginal administration include vaginal tablets, vaginal creams, vaginal suppositories and vaginal rings. Vaginal diseases are mostly caused by bacterial and viral infections. Example drugs are the same as antibacterial, antiviral drugs and anti-inflammatory drugs.

The technical solution of the present invention can be used in an ocular administration system, specifically as follows.

Diseases in which the treatment is restricted due to the ocular barrier penetration are mainly divided into three categories: ocular mucosal infections, cornea/conjunctiva-related infections, and fundus-related diseases.

The first category, ocular mucosal diseases, is mainly due to decreased ocular immunity, vitamin deficiency or accidental injury, which leads to damage and inflammation of the mucosa, or local tissue inflammation caused by intrusion of pathogenic microorganisms such as bacteria, viruses, fungi, parasites, amoeba and chlamydia into the human eye mucosa, such as eyelid infection, orbital infection, etc., which, if not treated in time, will further infect the cornea and conjunctiva of the eyeball, resulting in worsening of the condition. At present, clinically, the treatment methods such as simple medicated eye drops, eye gels, eye ointments, etc. are commonly used, but there are disadvantages such as extremely low drug availability, poor comfort for patients with gels and ointments, and poor transparency and visibility. In view of this, the present invention uses the fluorinated chitosan as a carrier to deliver drugs for treating ocular surface diseases such as trachoma, blepharitis, meibomian cyst, hordeolum, meibomianitis through the mucosa to the focal site for treatment. The treatment drugs are shown in Table 4-1.

The second category, cornea/conjunctiva-related infections, mainly includes keratitis and conjunctivitis from infection with various microorganisms, corneal/conjunctival neovascular diseases, and ocular allergies. The infection at these sites has the shortcomings of high pathogenicity, difficult treatment, troublesome treatment, being easy to attack, and poor patient compliance. Cornea/conjunctiva-related infections refer to local tissue inflammation caused by intrusion of pathogenic microorganisms such as bacteria, viruses, fungi, parasites, amoeba and chlamydia into the cornea and conjunctiva of the human eye, such as conjunctival inflammation, keratitis, and endophthalmitis. There are many reasons for infection, including poor eye hygiene; direct or indirect infection by an infected person; foreign body-induced infection in the cornea caused by dust entering the eyes; and mother-to-child transmission, and so on. The eyes are also often exposed to pathogenic microorganisms such as Siccibacter, Staphylococcus, Pneumococcus, Streptococcus, Pseudomonas aeruginosa, Escherichia coli, etc. Under normal circumstances, these bacteria are not disease-causing, because conjunctival and corneal barriers are intact and bacteria are not easy to invade. However, when decreased resistance and damage to the corneal and conjunctival epithelium occur in the eyes, there will be infections in different parts of the eye. Common pathogenic fungi in the eyes are, for example, Fusarium, Aspergillus, Penicillium, Candida albicans, etc.; common viruses in the eyes are adenoviruses, rubella viruses, herpes simplex virus, varicella-zoster virus, and enteroviruses, etc.; common chlamydia in the eyes is Chlamydia trachomatis, which often causes trachoma, conjunctivitis, and lymphogranuloma; Chlamydia psittaci, which often causes psittacosis, and so on. Although there are eye drops to treat related inflammations and to fight pathogens, once the inflammation goes deep into the eyeball, ordinary eye drops are almost helpless. Therefore, it is necessary to develop a strong barrier-penetrating material or carrier for administration and treatment, otherwise the treatment time is delayed and the bacteria may also become multi-drug resistant, which cause the infection to deepen and make the treatment more difficult. Targeted drugs are often used as therapeutic drugs, and see Table 4-1. The present invention uses fluorinated chitosan as a drug carrier. Because of the positive adsorption capacity of fluorinated chitosan, it can adhere to the surface of the cornea/conjunctiva, open the corneal/conjunctival epithelial cell channel, and deliver drugs related to the treatment of eye infections to the site of the corneal/conjunctival infection to inhibit or remove the microorganisms. It can also deliver ocular anti-inflammatory drugs to play anti-inflammatory and analgesic effects.

The third category, fundus diseases, is the most troublesome. To reach the lesion site, the drug needs to penetrate the multiple barriers of the eyeball. At present, intravitreal injection and subconjunctival or conjunctival sac administration are mainly used, all of which require repeated injections using a syringe, causing irreparable scars on the eyeballs, and affecting vision. For example, for fundus macular degeneration, the fundus macular area is an important area of the retina, which is related to visual functions such as fine vision and color vision. Once the macular area has lesions, vision loss, dark shadows in front of the eyes, or distortion of vision often occur. Fundus macular degeneration may be caused by genetic degeneration, age-related degeneration, inflammatory degeneration, etc., or may also be affected by other fundus diseases, and is troublesome to treat; age-related macular degeneration mainly includes changes such as age-related macular degeneration, age-related idiopathic epiretinal membrane and age-related macular holes, where the condition can be improved or stabilized by means of early diagnosis and appropriate treatment; inflammatory macular degeneration is more common in various retinal choroiditis, such as toxoplasmosis, uveitis, etc.; in addition, retinal vein occlusion, retinal vasculitis, diabetic retinopathy, high myopia, and traumatic choroidal rupture can cause damage to the macular area, which can lead to edema or hemorrhage in the macular area and also a certain degree of visual impairment. The current treatment is divided into surgical treatment and medical treatment. Surgical treatment mainly includes laser treatment, transpupillary thermotherapy, photodynamic therapy, surgical resection of new blood vessels, macular translocation, and retinal transplantation. However, patients bear greater pain and greater risk during the operation. Medical treatment is mainly intravital administration of anti-VEGF series of monoclonal antibodies. However, this can still cause conjunctival and retinal scars to affect vision, and also makes the patients bear greater pain and greater risk. Eye diseases and eye tumors bring great pain to patients. Eye tumors are divided into internal eye tumor and external eye tumor. The internal eye tumor is accompanied by yellow and white reflections in the pupils (commonly known as cat eyes), vision loss, elevated intraocular pressure, anterior chamber bleeding and other symptoms. The external eye tumor is manifest as local induration in the early stage, and can invade all eyelids, orbits and paranasal sinuses in the late stage, forming severe local tissue defects. Among infant eye diseases, it is the most serious and most harmful malignant tumor. It occurs in the nuclear layer of the retina and has a family genetic tendency. It mostly occurs at 5 years old and below. The disease is prone to intracranial and distant metastases, and often endangers the lives of children. Therefore, early detection, early diagnosis and early treatment are the keys to improve the cure rate and reduce the mortality rate. The current treatment methods are mainly surgical treatment including tumor resection, eyeball enucleation, evisceration of orbit; radiotherapy, using deep irradiation with deep X-ray and Co and the like, or shallow irradiation with P and Sr and the like, according to the conventional treatment of radiotherapy; comprehensive therapy, with the comprehensive use of traditional Chinese medicine, western medicine, radiotherapy and surgery; immunotherapy, using treatment with immunosuppressants to control tumor proliferation. Tumor treatment drugs and derivatives thereof are shown in Table 4-2. However, there is also the problem of the above-mentioned macular degeneration, and it is difficult for drugs to pass through the barrier for treatment.

The present invention provides a use of fluorinated chitosan in promoting the efficiency of drug absorption; and provides a fluorinated chitosan and use thereof as a variety of drug carriers.

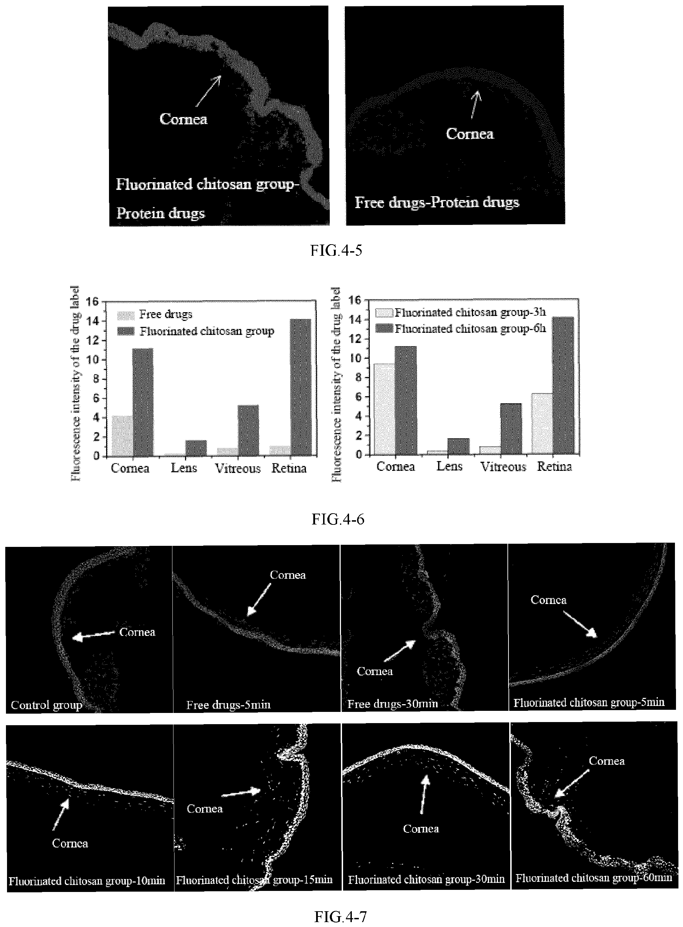

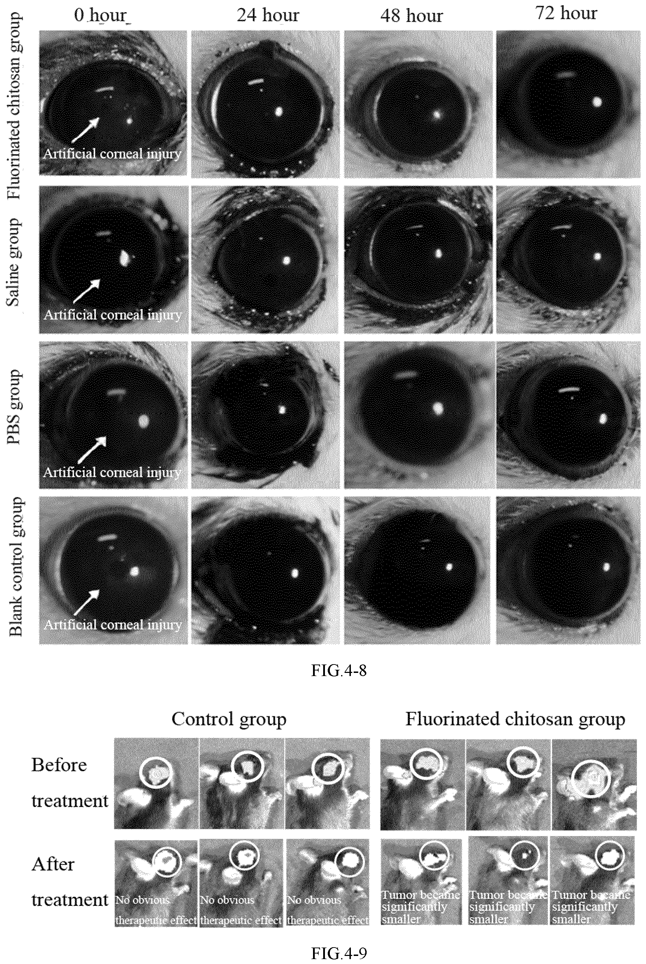

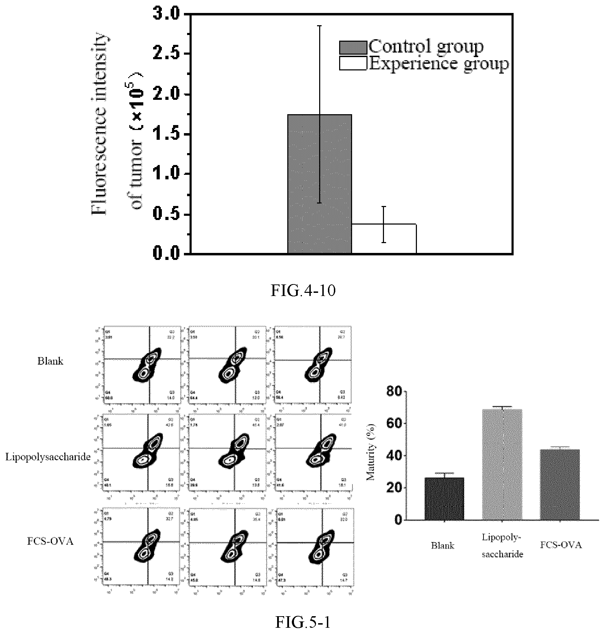

Further, the transdermal effect produced by the technical solution of the present invention is temporary. After the drug is removed, the stratum corneum cells of the skin will close the channel to protect the safety of the human body. The skin consists of epidermis and dermis. The epidermis is divided into stratum corneum, stratum lucidum, stratum granulosum and stratum germinativum in order from superficial to deep. The dermis is composed of dense connective tissues, and is divided into papillary layer and reticular layer in order from superficial to deep. The papillary layer is connected to the stratum germinativum of the epidermis, and contains abundant receptors such as capillaries, lymphatic vessels, nerve endings, and tactile corpuscles. The stratum corneum is the largest rate-limiting barrier for transdermal administration. The stratum corneum in most of the skin consists of 5-25 layers of flat keratinocytes. These cells have no nucleus and organelles, and have thicker cell membranes. They are lifeless and water-tight, and have the functions of preventing tissue fluid from flowing out, resisting friction and preventing infection. The fluorine-containing compound-modified cationic polymer can stimulate a change in the distribution of tight junction proteins in these cells to reduce tight junctions between cells, and further stimulate actin phosphorylation, thereby promoting the paracellular transport and opening the intercellular space to forms channels, so that the drug is carried through the stratum corneum and is further penetrated into the skin, and then into the dermis and into the skin capillaries and lymphatic circulation to exert the drug effect (see FIG. 4-1).

In summary, the fluorine-containing compound-modified cationic polymer provided by the present invention can be universally bind to a variety of drugs to promote drug absorption, improve drug bioavailability, and reduce toxicity, and has good effects, broad applicability, and great commercial value. In addition, the fluorine-containing compound-modified cationic polymer proposed by the present invention is easy to produce and has a commercial basis.

In the present invention, fluorinated chitosan and a drug are prepared into eye drops for administration, which can penetrate the ocular barrier and partially deliver the drug into the eye for a therapeutic effect.

Exemplary drugs may include, but are not limited to, specific drugs and derivatives thereof for eye-related diseases in Table 4-1 below.

**Table 4-1**

| | |
|---|---|
| Eye bacterial infection Treatment | penicillin drugs (penicillin G, procaine penicillin, penicillin V, benzathine penicillin, oxacillin, cloxacillin, flucloxacillin, amoxicillin, ampicillin, piperacillin, mezlocillin, azlocillin, amoxicillin and clavulanate potassium, piperacillin sodium and tazobactam sodium, ampicillin sodium and sulbactam sodium), cephalosporins (cephalothin, cefazolin, cefathiamidine, cefalexin, cefadroxil, cefradine, cefuroxime, cefamandole, cefotiam, cefonicid, cefuroxime axetil, cefaclor, cefprozil, cefotaxime, ceftazidime, cefoperazone, cefazoxime, ceftriaxone, cefixime, cefdizime, cefmenoxime, cefteram, ceftibuten, cefdinir, cefpiramide, cefpirome, cefepime, cefoperazone sodium and sulbactam sodium) and derivatives thereof in various dosage forms. Atypical β-lactams (cephalosporins such as cefoxitin, cefmetazole, cefotetan, cefminox, cefbuperazone, monocyclic β-lactams such as aztreonam, carumonam, carbapenems such as imipenem, meropenem, panipenem, faropenem, ertapenem, biapenem, doripenem, and oxycephalosporins such as latamoxef and flomoxef) and derivatives thereof in various dosage forms. Aminoglycosides include but are not limited to etimicin, streptomycin, gentamicin, kanamycin, amikacin, tobramycin, netilmicin, spectinomycin, isepamicin, neomycin, paromomycin, kasugamycin, micronomicin, sisomicin, ribostamycin and derivatives thereof in various dosage forms. Macrolides include, but are not limited to, erythromycin, azithromycin, clarithromycin, roxithromycin, telithromycin, dirithromycin, midecamycin, acetylmidecamycin, kitasamycin, and acetylkitasamycin, acetylspiramycin, spiramycin, oleandomycin, tylosin, josamycin, rosamycin, erythromycin ethylsuccinate, tilmicosin, kitasamycin and derivatives thereof in various dosage forms. Polypeptides include, but are not limited to, vancomycin, norvancomycin, teicoplanin, bleomycin, polymyxin B, polymyxin E, bacitracin and derivatives thereof in various dosage forms. Lincosamides include, but are not limited to, lincomycin, clindamycin and derivatives thereof in various dosage forms. Tetracyclines include, but are not limited to, doxycycline, minocycline, tetracycline, chlortetracycline, oxytetracycline, demeclocycline, doxycycline, tigecycline and derivatives thereof in various dosage forms. Amide alcohols/chloram phenicols include, but are not limited to, chloramphenicol, thiamphenicol, chloramphenicol palmitate, chloramphenicol succinate, florfenicol and derivatives thereof in various dosage forms. Rifamycins include, but are not limited to, rifampicin, rifapentine, rifabutin, rifamycin sodium and derivatives thereof in various dosage forms. Other antibiotics such as fosfomycin, nystatin and the like and derivatives thereof |
| Eye viral infection Treatment | Antiviral drugs include, but are not limited to, idoxuridine (Idexur, IDU), trifluorothymidine (TFT), vidarabine (Ara-A), ribavirin (RBV), aciclovir (ACV), ganciclovir (DHPG), azidothymidine (AZT), dideoxyinosine (DDI), amantadine, rimantadine, moroxydine (Virugon), ftibamzone, foscarnet (PFA), isoprinosine and the like and derivatives thereof. |
| Anti -inflammatory treatment | aspirin, acetaminophen, non-specific cyclooxygenase inhibitors such as antipyrine, metamizole sodium, phenylbutazone, oxyphenbutazone, mefenamic acid, indometacin, sulindac, diclofenac sodium, ibuprofen, naproxen, piroxicam, meloxicam and derivatives thereof; steroidal anti-inflammatory drugs such as hydrocortisone, corticosterone, aldosterone, triamcinolone, prednisolone, dexamethasone acetate, methylprednisolone aceponate, and derivatives thereof |
| Anti-angiogenic treatment | monoclonal drugs such as bevacizumab and ranibizumab, and derivatives thereof; sorafenib, sunitinib, vandetanib, vatalanib and the like and derivatives thereof; Angiopoietin signaling pathway inhibitors and derivatives thereof |
| Glaucoma treatment | bromoxidine, acetazolamide, dorzolamide hydrochloride, pilocarpine and the like and derivatives thereof |

Anti-tumor drugs may include, but are not limited to, specific drugs and derivatives thereof in Table 4-2 below.

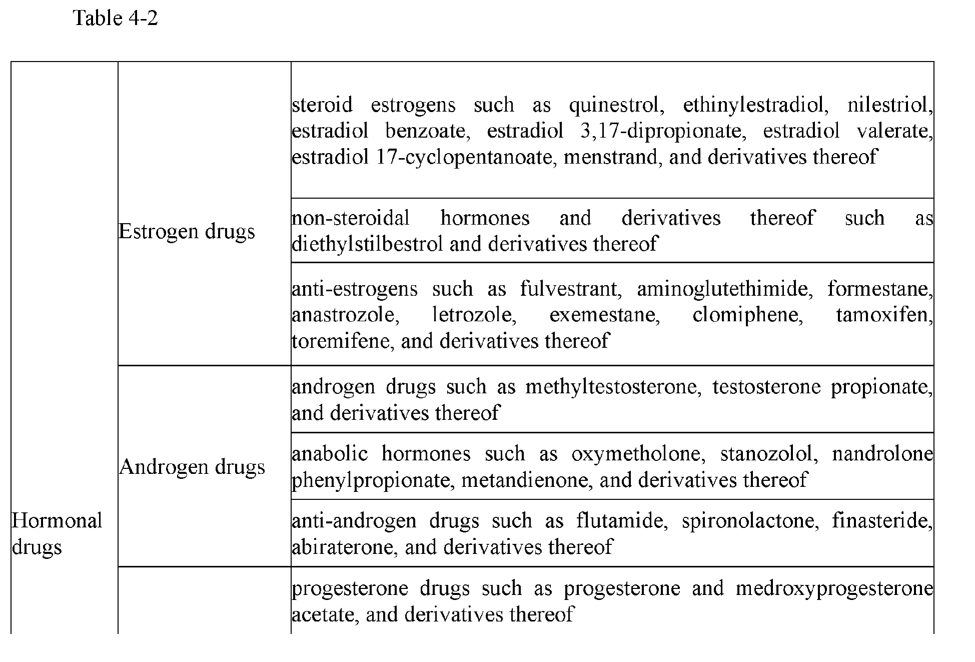

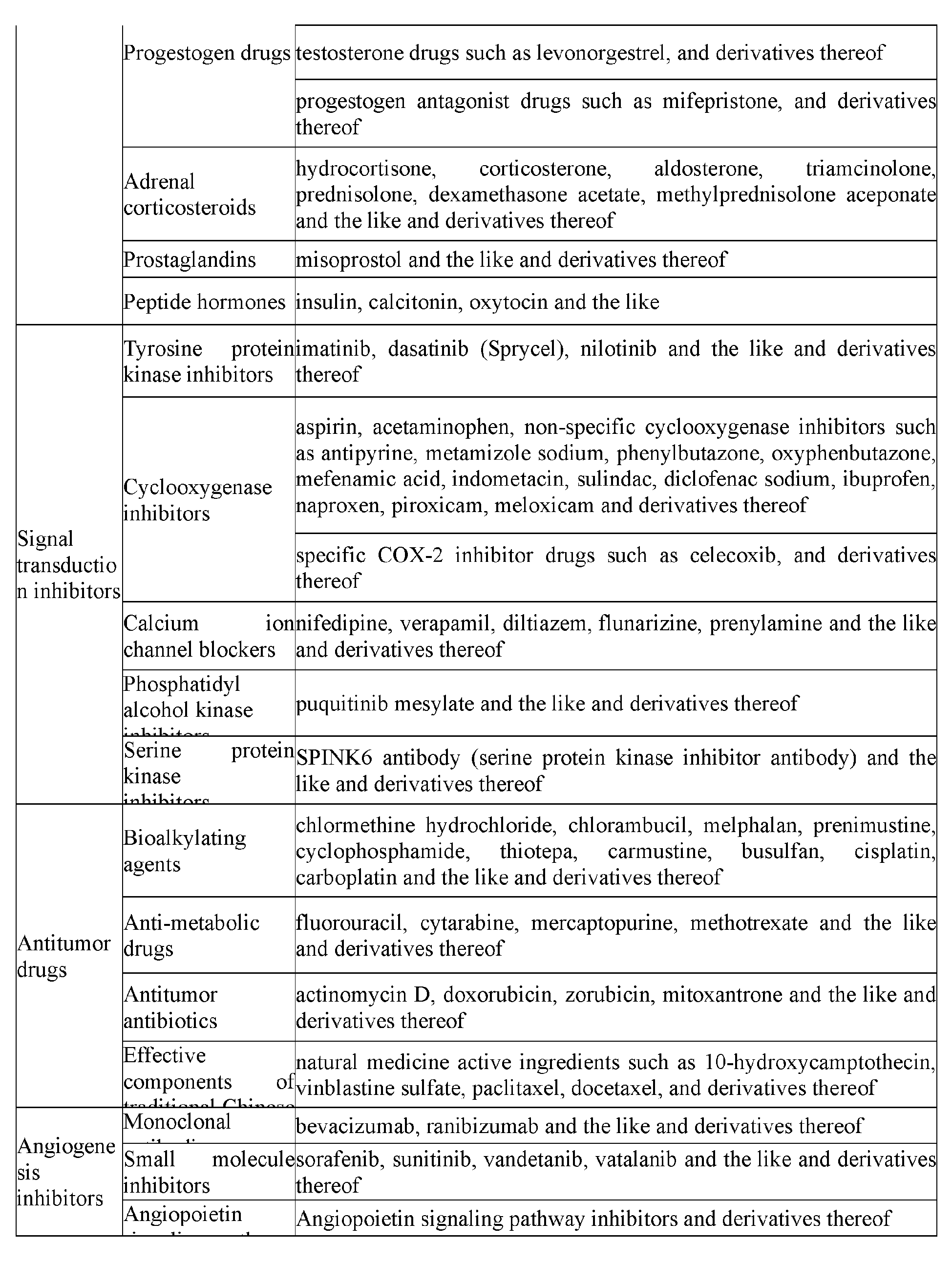

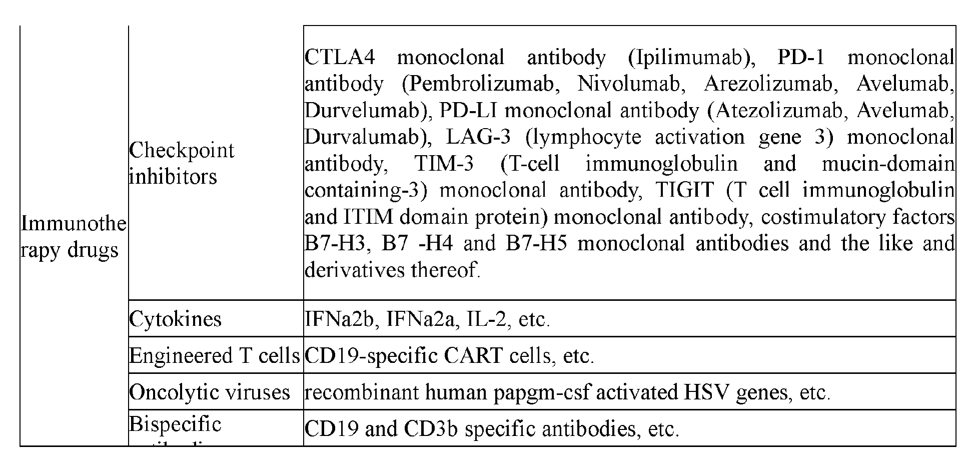

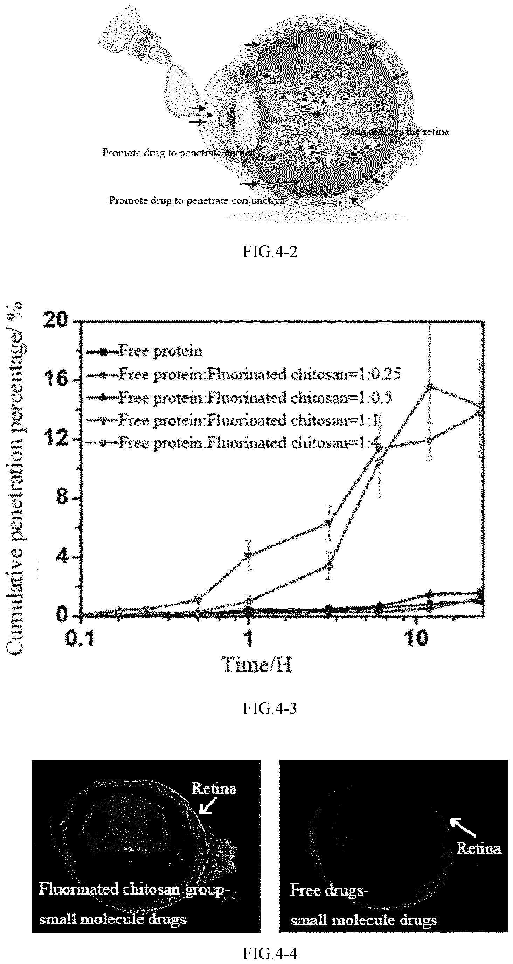

Next, the present invention relates to a use of a fluorine-containing compound-modified cationic polymer, especially fluorinated chitosan, as a drug carrier to penetrate the most important corneal barrier of the eyeball to achieve local drug treatment but treat intraocular or fundus diseases. The schematic diagram of mucosal penetration is shown in FIG. 4-2, where the black arrow represents the direction of drug permeation. When fluorinated chitosan binds to a drug to prepare an eye drop which is dropped on the ocular surface, fluorinated chitosan adheres to the surface of the cornea and conjunctiva due to positive charges, to increase the retention time of the drug, and open the intercellular channel protein to help the drug to penetrate the cornea and conjunctival barriers and penetrate the blood-retinal barrier, thereby treating related diseases.

Fluorinated chitosan can be used as a drug carrier to deliver therapeutic drugs into the eyes, and be administered to the cornea in the form of eye drops for the treatment of ocular diseases, such as cornea/conjunctiva-related inflammation, age-related macular degeneration, and retinal melanoma and so on.

In summary, by use of a fluorine-containing compound-modified chitosan as a drug carrier and drug delivery body, the present invention can increase the retention time of eye drops on the ocular surface, and open the eye-related barriers to deliver drugs to the lesion site for enrichment for therapeutic purposes, which not only has a good therapeutic effect for ocular mucosal infections and cornea/conjunctiva-related infections, but also has a good effect on fundus-related diseases.

Further, the drugs used to treat ocular neovascularization include bevacizumab, ranibizumab, sorafenib, sunitinib.

An antibiotic eye drop of the present invention using the above barrier-penetrating administration preparation, which is a composite formed by a drug for treating corneal bacterial infection and the fluorinated chitosan, the particle size range of the composite is less than 10 microns, or the particle size range of the composite is not greater than 500 nanometers, and the weight ratio of the fluorinated chitosan to the drug is 1:0.5-30.

Further, the drug for treating corneal bacterial infection and bacterial infection within the eyeball includes penicillin, erythromycin, cephalosporin, cephamycin, streptomycin, gentamicin, kanamycin, azithromycin, clarithromycin, roxithromycin, telithromycin, kitasamycin, vancomycin, norvancomycin, teicoplanin, bleomycin, polymyxin B, polymyxin E, bacitracin, lincomycin, clindamycin, doxycycline, minocycline, tetracycline, chlortetracycline, oxytetracycline, demeclocycline, doxycycline, tigecycline.

An antiviral eye drop of the present invention using the above barrier-penetrating administration preparation, which is a composite formed by a drug for treating corneal viral infection and the fluorinated chitosan, the particle size range of the composite is less than 10 microns, or the particle size range of the composite is not greater than 500 nm, and the weight ratio of the fluorinated chitosan to the drug is 1:0.5-50.

In an embodiment, the drug for treating corneal viral infection and viral infection within the eyeball includes idoxuridine, trifluorothymidine, vidarabine, ribavirin, aciclovir, ganciclovir, azidothymidine, dideoxyinosine, amantadine, rimantadine, moroxydine, ftibamzone, foscarnet, and isoprinosine.

The invention provides an anti-inflammatory eye drop of the present invention using the above barrier-penetrating administration preparation, which is a composite formed by an anti-inflammatory drug for treating eye inflammation caused by bacteria, viruses and injuries and the fluorinated chitosan, the particle size range of the composite is less than 10 microns, preferably not greater than 500 nm, and the weight ratio of the fluorinated chitosan to the drug is 1:0.5-50.

In an embodiment, the anti-inflammatory drug for treating eye inflammation caused by bacteria, viruses and injuries includes aspirin, acetaminophen, non-specific cyclooxygenase inhibitors such as antipyrine, metamizole sodium, phenylbutazone, oxyphenbutazone, mefenamic acid, indometacin, sulindac, diclofenac sodium, ibuprofen, naproxen, piroxicam, meloxicam, hydrocortisone, corticosterone, aldosterone, triamcinolone, prednisolone, dexamethasone acetate, methylprednisolone aceponate.

The invention provides an ocular barrier-penetrating administration preparation, the preparation includes a component (a), wherein the component (a) is a fluorine-containing compound-modified cationic polymer, and the fluorine-containing compound-modified cationic polymer can be used as a preparation administrated by penetrating a tear barrier, a corneal/conjunctival barrier, and a blood-aqueous barrier, or a blood-retinal barrier.

The invention provides an ocular barrier-penetrating administration preparation, the preparation includes a component (a), the component (a) is a fluorine-containing compound-modified cationic polymer, and the fluorine-containing compound-modified cationic polymer is used in the preparation of drugs for penetrating a tear barrier, a corneal/conjunctival barrier, and a blood-aqueous barrier, or a blood-retinal barrier.

The invention provides an ocular barrier-penetrating administration preparation, the preparation includes a component (a), the component (a) is a fluorine-containing compound-modified cationic polymer, and the fluorine-containing compound-modified cationic polymer is used in the preparation of drugs for the treatment of ocular mucosal infections, cornea/conjunctiva-related infections, and fundus diseases.

The technical solution of the present invention can be used in a skin cosmetic system, specifically as follows.

In view of the above-mentioned technical problems of the transdermal administration carrier, the object of the present invention is to provide a transdermal carrier as an administration preparation for medical, cosmetic and health care products. The present invention takes a fluorine-containing compound-modified chitosan as the main body, and can be made into corresponding transdermal preparations for drug delivery systems of medical, cosmetic and health care products that require administration through the epidermal layer or mucosal layer. The method can improve the delivery effect of drugs and improve people's experience of use, and has great application prospects. The drugs may be medical cosmetic drugs. The medical cosmetic medicines include, but are not limited to, hair health and cosmetic drugs, skin cosmetic and health drugs.

The hair health and cosmetic drugs described in the present invention may be hair growth drugs and hair care drugs. Using a fluorine-containing compound-modified chitosan as the main body, the drugs can be made into corresponding transdermal preparations for the treatment of various causes of hair loss, such as sprays, gels, ointments, lotions, etc. Therefore, using such a fluorine-containing compound-modified cationic polymer as a drug carrier to penetrate the epidermis can achieve local treatment of drugs to reach the subcutaneous and hair follicles to treat hair loss related diseases. As described in Example 6-1, a fluorine-containing compound-modified chitosan can be used as a drug carrier to deliver therapeutic drugs to the subcutaneous and hair follicles, to achieve local administration to the epidermis in the form of sprays for the treatment of hair loss diseases.

The skin cosmetic and health drugs may also be moisturizing drugs, skin rejuvenation drugs, anti-wrinkle drugs, freckle removal drugs, scar removal drugs, or other traditional Chinese medicine cosmetic drugs. The drugs can accelerate blood circulation and promote the metabolism and tissue repair of the body.

The drug may be topical drugs for dermatitis, eczema and other skin diseases, including compound dexamethasone acetate cream (Pi Yan Ping), compound miconazole nitrate cream, ketoconazole cream, triamcinolone acetonide and econazole nitrate cream, paeonol ointment, tretinoin ointment, geranium ointment, compound honeysuckle antipruritic liniment, terbinafine hydrochloride cream, calcipotriol ointment, penciclovir cream, acyclovir gel, mometasone furoate cream, triamcinolone acetonide acetate and miconazole nitrate and neomycin sulfate cream, ofloxacin gel, hydrocortisone butyrate cream, miconazole nitrate cream, triamcinolone acetonide and econazole acetate cream, metronidazole gel, bifonazole cream, etc.

The drugs may be hormone drugs or tretinoin drugs (also known as retinoic acid drugs). The hormone drugs may be glucocorticoids, such as Pi Kang Wang (clobetasol), Pi Yan Ping (dexamethasone), freckle cream (containing clobetasol), fluocinolone acetonide, and so on. The therapeutic effects of glucocorticoids mainly come from their anti-inflammatory effects, including inhibiting the release of lysosomal enzymes, inhibiting the stimulation of macrophages and capillary contraction, etc., but they are easy to cause side effects such as dermatitis, folliculitis, etc. The retinoic acid drugs include non-aromatic formic acid drugs (e.g., tretinoin and isotretinoin), monoaromatic formic acid drugs (e.g., isotretinoin and isotretinic acid), and polyaromatic tretinoin drugs (e.g., aromatic methyl ethyl ester and adapalene). Tretinoin drugs have the effects of anti-skin aging, reducing epidermal melanin, inhibiting sebum production, and immune regulation. They can protect skin, reduce wrinkles, fade melasma, and treat skin problems such as acne, pigmentation, and abnormal keratosis. However, oral administration of tretinoin drugs has large side effects, and the epidermal use can easily cause local irritation, resulting in burning sensation and slight pain, so their clinical use is subject to certain restrictions.

In particular, for scar removal, fluorine-containing compound-modified polymers can improve penetration efficiency to reduce the amount of drugs in the affected area, thereby achieving the effect of reducing side effects. In Example 6-2, the present invention uses a fluorine-containing compound-modified cationic polymer as a drug carrier to penetrate the epidermis to deliver polyinosinic acid below the stratum corneum, to induce the production of endogenous retinoic acid and achieve local drug treatment, thereby improving scars and reducing side effects.

The drugs may be topical antioxidants, including vitamin E, green tea polyphenols, acetylcysteine, coenzyme Q10, superoxide dismutase, etc., which can effectively respond to oxidative stress in cells to achieve anti-aging effect. The drugs may be ω-3 fatty acids, a-hydroxy acids, β-carotene and the like to increase skin elasticity and collagen synthesis.

The skin cosmetic and health drugs may also be vitamins and trace elements to improve the nutritional status of the skin, beautify the skin, and delay aging. Deficiency of nutrients such as iron, iodine, zinc, vitamin A, vitamin B2, niacin, biotin, folic acid, vitamin B 12, vitamin C, and vitamin D may impair growth and cause diseases, which is a health issue of widespread public concern. Although nutrient deficiencies can be effectively treated by fortifying the nutrition of food, there are still problems of insufficient stability and insufficient absorption efficiency of nutrients. Compared with free nutrients, the nutrients encapsulated in nanoparticles are more stable, but their bioavailability is lower. A nutrient preparation using perfluoroheptanoic acid modified chitosan as a carrier can effectively improve bioavailability and promote nutrient absorption.

The skin cosmetic and health drugs may also be traditional Chinese medicine cosmetic drugs, which can improve skin problems such as dull skin, pigmentation, loose skin, enlarged pores, rough skin, or repair skin barriers, and restore skin health. The traditional Chinese medicine cosmetic drugs may be drugs for delaying cell aging (extracts of astragalus and ginseng); drugs for scavenging free radicals (extracts of schisandra and Panax notoginseng, flavonoid drugs, silvianic acid B and salvianolic acid C active substances); collagen repairing drugs (Poria cocos extract, polygonatum rhizome extract and wolfberry extract, silvianic acid B and salvianolic acid C active substances); immunomodulatory drugs (schisandra chinensis extract, saponins, semen cuscutae extract); skin microcirculation-promoting drugs (salvia miltiorrhiza extract, flavonoids) and other Chinese medicines and Chinese herbal medicines.

Melasma is yellowish-brown pigmentation of the face. It has complicated etiology and is difficult to treat and easy to relapse, which brings great troubles to patients. Antioxidant drugs or glucocorticoid drugs are often used in medical treatment, such as tranexamic acid, proanthocyanidins, glutathione, hydroquinone, tretinoin, cysteamine salts, oligopeptides, Jessner solution, salicylic acid, lactic acid. Among them, tranexamic acid can inhibit ultraviolet-induced plasmin activity in keratinocytes, prevent plasminogen from binding to keratinocytes, reduce prostaglandin synthesis, and thereby inhibit pigment production. Oral administration or local injection of tranexamic acid can treat melasma. However, individuals and families need to be screened for the risk of thromboembolism before oral administration, and the penetration effect with local administration is not satisfactory. Therefore, in the present invention, using a fluorine-containing compound-modified cationic polymer as a drug carrier to penetrate the epidermis achieves local treatment of drugs to reach the subcutaneous to treat melasma. As described in Example 6-3, a fluorine-containing compound-modified chitosan can be used as a drug carrier to deliver therapeutic drugs to the subcutaneous, to achieve local administration to the epidermis for the treatment of melasma.

The present invention provides a drug composite using the above-mentioned carrier for cosmetic and health care products, which includes a carrier for cosmetic and health care products (a) and a drug component (b), wherein the drug component (b) is a hair growth drug or a hair care drug; or a moisturizing drug, a skin rejuvenation drug, an anti-wrinkle drug, a freckle removal drug, or a scar removal drug; or a topical drug for skin diseases dermatitis or eczema; or a hormone drug or a tretinoin drug; or vitamin E, a green tea polyphenol, acetylcysteine, coenzyme Q10, or superoxide dismutase.

The present invention providesa carrier preparation for cosmetic and health care products, the preparation includes a component (a), wherein the component (a) is a fluorine-containing compound-modified cationic polymer, and the fluorine-containing compound-modified cationic polymer can be used as preparations for the administration of hair growth drugs and hair care drugs, cosmetic drugs, and health care drugs.

The invention provides a topical preparation for hair growth prepared by using the above-mentioned carrier preparation for cosmetic and health care products, which includes a composite formed by an effective MPC (mitochondrial pyruvate carrier) inhibitor UK5099, metformin, minoxidil, spironolactone, or finasteride with fluorinated chitosan, wherein the particle size range of the composite is less than 10 microns, or the particle size range of the composite is not greater than 500 nanometers, and the weight ratio of the fluorinated chitosan to the hair growth drug is 1:0.5-50.

The present invention provides a composite for removing scars prepared by using the above-mentioned carrier preparation for cosmetic and health care products, which includes a particulate composite formed by using perfluoroheptanoic acid-modified chitosan and polyinosinic acid-polycytidylic acid as the main body, wherein the particle size range is less than 10 microns, or the particulate composite is a composite of not greater than 500 nanometers, and the weight ratio of the fluorinated chitosan to the anti-freckle drug is 1: 0.5-50.

The invention provides a drug composite for treating melasma prepared by using the above-mentioned carrier preparation for cosmetic and health care products, wherein the composite is perfluoroheptanoic acid modified chitosan encapsulating tranexamic acid, and the weight ratio of the fluorinated chitosan to the melasma treatment drug is 1:0.5-50.

To solve the above technical problems, the present invention adapts the following technical solution:

Further, the transdermal effect produced by the technical solution of the present invention is temporary. After the drug is removed, the stratum corneum cells of the skin will close the channel to protect the safety of the human body. The skin consists of epidermis and dermis. The epidermis is divided into stratum corneum, stratum lucidum, stratum granulosum and stratum germinativum in order from superficial to deep. The dermis is composed of dense connective tissues, and is divided into papillary layer and reticular layer in order from superficial to deep. The papillary layer is connected to the stratum germinativum of the epidermis, and contains abundant receptors such as capillaries, lymphatic vessels, nerve endings, and tactile corpuscles. The stratum corneum is the largest rate-limiting barrier for transdermal administration. The stratum corneum in most of the skin consists of 5-25 layers of flat keratinocytes. These cells have no nucleus and organelles, and have thicker cell membranes. They are lifeless and water-tight, and have the functions of preventing tissue fluid from flowing out, resisting friction and preventing infection. The fluorine-containing compound-modified cationic polymer can stimulate a change in the distribution of tight junction proteins in these cells to reduce tight junctions between cells, and further stimulate actin phosphorylation, thereby promoting the paracellular transport and opening the intercellular space to forms channels, so that the drug is carried through the stratum corneum and is further penetrated into the skin, and then into the dermis and into the skin capillaries and lymphatic circulation to exert the drug effect.

In summary, the fluorine-containing compound-modified cationic polymer provided by the present invention can be universally bound to a variety of drugs to promote drug absorption, improve drug bioavailability, and reduce toxicity, and has good effects, broad applicability, and great commercial value. In addition, the fluorine-containing compound-modified cationic polymer proposed by the present invention is easy to produce and has a commercial basis.

The present invention also provides a fluorine-containing compound-modified cationic polymer, especially fluorinated chitosan, which promotes drug absorption across mucosal membranes and has good biological safety. The fluorine-containing compound-modified cationic polymer proposed in the present invention has a simple synthesis process and universal applicability; and has obvious advantages in transmucosal administration, mainly including: (1) it has good permeability and can span various mucosal membranes (nasal mucosa, lung mucosa, vaginal mucosa, oral mucosa, gastrointestinal mucosa, etc.) to increase the concentration of drugs in blood and tissues; (2) it has good adhesion and can adhere to various mucosal surfaces to achieve sustained drug release; and (3) it has a wide range of applications, and can bind to large molecule drugs, can also adsorb small molecule drugs, and can also bind to compound drugs with complex components, for the treatment of various diseases, and thus has great commercial value.

In summary, the fluorinated chitosan drug carrier provided by the present invention has the advantages of obvious drug absorption promoting effect and low toxicity, etc. The fluorinated chitosan proposed by the present invention has a mature synthesis process, simple operation, high synthesis efficiency, short cycle time, and high yield of a drug carrier without complicated purification steps, and this simple synthesis method provides a good basis for commercialization. The fluorinated chitosan of the present invention is useful as a variety of drug carriers, can effectively improve the therapeutic effect, and has a wide range of uses and a low cost.

Further, the transmucosal effect produced by the technical solution of the present invention is temporary. After the drug is removed, the intestinal mucosal cells will close the channel to protect the human body. (See FIG. 3-1).

The fluorine-containing compound-modified cationic polymer, especially fluorinated chitosan, provided by the present invention, can be universally bound to a variety of drugs to promote drug absorption, improve drug bioavailability, and reduce toxicity, and has good effects, broad applicability, and great commercial value. In addition, the fluorine-containing compound-modified cationic polymer proposed by the present invention is easy to produce and has a commercial basis.

The fluorinated cationic polymer designed in the present invention has applications in the fields of biochemistry and pharmacy, including, but not limited to the following administration modes: oral administration systems, sprays/inhalants, and nasal drops. As a preferred solution of the fluorine-containing compound-modified cationic polymer of the present invention: the cationic polymer is chitosan with a degree of deacetylation >95%. The following application takes fluorinated chitosan as an example. Other cationic polymers can also be fluorinated to achieve similar effects.

### Brief Description of the Drawings

FIG. 1-1 shows the effect of heptafluorobutyric acid modified chitosan (7FCS) on the distribution and intensity of THP in mouse bladder tissue in Example 1-5. THP, epirubicin; CS, chitosan; FCS, fluorinated chitosan; the right panel shows the relative fluorescence intensity analysis of THP corresponding to the left panel.
FIG. 1-2 shows the effect of tridecafluoroheptanoic acid-modified chitosan (13FCS) on the distribution and intensity of THP in mouse bladder tissue in Example 1-6. THP, epirubicin; CS, chitosan; FCS, fluorinated chitosan; the right panel shows the relative fluorescence intensity analysis of THP corresponding to the left panel.
FIG. 1-3 shows the effect of different fluorinated fatty acid-modified chitosans (7FCS, 13FCS, 19FCS) on the distribution and intensity of THP in mouse bladder tissue in Example 1-7. THP, epirubicin; CS, chitosan; FCS, fluorinated chitosan; the right panel shows the relative fluorescence intensity analysis of THP corresponding to the left panel.
FIG. 1-4a is a comparison diagram showing that 13F-3 has good in vitro cell safety in Example 1-8.
FIG. 1-4b shows that there is no significant difference between the body weight of mice in the FCS group and the blank control group in Example 1-8.
FIG. 1-4c is a comparison diagram of mouse bladder tissues and HE (hematoxylin-eosin) stained sections after perfusion in each group and those of the blank control group in Example 1-8.
FIG. 1-5 is a comparison diagram of immunofluorescence results in Example 1-8, showing that the bladder of mice in the chitosan perfusion group has severe inflammatory stress and congestion and edema, while there is no significant difference between the FCS group and the blank control group. The left panel is a confocal fluorescence image of the bladder tissue sections, and the right panel shows the results of the CS and FCS treatment groups and the blank group.
FIG. 1-6 is a related image of MPI/FPEI measured by transmission electron microscopy in Example 1-9.
FIG. 1-7 is a related image of MPI/PEI measured by transmission electron microscopy in Example 1-9.
FIG. 1-8 shows that the mucosal permeability index of a polypeptide in the F-PEI group is significantly higher than that of the PEI group and the blank control group in Example 1-9, where the abscissa is the feed weight ratio of the polypeptide drug MPI to the material PEI or FPEI, and the ordinate is the permeability coefficient papp.
FIG. 1-9 shows that the mucosal permeability index of a protein drug in the F-PEI group is significantly higher than that of the PEI group and the blank control group in Example 1-9, where the abscissa is the feed weight ratio of the polypeptide drug CAT-Ce6 to the material PEI or FPEI, and the ordinate is the permeability coefficient papp.
FIG. 1-10 is a comparison diagram showing distribution and intensity of drug fluorescence in tissue when frozen sections of the bladder are prepared at different times after drug perfusion with different drug systems of MPI in Example 1-9, where the abscissa is the perfusion time, and the ordinate is the relative value of the fluorescence intensity of the polypeptide drug.
FIG. 1-11 is a comparison diagram showing distribution and intensity of drug fluorescence in tissue when frozen sections of the bladder are prepared after perfusion with different drug systems of CAT in Example 1-9, where the left panel is the fluorescence distribution of different drug systems of the drug CAT-Ce6 in the bladder tissue, and the right panel is the fluorescence intensity analysis of the drug.
FIG. 1-12 is a transmission electron microscope (TEM) image in Example 1-10.
FIG. 1-13 is a comparison diagram of frozen sections of the bladder and the fluorescence intensity analyzed by a fluorescent confocal microscope in Example 1-10. The left panel shows the fluorescence distribution of the drug CAT-TCPP in the transverse and longitudinal sections of the bladder tissue, from left to right; the right panel shows the fluorescence intensity of the drug in the homogenate of bladder tissue after transverse section and longitudinal section of the bladder, from top to bottom.
FIG. 1-14 shows a synthetic route diagram of a fluorine-containing carboxylic acid-modified chitosan as a bladder perfusion drug carrier.
FIG. 2-1 shows a schematic diagram of the transdermal mechanism of a fluorine-containing compound-modified cationic polymer.
FIG. 2-2 shows changes in particle size of perfluoroheptanoic acid-modified chitosan and a drug in different ratios in an aqueous solution before and after the reaction, taking perfluoroheptanoic acid-modified chitosan-insulin (FCS-Insulin) as an example.

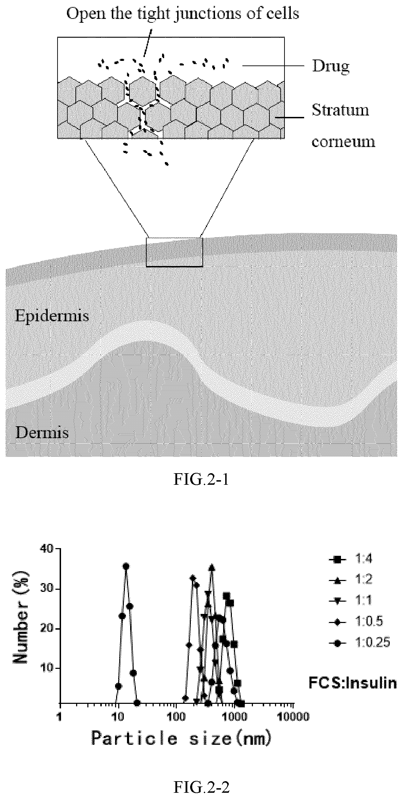

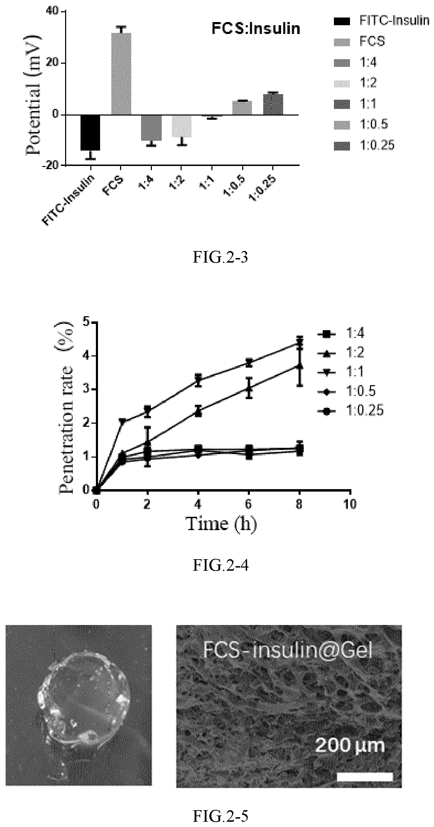

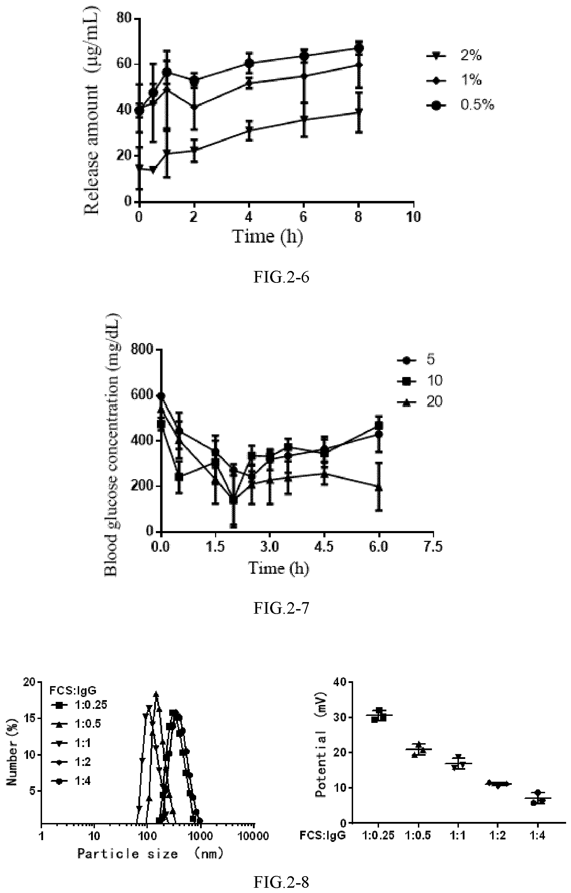

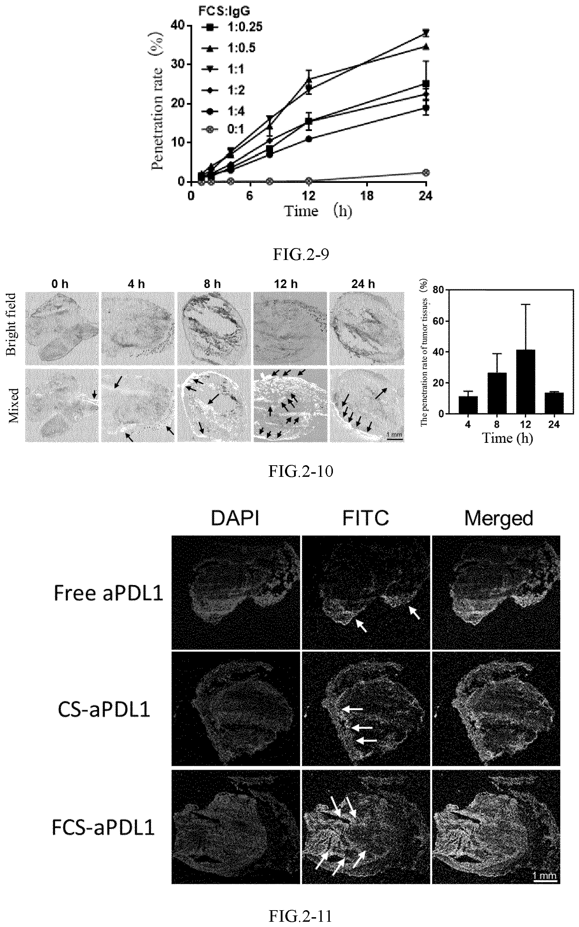

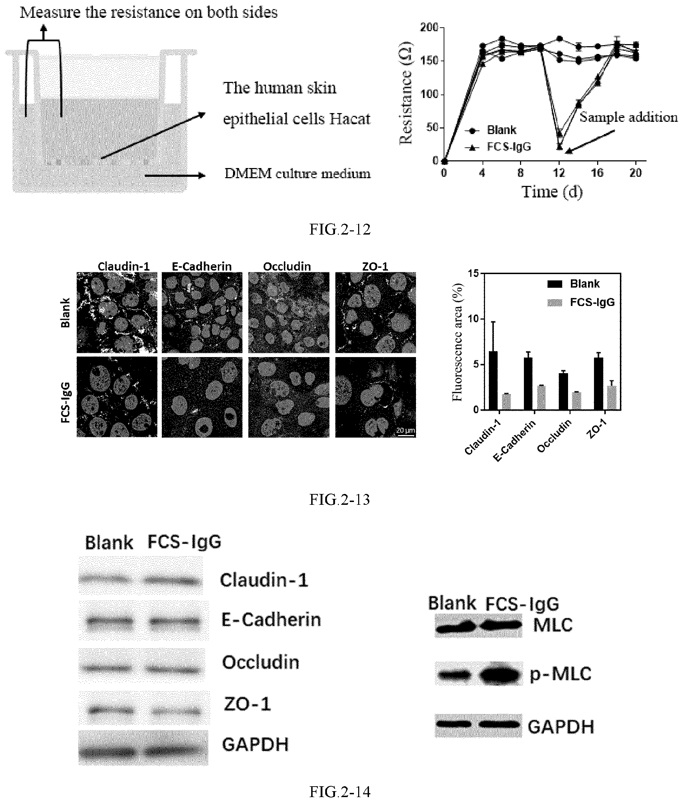

FIG. 2-3 shows changes in potential of perfluoroheptanoic acid-modified chitosan and a drug in different ratios in an aqueous solution before and after the reaction, taking perfluoroheptanoic acid-modified chitosan-insulin (FCS-Insulin) as an example.
FIG. 2-4 shows the difference of transdermal effect of perfluoroheptanoic acid-modified chitosan and a drug in different ratios, where the right panel is a schematic diagram of a transdermal diffusion cell, taking perfluoroheptanoic acid-modified chitosan-insulin (FCS-Insulin) as an example.
FIG. 2-5 shows a real photo and a SEM photo of a gel, taking perfluoroheptanoic acid-modified chitosan-insulin (FCS-Insulin) as an example.
FIG. 2-6 shows release of a drug from a gel, taking perfluoroheptanoic acid-modified chitosan-insulin (FCS-Insulin) as an example.
FIG. 2-7 shows the therapeutic effect of a drug at the living body level, namely, the fluctuation of blood sugar after the application of a patch, take perfluoroheptanoic acid-modified chitosan-insulin (FCS-Insulin) as an example.
FIG. 2-8 shows the particle size and potential of perfluoroheptanoic acid-modified chitosan-immunoglobulin G (FCS-IgG) in different ratios in an aqueous solution.
FIG. 2-9 shows cumulative penetration to the skin of perfluoroheptanoic acid-modified chitosan-immunoglobulin G (FCS-IgG) in different ratios at different time points, in which the ratios of perfluoroheptanoic acid-modified chitosan-immunoglobulin G are 1:0.25, 1:0.5, 1:1, 1:2, 1:4 and 0:1 (pure immunoglobulin G).
FIG. 2-10 shows dynamic analysis of *in vivo* skin penetration of perfluoroheptanoic acid-modified chitosan-immunoglobulin G (hereinafter referred to as FCS-IgG), in which the first five are the fluorescence intensity (white) of mouse tumor tissue (gray) sections at different time points, and the sixth one is quantitative analysis of fluorescence intensity detected after tumor tissue lysis at different time points in mice.
FIG. 2-11 shows comparison of transdermal efficiency in *in vivo* tumor tissue of perfluoroheptanoic acid-modified chitosan-programmed cell death-ligand 1 antibody, chitosan-programmed cell death-ligand 1 antibody and pure programmed cell death-ligand 1 antibody: from the top to the bottom, pure programmed cell death-ligand 1 antibody (free aPDL1), chitosan-programmed cell death-ligand 1 antibody (CS-aPDL1), and perfluoroheptanoic acid-modified chitosan-programmed cell death-ligand 1 antibody (FCS-aPDL1) group; from left to right, the fluorescence intensity of DAPI channel (gray, indicating tumor tissue), FITC fluorescence channel (white, indicating programmed cell death-ligand 1 antibody) and mixed channel.
FIG. 2-12 shows the transdermal mechanism of perfluoroheptanoic acid-modified chitosan-immunoglobulin G (FCS-IgG): the influence of perfluoroheptanoic acid-modified chitosan-immunoglobulin G (FCS-IgG) on the cell resistance of a dense cell monolayer, where the left panel is a schematic diagram of a measurement method, and the right panel is the resistance change after adding perfluoroheptanoic acid-modified chitosan-immunoglobulin G (FCS-IgG).
FIG. 2-13 shows the transdermal mechanism of perfluoroheptanoic acid-modified chitosan-immunoglobulin G (FCS-IgG): an immunofluorescence staining image of the influence of perfluoroheptanoic acid-modified chitosan-immunoglobulin G (FCS-IgG) on related tight junction proteins of a dense cell monolayer. The right panel is a semi-quantitative analysis graph of fluorescence intensity.
FIG. 2-14 shows the transdermal mechanism of perfluoroheptanoic acid-modified chitosan-immunoglobulin G (FCS-IgG): Western Blotting analysis of the influence of perfluoroheptanoic acid-modified chitosan-immunoglobulin G (FCS-IgG) on related tight junction proteins of a dense cell monolayer.
FIG. 2-15 shows *in vivo* subcutaneous tumor treatment with perfluoroheptanoic acid-modified chitosan-programmed cell death-ligand 1 antibody, divided into a blank group (blank), a pure programmed cell death-ligand 1 antibody (free aPDL1) group, a chitosan-programmed cell death-ligand 1 antibody (CS-aPDL1) group, and a perfluoroheptanoic acid-modified chitosan-programmed cell death-ligand 1 antibody (FCS-aPDL1) group, where the left panel is mouse tumor growth curve, and the right panel is the mouse survival rate curve (defining mouse tumors greater than 1500 cubic millimeters as dead).
FIG. 3-1 is a schematic diagram of the intestinal mucosa.
FIG. 3-2 is a schematic diagram showing the opening of the epithelial cell barrier of intestinal mucosa by perfluoroheptanoic acid-modified chitosan.
FIG. 3-3 shows changes in particle size of perfluoroheptanoic acid-modified chitosan and a drug in different ratios in an aqueous solution before and after the reaction in Example 3-1, taking perfluoroheptanoic acid-modified chitosan-insulin (FCS-Insulin) as an example.
FIG. 3-4 shows changes in potential of perfluoroheptanoic acid-modified chitosan and a drug in different ratios in an aqueous solution before and after the reaction in Example 3-1, taking perfluoroheptanoic acid-modified chitosan-insulin (FCS-Insulin) as an example.
FIG. 3-5 shows changes in particle size of a perfluoroheptanoic acid-modified chitosan and drug composite before and after lyophilization in Example 3-1, taking perfluoroheptanoic acid-modified chitosan-insulin (FCS-Insulin) as an example.
FIG. 3-6 shows the difference of transmucosal effect of perfluoroheptanoic acid-modified chitosan and a drug in different ratios in Example 3-1, taking perfluoroheptanoic acid-modified chitosan-insulin (FCS-Insulin) as an example.
FIG. 3-7 shows the effect of transmucosal drug delivery of a drug-loaded capsule in gastric juice and intestinal juice in Example 3-1, taking perfluoroheptanoic acid-modified chitosan-insulin (FCS-Insulin) as an example.
FIG. 3-8 shows blood glucose fluctuation of mice after oral delivery of a drug-loaded capsule in Example 3-1, taking perfluoroheptanoic acid-modified chitosan-insulin (FCS-Insulin) as an example.
FIG. 3-9 shows cumulative penetration to the intestinal mucosa of perfluoroheptanoic acid-modified chitosan-immunoglobulin G (FCS-IgG) in different ratios at different time points in Example 3-2. The abscissa is the injection time, and the ordinate is the penetration rate of the cumulative penetration amount divided by the total injection amount. Compared with pure macromolecular protein immunoglobulin G, different molecular weights of perfluoroheptanoic acid-modified chitosan-immunoglobulin G all can increase the efficiency of immunoglobulin G permeating the intestinal mucosa of rats. Preferably, a perfluoroheptanoic acid-modified chitosan-immunoglobulin G composite with a ratio of 1:1 is obtained.
FIG. 3-10 shows that perfluoroheptanoic acid-modified chitosan-bovine serum albumin-cyanine dye Cy5.5 is at the site of the digestive tract at 3h and at 5h in Example 3-2, where white represents the cyanine dye Cy5.5 labeled bovine serum albumin, indicating that the capsule can be targeted to the colorectal release, so the capsule is selected for subsequent experiments.
FIG. 3-11 is a schematic diagram of the activity of programmed cell death-ligand 1 antibody before and after lyophilization in Example 3-2. After a lyoprotectant is added for lyophilization, the activity of the programmed cell death-ligand 1 antibody after turning into a lyophilized powder keeps unchanged. It shows that the perfluoroheptanoic acid-modified chitosan-programmed cell death-ligand 1 antibody particles can be freeze-dried and can be used for filling of oral capsules.
FIG. 3-12 shows the binding activity of FCS/a PD-L1 and PD-L1 on CT-26 cell surface before and after lyophilization in Example 3-2.
FIG. 3-13 shows changes in the distribution of tight junction proteins in epithelial cells of human colorectal cancer before and after treatment with perfluoroheptanoic acid-modified chitosan and perfluoroheptanoic acid-modified chitosan/immunoglobulin G in Example 3-2.
FIG. 3-14 shows the effect of a capsule containing freeze-dried powders of perfluoroheptanoic acid-modified chitosan-programmed cell death-ligand 1 antibody in the treatment of colorectal cancer in Balb/c mice in Example 3-2, where the black color indicates the bioluminescence intensity of colorectal cancer cells in mice.
FIG. 3-15 shows the transmucosal effect of different molecular weights of perfluoroheptanoic acid-modified chitosan/immunoglobulin G in Example 3-2.
FIG. 3-16 is immunofluorescence section of the lungs of mice in Example 3-3. The fluorescence signal indicates the retention of a drug in the lungs.
FIG. 3-17 shows photos of mouse brain in each experimental group in Example 3-4, in which the white arrows indicate the fluorescence signal of Cy5.5 in the mouse brain.
FIG. 3-18 shows laser confocal photos of mouse brain sections in Example 3-4, in which the white arrows indicate the fluorescence signal of Cy5.5 in the tumor site in the mouse brain.
FIG. 4-1 is a schematic diagram of the eyeball structure.
FIG. 4-2 is a schematic diagram of membrane permeation of a fluorine-containing drug eye drop.
FIG. 4-3 shows the cumulative permeation percentage of perfluoroheptanoic acid-modified chitosan in different ratios through the cornea in vitro in Example 4-1.
FIG. 4-4 is an immunofluorescence staining image of a macromolecular drug bovine serum albumin in the eyeball in Example 4-1, where the left panel is an immunofluorescence staining image of membrane penetration of perfluoroheptanoic acid-modified chitosan, and the right panel is an immunofluorescence staining image of membrane penetration of the free drug.
FIG. 4-5 is an immunofluorescence staining image of a small molecule drug Rhodamine B in the eyeball in Example 4-1, where the left panel is an immunofluorescence staining image of membrane penetration of perfluoroheptanoic acid-modified chitosan, and the right panel is an immunofluorescence staining image of membrane penetration of the free drug.
FIG. 4-6 shows the eye tissue content of albumin of perfluoroheptanoic acid-modified chitosan after eye instillation in Example 4-1.
FIG. 4-7 shows immunofluorescence staining of perfluoroheptanoic acid-modified chitosan at different times after eye instillation in local cornea in Example 4-1.
FIG. 4-8 shows photos of corneal fluorescence staining of perfluoroheptanoic acid-modified chitosan at various time points in mice to detect the safety of perfluoroheptanoic acid-modified chitosan in Example 4-1.
FIG. 4-9 shows bioluminescence images of malignant choroidal melanoma in situ before treatment and one week after treatment in Example 4-2.
FIG. 4-10 shows bioluminescence quantitative analysis of malignant choroidal melanoma in situ one week after treatment in Example 4-2.
FIG. 5-1 shows Flow cytometry processing graph and statistical graph of the percentage of mature dendritic cells in total dendritic cells after in vitro stimulation with a perfluoroheptanoic acid-modified chitosan-chicken ovalbumin (FCS-OVA) composite in Example 5-1. Mature dendritic cells can effectively present antigens, activate T cells, and initiate immune response. The blank is a naturally mature negative control group without treatment, the lipopolysaccharide group is a positive control group added with lipopolysaccharide, and the perfluoroheptanoic acid-modified chitosan-chicken ovalbumin group serves as an experimental group.

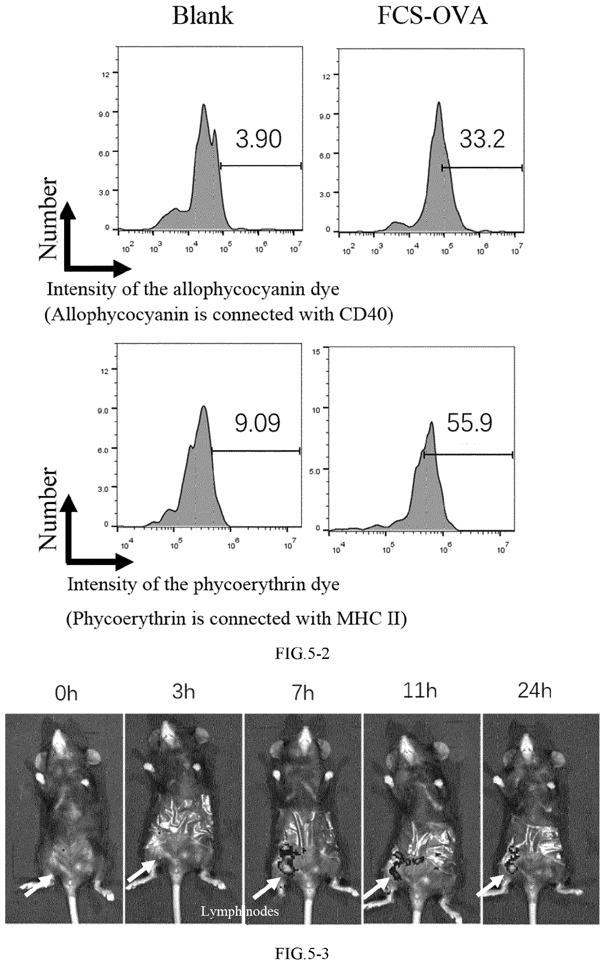

FIG. 5-2 shows changes in major histocompatibility complex class II (MHC II) and cluster of differentiation 40 (CD40), which are the key proteins for the expression and presentation of antigens by dendritic cells, after in vitro stimulation with a perfluoroheptanoic acid-modified chitosan-chicken ovalbumin composite in Example 5-1. The stronger the intensity of the allophycocyanin dye, the more CD40 protein is expressed on the surface of dendritic cells, and the stronger the intensity of the phycoerythrin dye indicates the more MHC II is expressed on the surface of dendritic cells.
FIG. 5-3 shows *in vivo* imaging of the accumulation of a perfluoroheptanoic acid-modified chitosan-chicken ovalbumin composite in lymph nodes over time, after a fluorescent perfluoroheptanoic acid-modified chitosan-chicken ovalbumin composite and an ointment are mixed and applied to the back of mice in Example 5-1. The white arrow indicates one of the lymph nodes in mice.

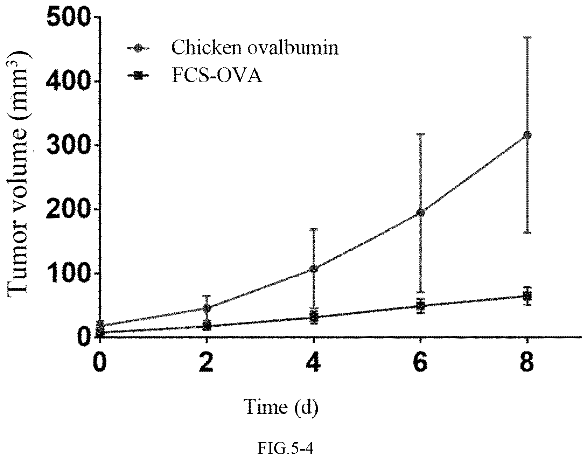

FIG. 5-4 shows tumor growth over time after implanting a melanoma expressing chicken ovalbumin on the cell surface (B16-OVA) on the back of mice, after vaccination for mice implanted with a perfluoroheptanoic acid-modified chitosan-chicken ovalbumin vaccine complex using a transdermal patch and for mice implanted with a chicken ovalbumin vaccine using a transdermal patch in Example 5-2.

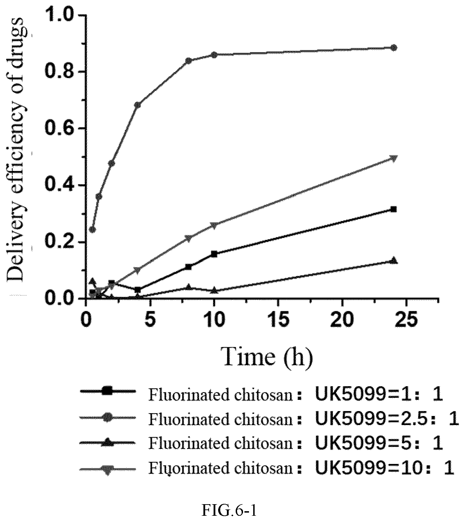

FIG. 6-1 shows the in vitro transdermal delivery efficiency of perfluoroheptanoic acid-modified chitosan and a small molecule drug UK5099 with hair growth treatment effect in different ratios at different time points in Example 6-1.

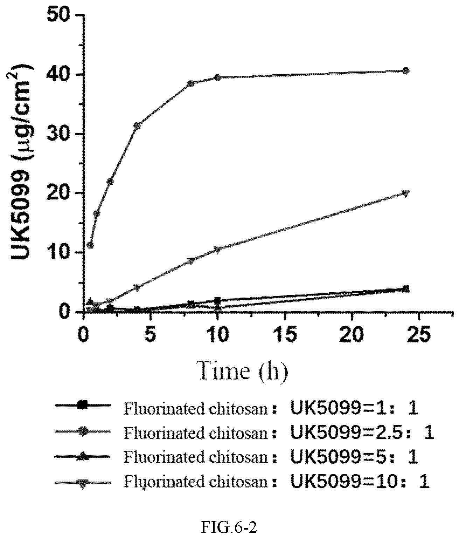

FIG. 6-2 shows the in vitro transdermal cumulative permeation of perfluoroheptanoic acid-modified chitosan and a small molecule drug UK5099 with hair growth treatment effect in different ratios at different time points in Example 6-1.

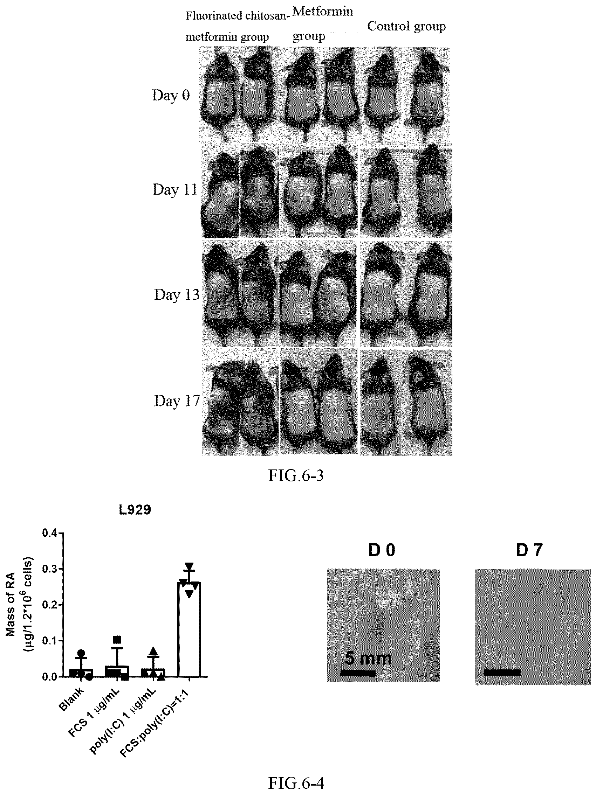

FIG. 6-3 shows the hair growth on the back of mice at different time points when treated with different treatments in Example 6-1. FCS-metformin group is an experimental group in which metformin is modified with perfluoroheptanoic acid-modified chitosan for transdermal administration, and metformin group is a free drug group.
FIG. 6-4 shows the results of Example 6-2. The left panel shows the efficiency of a mixture of perfluoroheptanoic acid-modified chitosan and poly(I:C) for the production of retinoic acid in mouse fibroblasts. The right panel shows the effect of the mixture of perfluoroheptanoic acid-modified chitosan and poly(I:C) when applied to the scars of mice.

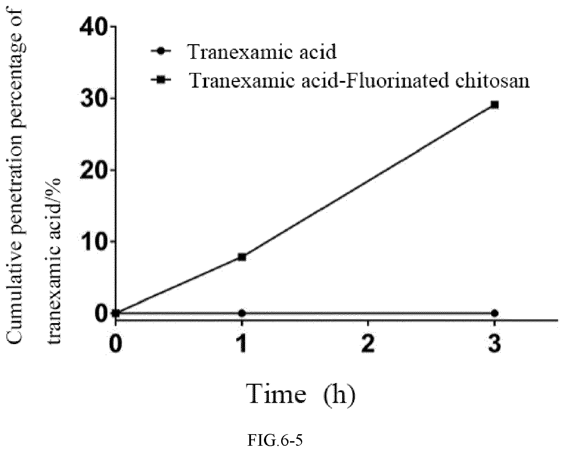

FIG. 6-5 shows that in a rat skin permeation experiment with a diffusion cell, the cumulative permeation percentage of tranexamic acid through rat skin [(cumulative permeation/theoretical total permeation) × 100%] within three hours using a mixture of perfluoroheptanoic acid-modified chitosan and tranexamic acid and pure tranexamic acid in Example 6-3.

### DETAILED DESCRIPTION OF THE INVENTION

The following describes the present invention in further detail with reference to specific examples and drawings, and the protection content of the present invention is not limited to the following examples. Without departing from the spirit and scope of the inventive concept, changes and advantages that those skilled in the art can think of are all included in the present invention, and the scope of protection is subject to the attached claims.

Description of abbreviations: THP (epirubicin); EDC (1-(3-dimethylaminopropyl)-3-ethylcarbodiimide hydrochloride), NHS (N-hydroxysulfosuccinyl imine); DMSO (dimethyl sulfoxide); MPI (a polypeptide drug Polybia-MPI); MPICy5.5 (fluorescence labeling of polypeptide drug MPI); PEI (polyethylene imine); FPEI (fluorinated polyethylene imine); CAT (a protein drug catalase); CAT-Ce6 (a composite protein drug labeled with photosensitizer Ce6); CAT-TCPP (a composite protein drug labeled with sonosensitizer TCPP).

In view of the fact that there are many examples and drawings in the present document, in order to distinguish between different groups of examples and different drawings, the naming of the examples adopts the following rules: the first series of examples is Example 1-1, Example 1-2, Example 1-3, and so on, the second series of examples are Example 2-1, Example 2-2, and so on. Similar rules are adopted for the naming of the drawings: the first series of drawings are FIG. 1-1, FIG. 1-2, and FIG. 1-3, and so on, the second series of drawings are FIG. 2-1, FIG. 2-2, and so on.

### Reference Example

Experimental designers used adriamycin, epirubicin (THP) and fluorescent dye rhodamine B as a bladder perfusion drug respectively to mix with chitosan (1% acetic acid aqueous solution) to prepare chitosan bladder perfusion drug systems. After 1 h of bladder perfusion, the bladder tissue was removed, frozen sections were prepared, the fluorescence intensity of the drug was analyzed with a confocal fluorescence microscope, and the distribution of the drug in the bladder tissue was investigated. The experimental results show that the adhesion and penetration ability of the chitosan (5 mg/ml, 10 mg/ml, 15 mg/ml) solution of the bladder perfusion drug of equal concentration in the bladder mucosa is significantly better than that in the case of perfusion of the aqueous solution of the pure drug. As the concentration of chitosan increases, the adhesion and penetration ability of the perfusion system is stronger. In order to further investigate the biological safety of chitosan as a drug carrier for perfusion, the designers of the project perfused the mouse bladder with a 15 mg/ml chitosan aqueous solution, stopped the perfusion after 1 h, continued to raise the mice under normal feeding conditions, and recorded the mice weight. The results of the experiment show that the weight of the mice dropped sharply on the second day after perfusion of chitosan, and the mice were sluggish in activity. From the second day after treatment, the mice began to die, and all 8 mice in the experimental group died within 3 days. After dissection, the bladder of the mice in the experimental group was compared with that of the mice in the control group. It was found that the bladder of mice in the chitosan perfusion group was more congested compared with that in the blank group, and the results of HE staining and CD45 and Ki67 immunofluorescence showed that the bladder of the mice in the chitosan perfusion group had severe inflammatory stress and congestion and edema. The above experimental results show that although chitosan can significantly improve the bioavailability of the perfusion drug in the bladder mucosa, high concentrations of chitosan can also cause serious bladder mucosal and epithelial damage, which severely limits its clinical use as a drug carrier for bladder perfusion.

Example 1-1: Preparation of chitosan with different modification degrees of 3-fluorobenzoic acid (degree of deacetylation ≧ 95%, viscosity 100-200 MPa.s), in which the feed molar ratios of 3-fluorobenzoic acid and N-glucosamine unit were 1:1.1, 1:2.2, 1:4.4, and 1:8.8.

Synthesis method: (1) preparation of a solution of chitosan in an aqueous acetic acid solution: 200 mg of fully dried chitosan was weighed and added into 10 mol% of an aqueous acetic acid solution (of course, an aqueous hydrochloric acid solution could also be used) and stirred for 30 min to be fully dissolved, and then 1.6 ml of 0.5M sodium hydroxide was slowly added dropwise and stirred until a clear solution was obtained and the pH was about 6.5. From the perspective of simply considering the alkalizing solution, sodium hydroxide may be replaced by alkali such as ammonia or triethylamine, but from the perspective of product technology, the by-product is sodium chloride when sodium hydroxide is used, which is more suitable for industrialization. In this way, 4 parts of aqueous solutions of chitosan in acetic acid were prepared. (2) activation of 3-fluorobenzoic acid: 5.0 mg, 9.8 mg, 19.7 mg and 40 mg of 3-fluorobenzoic acid were weighed respectively and dissolved in an appropriate amount of anhydrous dimethyl sulfoxide, and the reaction amounts of EDC and NHS were sequentially added, and stirred for 1h under protection from light. (3) preparation of 3-fluorobenzoyl chitosan: the above-mentioned activated 3-fluorobenzoic acid solutions were slowly added dropwise respectively to the rapidly stirring chitosan solution, and stirred for 20h under protection from light. After the reaction was complete, the reacted solutions above were slowly added dropwise successively to 100 ml of a 0.5M solution of potassium hydroxide in ethanol and stirred for 8h, a precipitate was filtered and washed with a large amount of absolute ethanol until a filtrate was neutral, the precipitate was dehydrated by washing with methanol and diethyl ether and dried in vacuum for 30 min. The dried precipitate was dissolved in 10 ml 0.1M of a hydrochloric acid solution and lyophilized to obtain 3-fluorobenzoic acid fluorinated chitosan hydrochloride with different degrees of fluorination modification in the appearance of white powder (the products were named 1FCS-1, 1FCS-2, 1FCS-3, and 1FCS-4).

The materials obtained from the above reactions were tested for the degree of modification of the fluorinated fatty chains on the surface of the fluorinated chitosan (FCS) polymer by means of the ninhydrin reaction method. The ninhydrin reaction method is a simple, fast, accurate and reliable method, which can accurately detect the number of primary amino groups on the surface of FCS polymer in the aqueous solution, to calculate the number of fluorinated groups on the surface of FCS.

Example 1-2: Preparation of chitosan with different modification degrees of heptafluorobutyric acid (degree of deacetylation ≧ 95%, viscosity 100-200 MPa.s), in which the feed molar ratios of heptafluorobutyric acid and N-glucosamine unit were 1:1.1, 1:2.2, 1:4.4, and 1:8.8.

Synthesis method: (1) preparation of a solution of chitosan in an aqueous acetic acid solution: 200 mg of fully dried chitosan was weighed and added into 10 mol% of an aqueous acetic acid solution and stirred for 30 min to be fully dissolved, and then 1.6 ml of 0.5M sodium hydroxide was slowly added dropwise and stirred until a clear solution was obtained and the pH was about 6.5. In this way, 4 parts of aqueous solutions of chitosan in acetic acid were prepared. (2) activation of heptafluorobutyric acid: 7.6 mg, 15 mg, 30 mg, and 61 mg of heptafluorobutyric acid were weighed respectively and dissolved in an appropriate amount of anhydrous dimethyl sulfoxide, and the reaction amounts of EDC and NHS were sequentially added, and stirred for 1h under protection from light. (3) preparation of heptafluorobutyric acid chitosan: the above-mentioned activated heptafluorobutyric acid solutions were slowly added dropwise respectively to the rapidly stirring chitosan solution, and stirred for 20h under protection from light. After the reaction was complete, the reactions were slowly added dropwise successively to 100 ml of a 0.5M solution of potassium hydroxide in ethanol and stirred for 8h, a precipitate was filtered and washed with a large amount of absolute ethanol until a filtrate was neutral, the precipitate was dehydrated by washing with methanol and diethyl ether and dried in vacuum for 30 min. The dried precipitate was dissolved in 10 ml 0.1M of a hydrochloric acid solution and lyophilized to obtain heptafluorobutyric acid fluorinated chitosan hydrochloride with different degrees of fluorination modification in the appearance of white powder (the products were named 7FCS-1, 7FCS-2, 7FCS-3, and 7FCS-4).

The materials obtained from the above reactions were tested for the degree of modification of the fluorinated fatty chains on the surface of the fluorinated chitosan (FCS) polymer by means of the ninhydrin reaction method. The ninhydrin reaction method is a simple, fast, accurate and reliable method, which can accurately detect the number of primary amino groups on the surface of FCS polymer in the aqueous solution, to calculate the number of fluorinated groups on the surface of FCS. By the ninhydrin reaction method, the degrees of fluorination modification of FCS prepared above were calculated to be: 7FCS-1, 6.9%; 7FCS-2, 10.4%; 7FCS-3, 23.5%; 7FCS-4, 42.3%.

Example 1-3: Preparation of chitosan with different modification degrees of perfluoroheptanoic acid (degree of deacetylation ≧ 95%, viscosity 100-200 MPa.s), in which the feed molar ratios of perfluoroheptanoic acid and N-glucosamine unit were 1:1.1, 1:2.2, 1:4.4, and 1:8.8.

Synthesis method: (1) preparation of a solution of chitosan in an aqueous acetic acid solution: 200 mg of fully dried chitosan was weighed and added into 10 mol% of an aqueous acetic acid solution and stirred for 30 min to be fully dissolved, and then 1.6 ml of 0.5M sodium hydroxide was slowly added dropwise and stirred until a clear solution was obtained and the pH was about 6.5. In this way, 4 parts of aqueous solutions of chitosan in acetic acid were prepared. (2) activation of perfluoroheptanoic acid (13-fluoroheptanoic acid): 13 mg, 26 mg, 51.5 mg and 103 mg of perfluoroheptanoic acid were weighed respectively and dissolved in an appropriate amount of anhydrous dimethyl sulfoxide, and appropriate amounts of EDC and NHS were sequentially added, and fully stirred for 1h under protection from light. (3) preparation of 13F-heptanoic acid chitosan: the above-mentioned activated perfluoroheptanoic acid solutions were slowly added dropwise respectively to the rapidly stirring chitosan solution, and stirred for 20h under protection from light. After the reaction was complete, the reactions were slowly added dropwise successively to 100 ml of a 0.5M solution of potassium hydroxide in ethanol and stirred for 8h, a precipitate was filtered and washed with a large amount of absolute ethanol until a filtrate was neutral, the precipitate was dehydrated by washing with methanol and diethyl ether and dried in vacuum for 30 min. The dried precipitate was dissolved in 10 ml 0.1M of a hydrochloric acid solution and lyophilized to obtain heptafluorobutyric acid fluorinated chitosan hydrochloride with different degrees of fluorination modification in the appearance of white powder (the products were named 13FCS-1, 13FCS-2, 13FCS-3, and 13FCS-4).
By the ninhydrin reaction method, the degrees of fluorination modification of FCS prepared above were calculated to be: 13FCS-1, 5.2%; 13FCS-2, 11.3%; 13FCS-3, 21.4%; 13FCS-4, 42.5%. The linking efficiency of 13FCS-1 to 13FCS-413 fluoroheptacarbonyl group is 5.2% to 42.5% with the increase of perfluoroheptanoic acid feed, that is, on average, 5.2% to 42.5% of the glucose structural units in each chitosan molecule have been fluorinated and the products are named 13FCS-1, 13FCS-2, 13FCS-3, 13FCS-4.

Example 1-4: Preparation of chitosan with different modification degrees of 19F-capric acid (degree of deacetylation ≧ 95%, viscosity 100-200 MPa.s), in which the feed molar ratios of 19F-capric acid and N-glucosamine unit were 1:1.1, 1:2.2.

Synthesis method: (1) preparation of a solution of chitosan in an aqueous acetic acid solution: 200 mg of fully dried chitosan was weighed and added into 10 mol% of an aqueous acetic acid solution and stirred for 30 min to be fully dissolved, and then 1.6 ml of 0.5M sodium hydroxide was slowly added dropwise and stirred until a clear solution was obtained and the pH was about 6.5. In this way, 2 parts of aqueous solutions of chitosan in acetic acid were prepared. (2) activation of 19F-capric acid: 18 mg and 36.7 mg of 19F-capric acid were weighed respectively and dissolved in an appropriate amount of anhydrous dimethyl sulfoxide, and appropriate amounts of EDC and NHS were sequentially added, and fully stirred for 1h under protection from light. (3) preparation of 19F-capric acid chitosan: the above-mentioned activated 19F-capric acid solutions were slowly added dropwise respectively to the rapidly stirring chitosan solution, and stirred for 20h under protection from light. After the reaction was complete, the reactions were slowly added dropwise successively to 100 ml of a 0.5M solution of potassium hydroxide in ethanol and stirred for 8h, a precipitate was filtered and washed with a large amount of absolute ethanol until a filtrate was neutral, the precipitate was dehydrated by washing with methanol and diethyl ether and dried in vacuum for 30 min. The dried precipitate was dissolved in 10 ml 0.1M of a hydrochloric acid solution and lyophilized to obtain 19F-capric acid fluorinated chitosan hydrochloride with different degrees of fluorination modification in the appearance of white powder (the products were named 19FCS-1 and 19FCS-2).

The water solubility of 19FCS-2 was relatively poor, and subsequent characterization and application evaluation could not be carried out. Therefore, the degree of fluorination modification of 19FCS-1 prepared above was calculated by the ninhydrin reaction method as follows: 19FCS-1, 5.2%.

Example 1-5: Evaluation of bladder mucosa penetration promoting effect of prepared 7FCS: The 7FCS prepared in Example 1-2 was mixed with an aqueous THP solution, and perfused into the bladder through the mouse urethra. Then, frozen sections of the mouse bladder were prepared, and the promotion of the drug carrier on absorption efficiency of the drug in the bladder mucosa was evaluated by detecting the distribution of THP fluorescence in the tissue.

The specific method was as follows: female C57BL/6 mice aged 10-12 weeks were anesthetized with pentobarbital solution. 0.2% THP solution was prepared with 0.5% aqueous FCS solution, and 100 µl of the solution was perfused into the mouse bladder with a closed intravenous indwelling needle. The urethra was clamped for 1h, and then the perfusion solution in the bladder was released. The bladder was flushed with 1ml ultrapure water, the bladder tissue was removed and placed in a tissue embedding machine at -80°C, and then sections were made and examined with a fluorescent confocal microscope. An aqueous pure THP solution of equal concentration or an aqueous THP chitosan solution prepared in the same way was used as a control.

Experimental results: referring to FIG. 1-1, the left side is microscope pictures of mouse bladder tissue sections under different conditions, and the right side is the corresponding data statistics, where the right abscissa is different drug systems, and the ordinate is relative fluorescence intensity.

As shown in FIG. 1-1, a confocal fluorescence microscope was used to observe the mouse bladder tissue sections. The results showed that the distribution area and intensity of the drug fluorescence on the cross section of the bladder of the mice in the 7FCS group were significantly higher than those of chitosan (CS) and the blank control group (aqueous pure THP solution), indicating that FCS can significantly improve the tissue permeability of the drug in the bladder mucosa, and the penetration promoting effect increases with the increase of the degree of fluorination, However, when the degree of fluorination reaches a certain level (7FCS, 42.3%), the prepared FCS has the most significant penetration promoting effect, and at this time, the prepared 7FCS has poorer solubility than low-fluorination substituted chitosan, which is not conducive to clinical use. The above results indicate that FCS can significantly improve the penetration and absorption of drugs and enhance the absorption efficiency of drugs in the bladder mucosa, but excessive fluorination substitution may not be conducive to the application of materials.

Example 1-6: Promotion of 13FCS on absorption of a bladder perfusion drug in the bladder mucosa: The 13FCS prepared in Example 1-3 was mixed with an aqueous THP solution, and perfused into the bladder through the mouse urethra. Then, frozen sections of the mouse bladder were prepared, and the efficiency of the drug carrier on promoting bladder mucosal absorption of the drug was evaluated by detecting the distribution of THP fluorescence in the tissue.

The specific method was as follows: female C57BL/6 mice aged 10-12 weeks were anesthetized with pentobarbital solution. 0.2% aqueous THP solution was prepared with 0.5% aqueous 13FCS solution, and 100 µl of the solution was perfused into the mouse bladder with a closed intravenous indwelling needle. The urethra was clamped for 1h, and then the perfusion solution in the bladder was released. The bladder was flushed with 1ml ultrapure water, the bladder tissue was removed and placed in a tissue embedding machine at -80°C, and then sections were made and examined with a fluorescent confocal microscope. An aqueous THP chitosan solution prepared in the same way was used as a control.

The experimental results are shown in FIG. 1-2. The distribution area and intensity of the drug fluorescence on the longitudinal section of the mouse bladder in the FCS group were significantly higher than that of chitosan (CS), indicating that 13FCS can significantly improve the permeability of the drug in the bladder mucosa; and also, 13FCS-3 has the strongest penetration promoting effect, and its degree of fluorination modification is 21.4%. The above results also indicate that the promoting effect of FCS on penetration of the perfusion drug into the bladder mucosa may be the result of the combined effect of the chitosan cationic skeleton and the fluorinated fatty chain. Of course, this reason may also be diverse.

Example 1-7: In order to screen fluorinated chitosan with the best promoting effect on penetration of a perfusion drug into the bladder mucosa, 19FCS-1 in Example 1-4 and FCS with the best effect among the above-mentioned fluorination modification types were subjected to *in vivo* evaluation in mice.

The specific method was as follows: female C57BL/6 mice aged 10-12 weeks were anesthetized with pentobarbital solution. 0.2% THP solution was prepared with 0.5% aqueous solution of 7FCS-4, 13FCS-3 and 19FCS-1 respectively, and 100 µl of the solution was perfused into the mouse bladder with a closed intravenous indwelling needle. The urethra was clamped for 1h, and then the perfusion solution in the bladder was released. The bladder was flushed with 1ml ultrapure water, the bladder tissue was removed and placed in a tissue embedding machine at -80°C, and then sections were made and examined with a fluorescent confocal microscope. An aqueous pure THP solution of equal concentration or an aqueous THP chitosan solution prepared in the same way was used as a control.

Experimental results: As shown in FIG. 1-3, the mouse bladder tissue sections were observed with a fluorescent confocal microscope. The results showed that the distribution area and intensity of drug fluorescence on the cross-section of the mouse bladder in the 19FCS group were significantly different than those in the THP and CS groups. However, 13FCS-3 has the most prominent effect of promoting the permeability of the perfusion drug.

Example 1-8: In vitro and *in vivo* safety evaluations of different types of fluorinated fluorinated chitosan in Example 1-7 were carried out. The specific experimental protocol was as follows:

The CCK-8 (Cell Counting Kit-8) method (a mature in vitro evaluation method for evaluating cell activity) was used to evaluate the cytotoxic effect of fluorinated chitosan on SV-HUC-1 human normal bladder epithelial cells. In vitro biological safety of fluorinated chitosan was investigated, and the specific operation was as follows: T24 cells were seeded into a 96-well plate at 1×10⁴ cells/well, and incubated overnight at 37°C, 5% CO₂. Serum-free medium with different types of fluorinated chitosan (500 ug/ml) were added for further 24h culture. Then, an appropriate amount of cck-8 was added. Finally, the in vitro safety of fluorinated chitosan was evaluated by cell viability. The experimental results are shown in FIG. 1-4a. 13F-3 (abbreviation of 13FCS-3) has good cell safety in vitro. Combined with the above research results, it is shown that 13F-3 has the most significant effect of promoting the absorption of the perfusion drug into the bladder mucosa, and also has good cell safety in vitro. In order to further evaluate the biosafety of FCS (13F-3), further safety evaluation experiment *in vivo* in mice was carried out.

Healthy C57BL/6 mice aged 10-12 weeks were divided into three groups, 8 mice/each group. The experimental group was perfused with 15 mg/ml fluorinated chitosan or 1% chitosan in aqueous acetic acid solution for 1 h, once a week for three weeks, and the blank control group was perfused with equal volume of double distilled water. The mouse body weight, survival rate, and HE and immunohistochemical analysis (CD45 and Ki67) results of mouse bladder sections on day 28 after the first administration were used to evaluate the *in vivo* biological safety of fluorinated chitosan.

The experiment results showed that the weight of the mice dropped sharply on the second day after perfusion of chitosan, the mice were sluggish in activity, and the mice began to die from the second day after the treatment, and all 8 mice in the experimental group died within 3 days, while no death occurred in the fluorinated chitosan group. As shown in FIG. 1-4b, there was no significant difference between the body weight of the FCS group and the blank control group. In addition, as shown in FIG. 1-4c, the mouse bladder after perfusion in each group was compared with that of the mice in the control group. It was found that the bladder of mice in the chitosan perfusion group was more congested compared with that in the blank group, and the results of HE staining and CD45 and Ki67 immunofluorescence in FIG. 1-5 showed that the bladder of the mice in the chitosan perfusion group had severe inflammatory stress and congestion and edema, while There was no significant difference between the FCS group and the blank control group. The above experimental results show that fluorinated chitosan (13FCS-3) can significantly improve the bioavailability of the perfusion drug in the bladder mucosa, and high concentration of fluorinated chitosan will not cause obvious bladder mucosal and epithelial damage, and has the potential as a drug carrier for bladder perfusion.

Example 1-9: Application of F-PEI Bladder Perfusion Polypeptide Protein Drug Carrier, taking polypeptide drug MPI and protein drug CAT-Ce6 as examples

### (1) Synthesis of fluorinated polyether imide (F-PEI)

The specific operation was as follows: an appropriate amount of 3-(perfluorohex-1-yl)-1,2-propenoxide was slowly added dropwise to a methanol solution of branched polyetherimide (PEI), and stirred at room temperature for 48 h. The crude reaction product was purified by dialysis with methanol/double distilled water (MWCO 3500Da), and freeze-dried to obtain the final product. The structure of the product was identified by 1H NMR, and the average number of fluorine substitutions in the molecule was calculated by fluorine element analysis.

### (2) Preparation and characterization of MPI/F-PEI, CAT-Ce6/F-PEI nano drug systems

MPI/FPEI, CAT/F-PEI NPs could be obtained by mixing polypeptide (MPI), protein (CAT) drug and aqueous F-PEI solution for 2h at room temperature. The hydrated particle size measured by a dynamic light scattering instrument was about 200-300 nm with a small amount of positive charge. It was characterized by transmission electron microscopy (TEM) imaging (FIGs. 1-6, 1-7) as uniform spherical particles.

### (3) Evaluation of bladder mucosa permeability of MPI/F-PEI NPs, CAT-Ce6/F-PEI NPs

The Ussing chamber (also called Ussing perfusion chamber) is a tool for studying transepithelial transport, and can be used for research including ion transport, nutrient transport, and drug transport. Through the study of transepithelial transport, we can understand the absorption of epithelial drugs through the epithelium. In this example, the Ussing chamber was used to evaluate the mucosal permeability of MPI-cy5.5/F-PEI NPs and CAT-ce6/F-PEI NPs prepared with different material ratios. MPI-cy5.5/PEI NPs and CAT-ce6/PEI NPs were respectively used as control. The mice were anesthetized, the bladder was removed and placed on ice, and the bladder mucosa was peeled off and fixed at the interface between the two chambers. 3 ml of MPI-cy5.5/F-PEI NPs or CAT-ce6/F-PEI NPs bench-top solution was added into the diffusion chamber, and an equal volume of blank bench-top solution was added into the receiving chamber. 0.5 ml of the bench-top solution was taken from the receiving chamber every 15 min and simultaneously, an equal volume of the blank bench-top solution was added in the receiving chamber for four consecutive times, and the corresponding drug content was detected by a fluorescence spectrophotometer. The experimental results showed that the mucosal permeability index papp of polypeptide (FIG. 1-8) or protein drug (FIG. 1-9) in the F-PEI group was significantly higher than that of the PEI group and the free group (blank group).

At the same time, we also investigated the permeability of the bladder mucosa in mice. After the mice were anesthetized, the bladder was perfused with solutions containing the same amount of fluorescently labeled polypeptide or protein drug respectively, and for different drug systems in the MPI group (free MPI-cy5.5, MPI-cy5.5/PEI, MPI-cy5.5/F-PEI), frozen sections were prepared from the bladder at different times (15, 30, 60 min) after drug perfusion (FIG. 1-10); for different drug systems in the CAT group (free CAT-ce6, CAT-ce6/PEI, CAT-ce6/F-PEI), frozen sections were prepared from the bladder after 1 h of perfusion, and the fluorescence intensity was analyzed by a fluorescent confocal microscope (FIG. 1-11). The experimental results show that the polypeptide and protein drugs in the F-PEI group have more significant bladder mucosal permeability compared with the PEI and free drug groups, that is, F-PEI can significantly improve the bladder mucosal permeability of polypeptide or protein drugs.

Example 1-10: Application of FCS Bladder Perfusion Protein Drug Carrier, taking the protein drug CAT-TCPP as an example.

### (1) Preparation and characterization of CAT-TCPP/FCS nano drug system

CAT-TCPP/FCS NPs could be obtained by mixing the protein drug (CAT-TCPP) with an aqueous solution of FCS for 2h at room temperature. The hydrated particle size measured by a dynamic light scattering instrument was about 200-300 nm with a small amount of positive charge. It was characterized by transmission electron microscopy (TEM) imaging (FIG. 1-12) as uniform spherical particles.

### (2) Evaluation of bladder mucosa permeability of CAT-TCPP/FCS NPs

We also investigated the permeability of the bladder mucosa in mice. After the mice were anesthetized, the bladder was perfused with solutions containing the same amount of fluorescently labeled protein drug respectively. For different drug systems (free CAT-TCPP, CAT-TCPP/CS, CAT-TCPP/FCS) in the CAT-TCPP group, frozen sections were prepared from the bladder at the same time point (60 min) after drug perfusion (FIG. 1-13), and the fluorescence intensity was analyzed by a fluorescent confocal microscope. The experimental results show that the protein drugs in the FCS group have more significant bladder mucosal retention capacity and permeability compared with the chitosan (CS) and the free drug group, that is, FCS can significantly improve the bladder mucosal permeability of the protein drug.

Unless otherwise specified, the perfluoroheptanoic acid-modified chitosan used in all the examples of the present invention is fluorinated chitosan in Example 1-3 where the feed molar ratio of perfluoroheptanoic acid to N-glucosamine unit is 1: 4.2.

The following is a schematic diagram of the structure of fluorinated chitosan in Example 1-3. 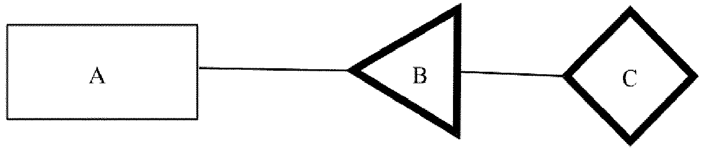 wherein A is a chitosan molecular skeleton containing primary amino groups, and the structural formula is as follows: 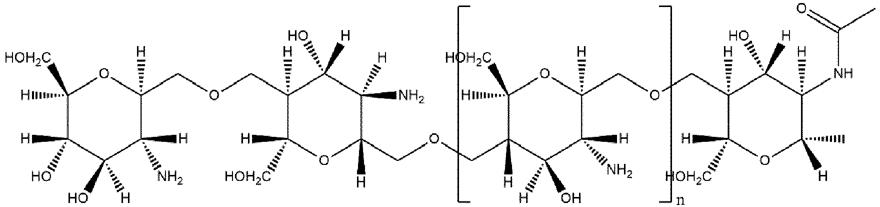 wherein B is a linking group formed by a fluorine-containing functional group and a primary amino group of the chitosan, and is herein an amide bond, namely, 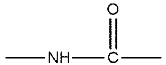 wherein C is a fluorine-containing aliphatic chain or an aromatic ring functional group; perfluoroheptanoic acid is used herein, and the structural formula is as follows:

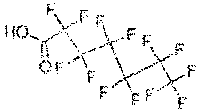

Example 2-1: A transdermal patch was prepared with perfluoroheptanoic acid-modified chitosan as a carrier, and insulin was transdermally delivered for the treatment of diabetes. For the specific preparation process of fluorinated chitosan in this example, refer to Example 2-5.

Specific method:
1. Preparation of a perfluoroheptanoic acid-modified chitosan-insulin composite: perfluoroheptanoic acid-modified chitosan and insulin were respectively dissolved in a weak acid solution environment until uniformly dissolved. A weak base solution was dropwise added during stirring after uniform mixing, the pH was adjusted to 6-7, and under neutral conditions, perfluoroheptanoic acid-modified chitosan and insulin were bound to together due to electrostatic adsorption to form a stable composite, in which the preferred reaction weight ratio of perfluoroheptanoic acid-modified chitosan to insulin was 1:0.25-4, more preferably 1: 0.5-2. After the reaction was complete, it was pre-added with a cryoprotectant for lyophilization, to obtain a perfluoroheptanoic acid-modified chitosan-insulin lyophilized powder. The particle size (FIG. 2-2) and the potential (FIG. 2-3) were analyzed by dynamic light scattering.

As shown in FIG. 2-2, reaction of perfluoroheptanoic acid-modified chitosan and insulin in different ratios all could generate composites. When the weight ratio of perfluoroheptanoic acid-modified chitosan to insulin was 1:0.5-2, the particle size was relatively uniform.

As shown in FIG. 2-3, the different ratios of perfluoroheptanoic acid-modified chitosan to insulin affect the overall potential of the composite.

2. In vitro transdermal kinetic analysis: the insulin in step 1 was replaced with fluorescently labeled insulin, and optimization and transdermal kinetic analysis of the prepared perfluoroheptanoic acid-modified chitosan-insulin (hereinafter referred to as FCS-Insulin) in different ratios were performed. First, perfluoroheptanoic acid-modified chitosan-insulin in different weight ratios was synthesized and put into the injection cell of the Franz vertical diffusion cell, and then the fluorescence intensity of perfluoroheptanoic acid-modified chitosan-insulin that enters the sampling pool through the sandwiched mouse skin was tested to characterize its transdermal effect at different time points. The ratios of perfluoroheptanoic acid-modified chitosan-insulin are 1:0.25, 1:0.5, 1:1, 1:2, 1:4 and 0:1 (pure immunoglobulin G). The abscissa is the penetration time, and the ordinate is the cumulative penetration calculated from fluorescence. The results are shown in FIG. 2-4, and the preferred ratio is 1:1.

3. Preparation of a transdermal patch of a perfluoroheptanoic acid-modified chitosan-insulin composite: perfluoroheptanoic acid-modified chitosan - insulin lyophilized powder was reconstituted and a hydrogel matrix prepared in advance was added and uniformly mixed to obtain a transdermal patch of a perfluoroheptanoic acid-modified chitosan-insulin composite, as shown in FIG. 2-5 on the left. The SEM characterization of the gel is shown in FIG. 2-5 on the right.

4. Measurement of drug release from the gel: the perfluoroheptanoic acid modified perfluoroheptanoic acid-modified chitosan - insulin lyophilized powder was reconstituted and different concentrations of a hydrogel matrix prepared in advance was added and uniformly mixed to obtain a transdermal patch of a perfluoroheptanoic acid-modified chitosan-insulin composite. The concentration of the gel affects the release behavior of the drug from the gel. The gel patch was soaked in a buffer solution and the supernatant of the solution was taken at different time points. According to the Coomassie brilliant blue staining, the cumulative insulin penetration was calculated, as shown in FIG. 2-6.

The abscissa is the time, and the ordinate is the cumulative insulin penetration calculated according to Coomassie Brilliant Blue staining.

5. Evaluation of the penetration enhancing effect of a drug-loaded transdermal patch by changes in blood glucose in mice: female C57BL/6 mice aged 10-12 weeks were anesthetized with isoflurane, and a drug-loaded transdermal patch was applied to the depilated skin on the back of the mouse, and fixed with an elastic bandage, and then the blood glucose fluctuation of the mouse was measured at different time points, and a blank patch was used as a control. FIG. 2-7 shows the blood glucose fluctuations of mice, where the abscissa is the time of action after the patch is applied, and the ordinate is the blood glucose concentration.

As shown in FIG. 2-7, the blood glucose of the mice was monitored by a blood glucose meter, and it is found that compared with the blank control group, hyperglycemia in the mice applied with perfluoroheptanoic acid-modified chitosan drug-loaded transdermal patch is significantly suppressed and remains stable for a long time, which indicates that perfluoroheptanoic acid-modified chitosan can significantly improve the permeability of the drug in the skin, and promote the drug to enter the blood to maintain a stable blood drug concentration, achieving a sustained role. The above results collectively indicate that the fluorine-containing compound-modified cationic polymer can successfully achieve the transdermal delivery of the drug, and has great medical value and transformation value.

Example 2-2: A transdermal ointment was prepared with perfluoroheptanoic acid-modified chitosan as a carrier, and programmed cell death-ligand 1 antibody was transdermally delivered for the treatment of surface melanoma.

Specific method:
1. Preparation of a perfluoroheptanoic acid-modified chitosan-immunoglobulin G composite (hereinafter referred to as FCS-IgG): Since immunoglobulin G and programmed cell death-ligand 1 antibody have the same structure, at the material level, immunoglobulin G was used as a template to study the optimal ratio of binding of perfluoroheptanoic acid-modified chitosan and programmed cell death-ligand 1 antibody. Different amounts of immunoglobulin G were added to an aqueous solution of perfluoroheptanoic acid-modified chitosan and stirred at room temperature for 1 h to form a stable composite. Herein, the reaction weight ratio of perfluoroheptanoic acid-modified chitosan and immunoglobulin G was 1:0.25-4, and was further preferably 1:1 through particle size analysis and potential analysis by dynamic light scattering. The results of particle size distribution and potential distribution are shown in FIG. 2-8.

An immunoglobulin refers to a globulin that has the activity or chemical structure of an antibody and is similar to an antibody molecule. The immunoglobulin G used in the present experiment is not specific. The antibody is an immunoglobulin that specifically binds to an antigen. The programmed cell death-ligand 1 antibody is also a type of immunoglobulin G, but the light chain end is specific, so immunoglobulin G can be used to simulate the behavior of programmed cell death-ligand 1 antibody in non-therapeutic experiments.

Experimental results: referring to FIG. 2-8, the left panel shows the particle size distribution of perfluoroheptanoic acid-modified chitosan-immunoglobulin G in different ratios in an aqueous solution, and the right panel shows the potential distribution. Herein, the 1:1 group that has a better particle size and maintains a higher positive charge is preferred.

2. Preparation of a transdermal ointment of perfluoroheptanoic acid-modified chitosan-programmed cell death-ligand 1 antibody: In the same way, a perfluoroheptanoic acid-modified chitosan-programmed cell death-ligand 1 antibody composite was obtained. An aqueous solution of the obtained perfluoroheptanoic acid-modified chitosan-programmed cell death-ligand 1 antibody was mixed with a blank ointment at a weight ratio of 1:1 to form a transdermal ointment of perfluoroheptanoic acid-modified chitosan-programmed cell death-ligand 1 antibody, in which the main component of the ointment was petrolatum.

3. In vitro transdermal kinetic analysis of perfluoroheptanoic acid-modified chitosan-programmed cell death-ligand 1 antibody: with fluorescently labeled immunoglobulin G as a template to simulate transdermal kinetics of programmed cell death-ligand 1 antibody, optimization and transdermal kinetic analysis of the prepared perfluoroheptanoic acid modified chitosan-immunoglobulin G (hereinafter referred to as FCS-IgG) were performed. First, perfluoroheptanoic acid-modified chitosan-immunoglobulin G in different weight ratios was synthesized and put into the injection cell of the Franz vertical diffusion cell, and then the fluorescence intensity of perfluoroheptanoic acid-modified chitosan-immunoglobulin G that enters the sampling pool through the sandwiched mouse skin was tested to characterize its transdermal effect at different time points. The ratios of perfluoroheptanoic acid-modified chitosan-immunoglobulin G are 1:0.25, 1:0.5, 1:1, 1:2, 1:4 and 0:1 (pure immunoglobulin G). In each group, samples were taken at 2 h, 4 h, 8 h, 12 h and 24 h after drug application, and the cumulative penetration was calculated. The results are shown in FIG. 2-9.

Experimental results: FIG. 2-9 shows cumulative penetration of perfluoroheptanoic acid-modified chitosan-immunoglobulin G (FCS-IgG) in different ratios at different time points. The abscissa is the injection time, and the ordinate is the penetration rate of the cumulative penetration amount divided by the total injection amount. Compared with pure macromolecular protein immunoglobulin G, perfluoroheptanoic acid-modified chitosan-immunoglobulin G in different ratios of all can penetrate the skin of mice to varying degrees. Preferably, a perfluoroheptanoic acid-modified chitosan-immunoglobulin G composite with a ratio of 1:1 is obtained, and used for subsequent experiments.

4. *In vivo* transdermal kinetic analysis of perfluoroheptanoic acid-modified chitosan-programmed cell death-ligand 1 antibody: with fluorescently labeled immunoglobulin G as a template to simulate transdermal kinetics of programmed cell death-ligand 1 antibody, dynamic analysis of *in vivo* skin penetration of the prepared perfluoroheptanoic acid modified chitosan-immunoglobulin G (hereinafter referred to as FCS-IgG) were performed. First, C57 mice were subcutaneously injected with B16 melanocyte suspension (about 1^{∗}10^6 cells/mouse). When the tumor volume was about 60 cubic millimeters, the surface of the mouse tumor was smeared with a perfluoroheptanoic acid modified chitosan-immunoglobulin G ointment in a weight ratio of 1:1, and fixed with a transdermal patch to prevent falling. The mice were killed at different time points, the tumor tissue was removed, the remaining ointment on the surface was wiped off and the epidermis was removed. The tumor tissue was divided into two, half of which was lysed and the fluorescence intensity in the tissue was measured, and the other half of which was sliced for fluorescence imaging under a confocal microscope. The results are shown in FIG. 2-10.

Experimental results: Refer to FIG. 2-10, showing fluorescence imaging with a confocal fluorescence microscope and fluorescence content in tumor after lysis for the tumor tissues dissected respectively at 0h, 4h, 8h, 12h and 24h of the perfluoroheptanoic acid-modified chitosan-immunoglobulin G ointment. It can be clearly found that the penetration rate of the ointment reaches a peak at 12 h and begins to decrease at 24 h. This may be due to the degradation of perfluoroheptanoic acid-modified chitosan-immunoglobulin G that enters the tumor.

5. Comparison of *in vivo* transdermal efficiency of perfluoroheptanoic acid-modified chitosan-programmed cell death-ligand 1 antibody: according to the kinetic analysis, the time point of applying the ointment for 12 h was preferred, and comparison of transdermal efficiency of pure programmed cell death-ligand 1 antibody, chitosan-programmed cell death-ligand 1 antibody and perfluoroheptanoic acid-modified chitosan-programmed cell death-ligand 1 antibody was performed. The same amounts of pure programmed cell death-ligand 1 antibody, chitosan-programmed cell death-ligand 1 antibody and perfluoroheptanoic acid-modified chitosan-programmed cell death-ligand 1 antibody were applied to the tumor site of the tumor-bearing mice, and fixed with transparent films. After 12 h, the mice were sacrificed and the tumor tissues were removed and subjected to immunofluorescence staining and confocal microscopy fluorescence imaging. The results are shown in FIG. 2-4.

Experimental results: see FIG. 2-11. From left to right are DAPI, FITC, and mixed channel fluorescence. DAPI is a nuclear dye that is indicative of the nucleus, and FITC is a fluorescent secondary antibody label for programmed cell death-ligand 1 antibody. It can be seen that the perfluoroheptanoic acid modified chitosan-programmed cell death-ligand 1 antibody group has the strongest transdermal efficiency under the same dosage of programmed cell death-ligand 1 antibody.

7. Transdermal mechanism study of perfluoroheptanoic acid-modified chitosan-programmed cell death-ligand 1 antibody: with immunoglobulin G as a template to simulate programmed cell death-ligand 1 antibody, the transdermal mechanism of perfluoroheptanoic acid-modified chitosan-immunoglobulin G was analyzed. The first was the verification of perfluoroheptanoic acid-modified chitosan-immunoglobulin G (FCS-IgG) for the transdermal effect in a dense cell monolayer. The human skin epithelial cells Hacat were incubated in a Transwell plate as shown in FIG. 2-12. A cell resistance tester was used to detect the resistance changes every 1 day until the growth reached the plateau in which the resistance no longer changed, and at this time, perfluoroheptanoic acid-modified chitosan-immunoglobulin G was added, and the detection of resistance changes was continued. The results are shown in FIG. 2-5.

Experimental results: see FIG. 2-12. The formation of a dense cell monolayer was observed on day 10, and FCS-IgG was added on day 11. The resistance change was detected 24 h later, and a sudden decrease in resistance was seen, indicating that the dense cell monolayer was opened, and then the resistance slowly recovered to a plateau, indicating that the material is temporary for opening tight junctions between cells.

8. Transdermal mechanism study of perfluoroheptanoic acid-modified chitosan-programmed cell death-ligand 1 antibody: with immunoglobulin G as a template to simulate programmed cell death-ligand 1 antibody, changes of related proteins that regulate tight junctions between cells after adding perfluoroheptanoic acid modified chitosan-immunoglobulin G (FCS-IgG) were further analyzed. The human skin epithelial cells Hacat were incubated in a special petri dish for a confocal microscope, and allowed to grow into a dense cell monolayer. Then, 24 h after adding FCS-IgG, the cells in the petri dish were subjected to immunofluorescence staining, and tight junction related proteins were detected respectively: Occludin, Claudin-1, E-Cadherin and ZO-1. The results are shown in FIG. 2-13.

Experimental results: see FIG. 2-13. After adding FCS-IgG, the protein fluorescence content in each group (the white band around the white cells in the figure) decreased significantly. It shows that FCS-IgG can open the tight junctions of cells by changing the distribution of tight junction proteins in the cell membrane, to penetrate the skin through the intercellular space.

9. Transdermal mechanism study of perfluoroheptanoic acid-modified chitosan-programmed cell death-ligand 1 antibody: with immunoglobulin G as a template to simulate programmed cell death-ligand 1 antibody, the mechanism of tight junction protein distribution changes was further studied. The human skin epithelial cells Hacat were incubated in a 25 mm2 petri dish and allowed to grow into a dense cell monolayer. Then, after the cells were lysed, the whole cell proteins were determined by Western Blotting. First, the changes in the content of the four tight junction proteins were detected, and then the changes in the phosphorylation level of actin were detected. The tight junction proteins are Occludin, Claudin-1, E-Cadherin and ZO-1; actin is MLC, phosphated actin is p-MLC; GAPDH is glyceraldehyde-3-phosphate dehydrogenase, which has relatively stable expression in various tissues, and is used here as an internal control. The results are shown in FIG. 2-14.

Experimental results: Refer to FIG. 2-14. The left panel shows the changes in the content of the four tight junction proteins. It can be seen that after adding FCS-IgG, the protein content has no significant change, indicating that FCS-IgG only affects the distribution of the tight junction proteins on the cell membrane surface, rather than reducing their expression, further indicating that this effect is only temporary. The right panel shows the changes in phosphorylation level of actin. It can be seen that there is significant phosphorylation of actin, indicating that FCS-IgG stimulates the paracellular transport by stimulating actin phosphorylation, thereby further opening the intercellular space and promoting the material to penetrate the skin through the intercellular space.

10. *In vivo* subcutaneous tumor treatment with perfluoroheptanoic acid-modified chitosan-programmed cell death-ligand 1 antibody: C57 mice were equally divided into 4 groups, a blank group, a group with intravenous injection of programmed cell death-ligand 1 antibody alone, a chitosan-programmed cell death-ligand 1 antibody group, and a perfluoroheptanoic acid-modified chitosan-programmed cell death-ligand 1 antibody group, respectively. Mice were injected subcutaneously with B16 cell suspension (approximately 1^{∗}10^6 pcs), the tumor size was monitored every day, and the tumor volume was calculated by the following formula: volume=0.5^{∗}tumor length^{∗}tumor width^2. When the initial tumor size was about 10 cubic millimeters, application of an ointment or intravenous administration was started, and the treatment was performed every other day for a total of 4 treatments, and the tumor size was recorded every other day. The results are shown in FIG. 2-8.

Experimental results: see FIG. 2-15. The left panel is a mouse tumor growth curve, and the right panel is a broken line graph of mouse survival rate. The tumor size of 1500 cubic millimeters serves as the standard of mouse death. It can be found that due to the small initial volume, the intravenous injection therapy conventionality used in clinical practice also has a certain inhibitory effect on mouse tumors, but in contrast, the therapeutic effect of perfluoroheptanoic acid-modified chitosan-programmed cell death-ligand 1 antibody is much higher than that of the other groups because the group has an antibody penetration rate of more than 50%.

The fluorine-containing compound-modified cationic polymers in the present invention, especially fluorinated chitosans involves drugs including but not limited to diabetes treatment drugs, anti-tumor drugs (see Table 2-1 below for details), immunomodulators, antiviral drugs, anti-inflammatory drugs, analgesic and anesthetic drugs, medical cosmetic drugs, and the like and derivatives thereof in various dosage forms.

**Table 2-1**

| | | |
|---|---|---|
| Hormone drugs | Estrogen drugs | steroid estrogens such as quinestrol, ethinylestradiol, nilestriol, estradiol benzoate, estradiol 3,17-dipropionate, estradiol valerate, estradiol 17-cyclopentanoate, menstrand, and derivatives thereof |
| | | non-steroidal hormones and derivatives thereof such as diethylstilbestrol and derivatives thereof |
| | | anti-estrogens such as fulvestrant, aminoglutethimide, formestane, anastrozole, letrozole, exemestane, clomiphene, tamoxifen, toremifene, and derivatives thereof |
| | Androgen drugs | androgen drugs such as methyltestosterone, testosterone propionate, and derivatives thereof |
| | | anabolic hormones such as oxymetholone, stanozolol, nandrolone phenylpropionate, metandienone, and derivatives thereof |
| | | anti-androgen drugs such as flutamide, spironolactone, finasteride, abiraterone, and derivatives thereof |
| | Progestogen drugs | progesterone drugs such as progesterone and medroxyprogesterone acetate, and derivatives thereof |
| | | testosterone drugs such as levonorgestrel, and derivatives thereof |
| | | progestogen antagonist drugs such as mifepristone, and derivatives thereof |
| | Adrenal corticosteroids | hydrocortisone, corticosterone, aldosterone, triamcinolone, prednisolone, dexamethasone acetate, methylprednisolone aceponate and the like and derivatives thereof |
| | Prostaglandins | misoprostol and the like and derivatives thereof |
| | Peptide hormones | insulin, calcitonin, oxytocin and the like |
| Signal transduction inhibitors | Tyrosine protein kinase inhibitors | imatinib, dasatinib (Sprycel), nilotinib and the like and derivatives thereof |
| | Cyclooxygenas e inhibitors | aspirin, acetaminophen, non-specific cyclooxygenase inhibitors such as antipyrine, metamizole sodium, phenylbutazone, oxyphenbutazone, mefenamic acid, indometacin, sulindac, diclofenac sodium, ibuprofen, naproxen, piroxicam, meloxicam and derivatives thereof |
| | | specific COX-2 inhibitor drugs such as celecoxib, and derivatives thereof |
| | Calcium ion channel blockers | nifedipine, verapamil, diltiazem, flunarizine, prenylamine and the like and derivatives thereof |
| | Phosphatidyl alcohol inhibitors | kinase puquitinib mesylate and the like and derivatives thereof |
| | Serine protein kinase inhibitors | SP1NK6 antibody (serine protein kinase inhibitor antibody) and the like and derivatives thereof |
| Antitumor drugs | Bioalkylating agents | chlormethine hydrochloride, chlorambucil, melphalan, prenimustine, cyclophosphamide, thiotepa, carmustine, busulfan, cisplatin, carboplatin and the like and derivatives thereof |
| | Anti -metabolic drugs | fluorouracil, cytarabine, mercaptopurine, methotrexate and the like and derivatives thereof |
| | Antitumor antibiotics | actinomycin D, doxorubicin, zorubicin, mitoxantrone and the like and derivatives thereof |
| | Effective components of traditional Chinese | of natural medicine active ingredients such as 10-hydroxycamptothecin, vinblastine sulfate, paclitaxel, docetaxel, and derivatives thereof |
| Angiogenesis inhibitors | Monoclonal antibodies | bevacizumab, ranibizumab and the like and derivatives thereof |
| | Small molecule inhibitors | sorafenib, sunitinib, vandetanib, vatalanib and the like and derivatives thereof |
| | Angiopoietin signaling 1 | Angiopoietin signaling pathway inhibitors and derivatives thereof |
| Immunotherapy drugs | Checkpoint inhibitors | CTLA4 monoclonal antibody (Ipilimumab^{®}), PD-1 monoclonal antibody (Pembrolizumab^{®}, Nivolumab^{®}, Arezolizumab^{®}, Avelumab^{®}, Durvelumab^{®}), PD-LI monoclonal antibody (Atezolizumab^{®}, Avelumab^{®}, Durvalumab^{®}), LAG-3 (lymphocyte activation gene 3) monoclonal antibody, TIM-3 (T-cell immunoglobulin and mucin-domain containing-3) monoclonal antibody, TIGIT (T cell immunoglobulin and ITIM domain protein) monoclonal antibody, costimulatory factors B7-H3, B7-H4 and B7-H5 monoclonal antibodies and the like and derivatives thereof. |
| | Cytokines | IFNa2b, IFNa2a, IL-2, etc. |
| | Oncolytic viruses | recombinant human papgm-csf activated HSV genes, etc. |
| | Bispecific | CD19 and CD3b specific antibodies, etc. |

The drugs may be immunomodulators, including, but not limited to cytokines, BCG, immune checkpoint blocking antibodies, and the like. Cytokines are a class of small molecular proteins with a wide range of biological activities synthesized or secreted by immune cells (such as monocytes, macrophages, T cells, B cells, NK cells, etc.) and some non-immune cells (endothelial cells, epidermal cells, fibroblasts, etc.) upon stimulation. The cytokines include, but are not limited to, interleukins (ILs), interferons (IFNs), tumor necrosis factors (TNFs), colony stimulating factors (CSFs), chemokine family, growth factors (GFs), transforming growth factor-β family (TGF-β family). The interleukins include, but are not limited to, IL-1-IL-38. The colony-stimulating factors include, but are not limited to, G (granulocyte)-CSF, M (macrophage)-CSF, GM (granulocyte, macrophage)-CSF, Multi (multiple)-CSF (IL-3), SCF, EPO etc. The interferons include, but are not limited to, IFN-α, IFN-β, and IFN-γ. The tumor necrosis factors include, but are not limited to, TNF-α and TNF-β. The transforming growth factor-β family includes, but is not limited to, TGF-β1, TGF-β2, TGF-β3, TGFβ1β2, and bone morphogenetic proteins (BMPs). The growth factors include, but are not limited to, epidermal growth factor (EGF), platelet-derived growth factor (PDGF), fibroblast growth factor (FGF), hepatocyte growth factor (HGF), insulin-like growth factor-I (IGF-1), IGF-II, leukemia inhibitory factor (LIF), nerve growth factor (NGF), oncostatin M (OSM), platelet-derived endothelial cell growth factor (PDECGF), transforming growth factor-a (TGF-α), vascular endothelial cell growth factor (VEGF). The chemokine family includes, but is not limited to, four subfamilies: (1) CXC/α subfamily, mainly chemotactic neutrophils, the main members of which are IL-8, melanoma growth stimulating activity (GRO/MGSA), platelet factor-4 (PF-4), platelet basic protein, proteolysis-derived products CTAP-III and β-thromboglobulin, inflammatory protein 10 (IP-10), ENA-78. (2) CC/β subfamily, mainly chemotactic monocytes, the members of which include macrophage inflammatory protein 1α (MIP-1α), MIP-1β, RANTES, monocyte chemotactic protein-1 (MCP-1/MCAF), MCP-2, MCP-3 and 1-309. (3) Type C subfamily, the representative of which is lymphotactin. (4) CX3C subfamily, Fractalkine, which is a CX3C type chemokine and has a chemotactic effect on monocytes-macrophages, T cells and NK cells.

The cytokines include, but are not limited to, cytokines used to treat cancer and cytokines that reduce the side effects of cancer treatment. They play an important role in the normal immune response of the human body and the ability of the immune system to respond to cancer. The cytokines used to treat cancer include, but are not limited to, interferons and interleukins. The cytokines may also be hematopoietic growth factors, which reduce the side effects of cancer treatment by promoting the growth of blood cells destroyed by chemotherapy. The cytokines that reduce the side effects of cancer treatment include, but are not limited to, erythropoietin, IL-11, granulocyte-macrophage colony stimulating factor (GM-CSF) and granulocyte-colony stimulating factor (G-CSF). BCG Vaccine is a live vaccine made from a suspension of attenuated Mycobacterium bovis, which can increase the activity of macrophages, improve the body's cellular immunity, and be used to treat bladder cancer. Immunomodulatory drugs include, but are not limited to thalidomide (Thalomid^{®}), lenalidomide (Revlimid^{®}), pomalidomide (Pomalyst^{®}), imiquimod (Aldara^{®}, Zyclara^{®}). Immune checkpoint blocking antibodies include but are not limited to CTLA4 monoclonal antibody(lpilimumab^{®}), PD-1 monoclonal antibody(Pembrolizumab^{®}, Nivolumab^{®}, Arezolizumab°, Avelumab^{®}, Durvelumab^{®}), PD-L1 monoclonal antibody(Atezolizumab^{®}, Avelumab^{®}, Durvalumab^{®}), LAG-3 (lymphocyte activation gene 3) monoclonal antibody, TIM-3 (T-cell immunoglobulin and mucin-domain containing-3) monoclonal antibody, TIGIT (T cell immunoglobulin and ITIM domain protein) monoclonal antibody, co-stimulatory factors B7-H3, B7-H4 and B7-H5 monoclonal antibodies and the like and derivatives thereof.

The drugs may be anesthetic drugs. Example drugs of general anesthesia include but are not limited to ketamine hydrochloride, propofol, sodium thiopental, etomidate, midazolam and sodium γ-hydroxybutyrate. Local anesthetics include, but are not limited to, aromatic acid esters, aromatic amides, amino ketones, amino ethers, carbamates, hydroxyprocaine, chloroprocaine, tetracaine, butacaine, thiocaine, procainamide, bupivacaine, articaine, etidocaine, ropivacaine, mepivacaine, clonin and the like and derivatives thereof.

The drugs may be diabetes treatment drugs including, but not limited to, sulfonylureas such as sulfambutamide, tolbutamide, chlorpropamide, acetohexamide, gliclazide, glipyride, glimepiride, and derivatives thereof; non-sulfonylureas such as repaglinide, nateglinide, and derivatives thereof; thiazolidinediones such as rosiglitazone, pioglitazone, and derivatives thereof; biguanides such as phenformin, metformin, and derivatives thereof; α-glucosidase inhibitors such as acarbose, voglibose, miglitol, and derivatives thereof; dipeptidyl peptidase-IV drugs such as glucagon-like peptide, DPP-IV inhibitors, sitagliptin, vildagliptin, and saxagliptin, and insulin and the like and derivatives thereof.

Diabetes is a metabolic endocrine disease mainly characterized by hyperglycemia, and the clinical dosage form is generally insulin injection. Patients need to endure the pain of repeated injections, and long-term medication can also cause side effects such as inflammation and induration at the injection site. The fluorine-containing compound-modified chitosan drugs can penetrate the skin, carry hypoglycemic drugs into the blood, and improve the bioavailability of the drugs. As described in Example 2-1, the fluorine-containing compound-modified chitosan drugs can be used as a drug carrier to deliver hypoglycemic drugs, which can be administered in the form of drug patches for the treatment of diabetes. By use of transdermal administration with fluorine-containing compound-modified chitosan drug patches, the effective concentration of the drugs is maintained for a long time, and the degree of action and maintenance time can be adjusted according to the area of application and the application time, which has the advantages of flexibility and convenience. In addition, it can also be prepared into a more flexible lotion, liniment, smear and other dosage forms.

The drugs may be anti-tumor drugs (see Table 2-1 for details). Although transdermal delivery is a non-invasive way of delivery, it brings great convenience, the stratum corneum barrier of the skin often prevents the drug from entering the subcutaneous lesion or even entering the blood vessel. Melanoma is a malignant tumor that originates from cells that can produce melanin. It is easy to metastasize, has strong drug resistance, poor prognosis, and extremely high mortality. Melanoma chemotherapy drugs are mainly delivered by oral and injection methods, but this often causes many adverse reactions. It even leads to organ damage, and also it is unable to deliver drugs efficiently, accurately and controllably. The transdermal delivery mode has unique advantages for the treatment of subcutaneous melanoma, but higher requirements for the efficiency of transdermal delivery is put forward. As described in b2-2, the fluorine-containing compound-modified chitosan can be used as a drug carrier to deliver anti-tumor drugs, which can be administered in the form of ointments for the treatment of tumor diseases.

The fluorinated chitosan in each of Examples 2-1 to 2-6 of the present invention can be used as a transdermal preparation, and is useful as a transdermal administration preparation for diabetes treatment drugs, tumor disease drugs, and anti-inflammatory drugs. Also, it can also be used as a transdermal administration preparation in the preparation of a medical cosmetic drug, a topical drug preparation, a topical preparation for medical devices, and a cosmetic skin care product.

Example 3-1: An oral drug was prepared with perfluoroheptanoic acid-modified chitosan as a carrier, and insulin was orally delivered for the treatment of treat diabetes.

Specific method:
1. Preparation of perfluoroheptanoic acid-modified chitosan-insulin capsules: perfluoroheptanoic acid-modified chitosan and insulin were dissolved in a weak acid solution until uniformly dissolved, a weak base solution was dropwise added under stirring after uniform mixing, the pH was adjusted to 6-7, and under neutral conditions, the perfluoroheptanoic acid-modified chitosa and the insulin bound together due to electrostatic adsorption to form stable nanoparticles. After the reaction was complete, it was pre-added with a cryoprotectant for lyophilization, to obtain a perfluoroheptanoic acid-modified chitosan-insulin lyophilized powder. The lyophilized powder was filled into capsules and wrapped in enteric coating.
2. The perfluoroheptanoic acid-modified chitosan - insulin lyophilized powder was reconstituted, and the particle size (FIG. 3-3) and the potential (FIG. 3-4) were analyzed by dynamic light scattering.
3. The perfluoroheptanoic acid-modified chitosan-insulin before and after lyophilization was subjected to particle size analysis by dynamic light scattering, and as shown in FIG. 3-5, there was no significant change before and after lyophilization.
4. *In vitro* transdermal kinetic analysis: the insulin in step 1 was replaced with fluorescently labeled insulin, and optimization and transdermal kinetic analysis of the prepared perfluoroheptanoic acid-modified chitosan-insulin (hereinafter referred to as FCS-Insulin) in different ratios were performed. First, perfluoroheptanoic acid-modified chitosan-insulin in different weight ratios was synthesized and put into the donor chamber of the Franz vertical diffusion cell, and then the fluorescence intensity of perfluoroheptanoic acid-modified chitosan-insulin that enters the receptor chamber through the mouse mucosa was tested to characterize its transdermal effect at different time points. The results are shown in FIG. 3-6, and the preferred ratio is 1: 0.5-2.
5. The drug-loaded capsule was put into simulated gastric juice or simulated intestinal juice, and the releasing effect in simulated gastric juice or simulated intestinal juice was characterized by the fluorescence intensity of the perfluoroheptanoic acid-modified chitosan-insulin released in the solution. As shown in FIG. 3-7, the left side is the release behavior in simulated gastric juice, and the right side is the release behavior in intestinal juice. The capsule can remain stable and is not released for a long time in the simulated gastric juice, while the drug can be released in the simulated intestinal juice.
6. Evaluation of the administration effect by changes in blood glucose of mice: perfluoroheptanoic acid-modified chitosan-insulin capsules were lavaged, and then blood glucose fluctuations in mice were measured at different time points, and a blank capsule was used as a control.

FIG. 3-8 shows the blood glucose fluctuations of mice, where the abscissa is the time after capsules were lavaged, and the ordinate is the blood glucose concentration.

As shown in FIG. 3-8, the blood glucose of the mice was monitored by a blood glucose meter, and it is found that compared with the blank control group, hyperglycemia in the mice lavaged with perfluoroheptanoic acid-modified chitosan insulin capsules is significantly suppressed and remains stable for a long time, which indicates that perfluoroheptanoic acid-modified chitosan can significantly improve the retention and penetration of the drug in the intestine, and promote the drug to enter the blood to maintain a stable blood drug concentration, achieving a sustained role. The above results collectively indicate that the fluorine-containing compound-modified cationic polymer can successfully achieve the oral delivery of the drug, and has good medical and transformation values.

Example 3-2: An oral capsule was prepared with perfluoroheptanoic acid-modified chitosan as a carrier, programmed cell death-ligand 1 antibody was orally delivered, and the mucous membrane penetrating effect of perfluoroheptanoic acid-modified chitosan-programmed cell death-ligand 1 antibody particles in different ratios was observed.

Specific method:
1. Preparation of perfluoroheptanoic acid-modified chitosan-programmed cell death-ligand 1 antibody particles: 0.2 mg of perfluoroheptanoic acid-modified chitosan was weighted and dissolved in 0.5 mL of ultrapure water to obtain 0.4 mg/mL of perfluoroheptanoic acid-modified chitosan, and gradient dilution was performed to finally obtain 0.4 mg/mL, 0.2 mg/mL, 0.1 mg/mL, 0.05 mg/mL, and 0.0025 mg/mL solutions of perfluoroheptanoic acid-modified chitosan. Fluorescently labeled immunoglobulin G was used as a template to replace programmed cell death-ligand 1 antibody, and 0.5 mL 0.1 mg/mL of FITC-labeled immunoglobulin G in 0.02M potassium phosphate buffer at pH = 7.2 was added dropwise under constant stirring, and stirred at room temperature for 30 min.
2. *In vitro* kinetic analysis through the intestinal mucosa of perfluoroheptanoic acid-modified chitosan-programmed cell death-ligand 1 antibody: the rat intestine was removed of the fascia layer, and fixed at the upper part of the transdermal diffusion cell. Perfluoroheptanoic acid-modified chitosan-FITC-immunoglobulin G particles with different weight ratios were added at the upper part of the transdermal diffusion cell, and a PBS solution was added at the lower part of the transdermal diffusion cell. The liquids were taken at the lower part of the transdermal diffusion cell at 0min, 15min, 30min, 45min, 60min, 90min, 120min, 150min, 180min, and 210min, the fluorescence intensity was measured, and the cumulative penetration rate was calculated. The results are shown in FIG. 3-9.

Experimental results: FIG. 3-9 shows cumulative penetration of perfluoroheptanoic acid-modified chitosan-immunoglobulin G (FCS-IgG) in different ratios at different time points. The abscissa is the injection time, and the ordinate is the penetration rate of the cumulative penetration amount divided by the total injection amount. Compared with pure macromolecular protein immunoglobulin G, perfluoroheptanoic acid-modified chitosan-immunoglobulin G in different ratios of all can penetrate the skin of mice to varying degrees. Preferably, perfluoroheptanoic acid-modified chitosan-immunoglobulin G nanoparticles with a ratio of 1:1 are obtained, and used for subsequent experiments.

3. Preparation of lyophilized powder of perfluoroheptanoic acid-modified chitosan-programmed cell death-ligand 1 antibody particles: 0.1 mg of perfluoroheptanoic acid-modified chitosan was weighted and dissolved in 1 mL of ultrapure water to obtain 0.1 mg/mL of a perfluoroheptanoic acid-modified chitosan solution. 0.5 mL 0.1 mg/mL of cy5.5-labeled immunoglobulin G in 0.02M potassium phosphate buffer at pH = 7.2 was added dropwise under constant stirring, and stirred at room temperature for 30 min. After the stirring was complete, a lyoprotectant was added, and the solution was pre-cooled in a refrigerator at -20°C and then dried in a freeze dryer.

4. Preparation of perfluoroheptanoic acid-modified chitosan-programmed cell death-ligand 1 antibody capsules and targeted release properties thereof: a certain amount of lyophilized powder was weighed and filled into dedicated capsules for mice, and coating was performed with Eudragit S100. The mice were fed the above capsules with an applicator, and the mice were sacrificed at 3h and 5h, the intestines were removed, and small animal imaging was used to photograph the fluorescence distribution of cy5.5 in the intestines to determine the distribution of the capsules.

Experimental results: Refer to FIG. 3-10. Fluorescence can be observed in the colorectal area at 3h and 5h, indicating that the capsule can be targeted to the colorectal release. Therefore, the capsule was selected for subsequent experiments.

5. Activity determination of lyophilized powder of perfluoroheptanoic acid-modified chitosan-programmed cell death-ligand 1 antibody particles before and after lyophilization: the frozen programmed cell death-ligand 1 was diluted in a carbonate buffer to different concentrations, and added to an enzyme-linked immunosorbent assay (ELISA) plate, and the plate was placed at 4°C overnight. After the coating was complete, it was washed with PBST three times, and a BSA solution was added and blocking was performed for 2h at room temperature. After the blocking was complete, it was washed with PBST three times, and the programmed cell death-ligand 1 antibody before and after lyophilization was added and incubated at room temperature for two hours. After the incubation was complete, it was washed with PBST three times, and horseradish peroxidase-labeled goat anti-rat secondary antibody was added and incubated at room temperature for one hour. After the incubation was complete, the color-developing agent TMB was added, and then a stop solution was added to stop the color development. The absorbance of the ELISA plate at OD450 was determined to calculate the antibody affinity before and after lyophilization.

Experimental results: Refer to FIG. 3-11. After a lyoprotectant is added for lyophilization, the activity of the programmed cell death-ligand 1 antibody after turning into a lyophilized powder keeps unchanged. It shows that the perfluoroheptanoic acid-modified chitosan-programmed cell death-ligand 1 antibody particles can be freeze-dried and can be used for filling of oral capsules.

6. Binding activity of FCS/a PD-L1 and PD-L1 on CT-26 cell surface before and after lyophilization
(1) 500 ng of IFN-γ was added to 10 mL of 1640 medium to serve as a culture medium for CT-26 cells, and co-incubated with CT-26 cells for 36 h.
(2) the adherent CT-26 was scraped off with a cell scraper, and washed three times with FACS buffer (50 mL PBS+0.5 mL serum), and the supernatant was discarded.
(3) 1^{∗}10⁶ cells were added to each 1.5 mL centrifuge tube and FCS/a PD-L1 was added with the content of α PD-L1 being 1 µg, incubation was performed at room temperature for 0.5 h, the supernatant was discarded, and the cells were washed three times with FACS buffer, and then the supernatant was discarded.
(4) 0.5 µL of PE anti PD-L1 antibody was added to each 1.5 mL centrifuge tube and incubated for 30 min at room temperature, the supernatant was discarded, and the cells were washed 3 times with FACs buffer. Finally, 200 µL FACS buffer was added.
(5) Binding activity of FCS/a PD-L1 and PD-L1 on CT-26 cell surface after lyophilization was tested with a flow cytometer. The results are shown in FIG. 3-13.

The experimental results are shown in FIG. 3-12. It can be seen that the binding capacity of FCS/a PD-L1 and PD-L1 on CT-26 cell surface after lyophilization is not much different from the binding activity of FCS/a PD-L1 before lyophilization. FCS/a PD-L1 can be used to further make capsules.

**.** 7. Changes in tight junction proteins in epithelial cells of human colorectal cancer before and after treatment with perfluoroheptanoic acid-modified chitosan and perfluoroheptanoic acid-modified chitosan/immunoglobulin G.
(1) 0.1 mg/mL FCS/IgG or FCS solution in medium was formulated, and then placed in a confocal small dish with CaCO-2 monolayer cells cultured, and incubated in a constant-temperature incubator at 37°C for 5h.
(2) the culture medium was removed, and the cells were washed three times with PBS for 5 min each time.
(3) 4% paraformaldehyde solution was added to each well, and the cells were fixed on ice for 20 min, and washed three times with PBS for 5 min each time.
(4) 0.1% Triton x-100 solution was added to each well, and the cells were left for 15 min, and washed three times with PBS for 5 min each time.
(5) 2% BSA solution was added to each small dish, and the cells were blocked at room temperature for 1 h.
(6) ZO-1 antibody solution was added at 1:200 or E-Caclherin antibody was added at 1:1000, and the temperature was maintained at 4 °C overnight; the cells were removed and washed three times with PBS for 5 min each time.
(7) FITC Goat anti rabbit was added to each well, and the cells were incubated for 1 h at room temperature, and washed three times with PBS.
(8) DAPI solution was added, and the cells were incubated for 5 min, and washed three times with PBS for 5 min each time.
(9) Pictures were taken with a confocal microscope, and the experimental results are shown in FIG. 3-13.

The experimental results are shown in FIG. 3-13. It can be seen that after FCS/IgG is added, the tight junction proteins in the cells become loose and the cytoskeleton rearranges.

8. Treatment of colorectal cancer in mice with perfluoroheptanoic acid-modified chitosan-programmed cell death-ligand 1 antibody
(1) 1 mg of perfluoroheptanoic acid-modified chitosan was weighted and dissolved in 1 mL of ultrapure water to obtain 1 mg/mL of a perfluoroheptanoic acid-modified chitosan solution. 1 mL of 1 mg/mL programmed cell death-ligand 1 antibody was added dropwise under constant stirring, and stirred at room temperature for 30 min. After the stirring was complete, a lyoprotectant was added, and the solution was pre-cooled in a refrigerator at -20°C and then dried in a freeze dryer.
(2) Lyophilized powder was weighed and filled into dedicated capsules for mice, and coating was performed with Eudragit S100. The prepared capsules were stored for future use.
(3) Balb/c mice were anesthetized with 1% sodium pentobarbital. The mice were fixed with the abdomen facing up, a small opening was made on the right side of the abdomen, the cecum was removed, and 500,000 CT-26 cells transfected with luciferase were injected into the wall of the cecum. Then, the cecum was put back, the wound was sutured, and treatment was started four days later.
(4) The mice were fed the capsules prepared in step 2 with an applicator, and then 100 µL of metoclopramide hydrochloride was gavaged to promote gastric emptying of the mice. The capsules were given on day 4, 7, 12, and 16 after the tumor was re-implanted.
(5) On the third day after administration, Balb/c mice were anesthetized with 1% sodium pentobarbital, and each mouse was injected with a bioluminescent substrate. Ten mins later, the mice were imaged and the tumor growth was observed. The experimental results are shown in FIG. 3-14.

The experimental results are shown in FIG. 3-14. It can be seen that after treatment with the capsules containing perfluoroheptanoic acid-modified chitosan-programmed cell death-ligand 1 antibody, the tumor growth trend can be significantly suppressed. This indicates that perfluoroheptanoic acid-modified chitosan can significantly improve the penetration of drugs in the colon, and promote the entry of drugs into tumor tissues, thereby achieving a sustained role.

9. The effect through the intestinal mucosa with different molecular weights of perfluoroheptanoic acid-modified chitosan-programmed cell death-ligand 1 antibody
(1) Preparation of different perfluoroheptanoic acid-modified chitosan-programmed cell death-ligand 1 antibody particles: perfluoroheptanoic acid-modified chitosan with molecular weights of 10Kda, 50Kda, 100Kda, 300Kda, and 400Kda were weighted and dissolved in ultrapure water to obtain 0.4 mg/mL of perfluoroheptanoic acid-modified chitosans. Fluorescently labeled immunoglobulin G was used as a template to replace programmed cell death-ligand 1 antibody, and 0.5 mL 0.1 mg/mL of FITC-labeled immunoglobulin G in 0.02M potassium phosphate buffer at pH = 7.2 was added dropwise under constant stirring, and stirred at room temperature for 30 min.
(2) *In vitro* kinetic analysis through the intestinal mucosa of perfluoroheptanoic acid-modified chitosan-programmed cell death-ligand 1 antibody: the rat intestine was removed of the fascia layer, and fixed at the upper part of the transdermal diffusion cell. Perfluoroheptanoic acid-modified chitosan-FITC-immunoglobulin G particles with different weight ratios were added at the upper part of the transdermal diffusion cell, and a PBS solution was added at the lower part of the transdermal diffusion cell. The liquids were taken at the lower part of the transdermal diffusion cell at 0min, 15min, 30min, 45min, 60min, 90min, 120min, 150min, 180min, and 210min, the fluorescence intensity was measured, and the cumulative penetration rate was calculated. The results are shown in FIG. 2-7.

As shown in FIG. 3-15, the perfluoroheptanoic acid-modified chitosans of different molecular weights have different effects on increasing the penetration of drugs through the mucosa. This indicates that the perfluoroheptanoic acid-modified chitosans can promote the drugs to enter tumor tissues.

Example 3-3: perfluoroheptanoic acid-modified chitosan-immunomodulator particles were prepared with perfluoroheptanoic acid-modified chitosan as a carrier, and administered as sprays/inhalants, and the ability of drug delivery to the lungs was investigated.

Specific method:
1. Preparation of perfluoroheptanoic acid-modified chitosan-antibody particles: the programmed cell death-ligand 1 antibody was added to the aqueous solution of perfluoroheptanoic acid-modified chitosan and stirred for 1 h at room temperature to form stable nanoparticles.
2. The perfluoroheptanoic acid-modified chitosan-antibody particles were delivered as aerosol via a pulmonary administration needle, tissue sections were made 24 h after delivery, and the retention of fluorescent signals in the lung tissue was observed. The results are shown in FIG. 3-16.

The experimental results are shown in FIG. 3-16. Perfluoroheptanoic acid-modified chitosan-antibody particles can stay in the lungs.

Example 3-4: With bovine serum albumin (BSA-Cy5.5) labeled with Cy5.5 fluorescent molecule as a model protein, and perfluoroheptanoic acid-modified chitosan (FCS) as a transnasal mucosa carrier, perfluoroheptanoic acid-modified chitosan-bovine serum albumin nanoparticles were prepared, and administered as nasal drops, and the delivery efficiency of BSA through the nose to the brain in a mouse glioma model was investigated by the fluorescence signal of Cy5.5.

Specific method:
1. Preparation of FCS/BSA-Cy5.5 nanoparticles: perfluoroheptanoic acid-modified chitosan was dissolved in 1% acetic acid until completely dissolved, and a weak base was added dropwise to adjust the pH of the solution to 6-7. Then, it was uniformly mixed with the BSA-Cy5.5 solution, and stirred at 4°C for 1 h to form stable nanoparticles as nasal drops. Herein, the preferred reaction weight ratio of perfluoroheptanoic acid-modified chitosan and BSA was 1:1, and the final bovine serum albumin concentration was 2.5 mg/mL.
2. Construction of a glioma model: male C57BL/6 mice (about 20 g/mouse) aged 7-8 weeks were intra-cerebrally injected with EGFP-expressing glioma cells (5000 cells/mouse) to construct a glioma model.
3. Administration with nasal drops: the successfully modeled C57BL/6 mice were anesthetized, and fixed on a heating pad in a supine position. A total volume of 20 µL of FCS/ BSA-Cy5.5 solution (with free BSA-Cy5.5 solution as a control group) was slowly dripped into the mouse nostrils with a pipette, and after the addition was completed, the supine position of the mouse was further maintained for 30 min.
4. 72 h after nasal drip, the mice were anesthetized and perfused with formalin, then the brain tissue was removed, lyophilized and sectioned, and the fluorescence signal of Cy5.5 at the tumor tissue in the mouse brain was observed by a confocal microscope.

The experimental results are shown in FIG. 3-17. Compared with the control groups free BSA-Cy5.5 and CS/BSA-Cy5.5, the experimental group FCS/BSA-Cy5.5 has the best brain fluorescence intensity at each time point, indicating that FCS has the best auxiliary effect for BSA delivery through the nasal mucosa to the brain.

Referring to FIG. 3-18, the left panel represents the EGFP signal of brain tumor in mouse brain tissue, and the right panel represents the Cy5.5 signal of BSA delivered to the brain via the nose. It can be seen from the results on the right panel that compared with the control groups free BSA-Cy5.5 and CS/BSA-Cy5.5, in the experimental group FCS/BSA-Cy5.5, the intact mouse brain tissue removed after nasal drip operation has the highest Cy5.5 fluorescence intensity, indicating that FCS/BSA-Cy5.5 has the best nasal to brain delivery efficiency.

Referring to FIG. 3-19, EGFP represents tumor tissue and Cy5.5 represents IgG. By comparing the Cy5.5 signal, it can be seen that the Cy5.5 signal of the brain tumor site in the FCS group is the strongest, indicating that FCS has the highest nasal to brain delivery efficiency of BSA.

Example 4-1: Taking bovine serum albumin labeled with Cy5.5 fluorescent substance and small molecule fluorescent substance Rhodamine B as examples, fluorinated chitosan was used to encapsulate the drugs, and length and depth of penetration in the eye were observed.

Specific method:
1. The experimental rabbit eyes were removed for dissection, the rabbit cornea was separated and fixed on the Franz diffusion cell, and compared to the free protein, the transmembrane effect of the protein at different time points after mixing with different proportions of perfluoroheptanoic acid-modified chitosan was monitored. Herein, the ratios of protein to perfluoroheptanoic acid-modified chitosan are1:0.25, 1:0.5, 1:1, and 1:4. In each group, samples were taken at 5 min, 30 min, 1 h, 3 h, 6 h, 12 h, and 24 h after drug application. Finally, the cumulative penetration was calculated by detecting the fluorescence of the label on the protein. The results are shown in FIG. 4-3. Finally, the ratio of protein and perfluoroheptanoic acid-modified chitosan of 1:1 was selected for animal experiments.
2. Preparation of fluorine-containing chitosan eye drops: the normal pH of tear fluid is 6.4-7.7, so a phosphate buffer solution with pH=7.4 was used to dissolve the solid powder of fluorine-containing chitosan, with a concentration of 2 mg/mL. A high concentration of bovine serum albumin labeled with Cy5.5 was added dropwise under constant stirring to form a stable composite, and the final concentration of bovine serum albumin was 2 mg/mL. The eye drops of perfluoroheptanoic acid-modified chitosan and small molecule rhodamine B were consistent with the above preparation.
3. To test the ability of perfluoroheptanoic acid-modified chitosan to penetrate the eye barrier, the degrees of penetration of the protein with perfluoroheptanoic acid-modified chitosan and the free protein into the inside of the eye were compared. The mice were anesthetized and administered on the ocular surface with a dosing device, 5 µL per eye, and the mice were treated under protection from light for 12 h. The control group was bovine serum albumin labeled Cy5.5 without fluorine-containing chitosan, and the concentration and dosage were the same as the experimental group. 12h later, the mice were sacrificed by cervical dislocation, and the surface of the eyeball was rinsed with PBS at pH=7.4, the eyeball was removed, the excess tissue was disposed, and the section preparation was made, and the central longitudinal section of the eyeball was photographed. The specific implementation method of the small molecule drug rhodamine B was the same as that of the large molecule drug. The membrane penetration effect of large molecule bovine serum albumin is shown in FIG. 4-4, and the membrane penetration effect of small molecule Rhodamine B is shown in FIG. 4-5. It is obvious from the figures that the protein and small molecule fluorescent substance rhodamine B with perfluoroheptanoic acid-modified chitosan can enter the inside of the eyeball, and that the fluorescence intensity of the protein label and the fluorescence of the small molecule are much higher than that of free protein and free small molecule in the eyes. It is concluded that the perfluoroheptanoic acid-modified chitosan can help a series of drugs to penetrate the eye barriers and enter the eyes to achieve the purpose of treatment.
4. In order to evaluate the protein drug concentrations in the eye at different time points, drug concentrations in each part of the dissected eyeball (cornea, lens, vitreous, retina) 3 h and 6 h after administration with the 1:1 ratio of perfluoroheptanoic acid-modified chitosan to protein and the free protein were compared. After the eyeball was dissected, it was broken by a tissue disrupter, and then lysed with a lysis solution, and centrifuged to remove the broken pieces. The supernatant is obtained, the fluorescence of the protein label was detected and the fluorescence intensity was calculated, as shown in FIG. 4-6. First, the protein fluorescence intensities of the drug with perfluoroheptanoic acid-modified chitosan and the free drug in the four tissue parts of the eye were compared. It is obvious that the group with perfluoroheptanoic acid-modified chitosan is significantly higher than the free protein group. Secondly, according to the accumulation of time, the group with perfluoroheptanoic acid-modified chitosan protein at 6 h has higher fluorescence intensity than that at 3 h. It can be seen that the perfluoroheptanoic acid-modified chitosan may have the effect of adhering to the ocular surface and slowly releasing into the eye.
5. In order to evaluate the penetrating ability for the cornea, the cornea was subjected to immunofluorescence staining at different time points to observe the penetrating ability. Samples were taken 5 min, 15 min, 30 min, and 60 min after the drug with perfluoroheptanoic acid-modified chitosan was applied. Frozen sections were prepared, and the nuclei of the sections were stained and observed under a confocal microscope as shown in FIG. 4-7. Herein, the red is the fluorescence of the protein label, and the blue is the nucleus of the eye tissue. It can be clearly seen from the figure that after 30 min, the group with perfluoroheptanoic acid-modified chitosan has clearly penetrated into the corneal epithelial cells, while the free protein substantially does not enter. It can be seen that the perfluoroheptanoic acid-modified chitosan can open the corneal barrier for targeted treatment of cornea related diseases.
6. In order to evaluate the biological safety of perfluoroheptanoic acid-modified chitosan, 20 Balb/c mice aged 6-8w were selected, regardless of gender, and the mice were divided into a perfluoroheptanoic acid-modified chitosan group, a saline group, a PBS group, and a blank control group, 5 mice in each group. The eye drop frequency was 4 times/day. The sodium fluorescein staining of the corneal epithelium was recorded by a slit-lamp biomicroscope at 24h, 48h and 72h respectively after administration, and the evaluation was carried out according to the clinical evaluation criteria in Table 4-3. The evaluation pictures are shown in FIG. 4-8. In the perfluoroheptanoic acid-modified chitosan group, 24 h after administration, the defect areas are reduced, and the defect areas are all < 30%; after 48 h, there are defects in the corneal epithelium of 2 mice, and the epitheliums of the other 3 mice are completely healed; after 72 h, the corneal epitheliums of 5 mice are completely healed. In the saline group: 24 h after administration, the defect areas are reduced, the defect areas in the corneal epithelium of 2 mice are between 30% and 70%, and those in the other three mice are all < 30%; after 48 h, there is a defect in the corneal epithelium of 1 mouse, and the defect area is < 30%, and the epitheliums of 4 mice are completely healed; after 72 h, there is a defect in the corneal epithelium of 1 mouse, and the defect area is < 30%, and the epitheliums of 4 mice are completely healed. In the PBS group: 24 h after administration, the defect areas are reduced, and the defect areas are all < 30%; after 48 h, there is punctate staining in the corneal epithelium of 1 mice, and the epitheliums of 4 mice are completely healed; after 72 h, the corneal epitheliums of 5 mice are completely healed. In the blank control group, 24 h after administration, the defect areas are reduced, and the defect areas are all < 30%; after 48 h, the corneal epitheliums of 5 mice are completely healed. Under the test of this clinical method, the perfluoroheptanoic acid-modified chitosan has extremely high safety to the eyes. The cornea stained with sodium fluorescein under the slit lamp in FIG. 8 is stained if there is a corneal defect, and should appear gray in black and white mode. Table 4-4 shows the evaluation results of FIG. 4-8.

**Table 4-3 Clinical Laboratory Reference**

| Grading | Criterion |
|---|---|
| 0% trauma | Epithelial defect healed, fluorescein staining turned negative |
| 20% trauma | Repair of defect area >70%, fluorescein staining ++ ^ +, or ++, +^very litt e ± |
| 80% trauma | Repair of defect area 30%-70%, corneal staining +++^++, or ++ ^ |
| 100% trauma | Repair of defect area <30%, no significant change in corneal staining |

Note: cure and markedly improvement are summed to be effective, and the effectiveness rate is calculated; improvement and failure are summed to be ineffective, and the ineffectiveness rate is calculated.

**Table 4-4**

| | 0 h | 24 h | 48 h | 72 h |
|---|---|---|---|---|
| Fluorinated chitosan group | 100% trauma | 100% trauma | 80% trauma | 0% trauma |
| Saline group | 100% trauma | 80% trauma | 20% trauma | 20% trauma |
| Phosphate buffer solution group | 100% trauma | 100% trauma | 20% trauma | 0% trauma |
| Blank control group | 100% trauma | 0% trauma | 0% trauma | 0% trauma |

It is obvious from the evaluation results in FIG. 4-8 and Table 4-4 that the cornea on the surface of the eyeballs of the experimental group and the control group has no visible gray areas showing corneal epithelial defects. It can be seen that perfluoroheptanoic acid-modified chitosan has substantially no effect on the cornea and is highly safe.

Example 4-2: Anti-PDLl as an immunotherapeutic drug and perfluoroheptanoic acid-modified chitosan were prepared into eye drops for the treatment of malignant choroidal melanoma, to demonstrate that perfluoroheptanoic acid-modified chitosan has a delivery effect.

Specific method:
1. Animal model: B16 melanoma in logarithmic phase transfected with bioluminescence gene was intra-ocularly injected into the choroid in the eyeball of the right eye of Balb/c mice at 1×10⁵ cells/each eye. After the injection, they were incubated for 4 days, and the tumor size was expressed by the bioluminescence intensity through a bioluminescence imaging system. FIG. 4-9 shows the formation and size of ocular tumors in each group before administration.
2. Preparation of perfluoroheptanoic acid-modified chitosan/anti-PDL1 eye drops: The preparation method was the same as that of item 2 in Example 4-1.
3. Evaluation method: the rightmost side in FIG. 4-9 is a diagram indicating the change in bioluminescence intensity. In the legend, the larger the spot, the darker the color, the more serious the tumor.

Treatment method: the successfully modeled mice were divided into groups, one without administration as a control group, and one given with eye drops as an experimental group, with three mice in each group. The treatment was started on the fourth day after the model was established. The experimental group was instilled once a day, with 2.5 µL each time, and the drug concentration was 2 mg/mL. After one week of treatment, bioluminescence imaging was performed. The results are shown in FIG. 4-9 and FIG. 4-10, and in FIG. 4-9, the upper three mice are in the control group, and the lower three are in the experimental group. It is obvious from the figures that the immune bioluminescence of the mice with perfluoroheptanoic acid-modified chitosan is weaker than the control group after one week of treatment, and the in situ bioluminescence quantitative analysis of malignant choroidal melanoma after one week of treatment shows that the autoluminescence intensity after treatment is one-fourth that of the control group, indicating a significant therapeutic effect.

Example 5-1:
I. A fluorinated chitosan-chicken ovalbumin composite was prepared with perfluoroheptanoic acid-modified chitosan as a carrier, and incubated with bone marrow-derived dendritic cells, and the ability of the composite to stimulate the maturation of dendritic cells was investigated.

Specific method:
1. Preparation of a perfluoroheptanoic acid-modified chitosan-chicken ovalbumin composite: 0.9 mg of perfluoroheptanoic acid-modified chitosan was weighed and dissolved in 900 µL of ultrapure water under stirring. Under constant stirring, 100 µL (20 mg/mL) of chicken ovalbumin was added dropwise, and continuously stirred for one hour to obtain a perfluoroheptanoic acid-modified chitosan-chicken ovalbumin (FCS-OVA) composite.
2. 10 µL of the above-prepared composite was added to a 24-well plate, and 1 mL of a cell suspension containing 1 million dendritic cells was added. They were incubated for 24 h in a 37°C incubator, the dendritic cells were stained with FITC-CD11c, PE-CD86 and APC-CD80, the fluorescence signal of FITC was analyzed with flow cytometry, the dendritic cells were selected and then the fluorescence signals of PE and APC in the dendritic cells were analyzed, and the results of cell maturity were determined, as shown in FIG. 1-1.

Experimental results: As shown in FIG. 5-1, the perfluoroheptanoic acid-modified chitosan-chicken ovalbumin (FCS-OVA) composite can stimulate the maturation of dendritic cells, compared with the group without treatment.

II. A perfluoroheptanoic acid-modified chitosan-chicken ovalbumin composite was prepared with perfluoroheptanoic acid-modified chitosan as a carrier, and incubated with bone marrow-derived dendritic cells, and how the composite stimulates dendritic cells to express histocompatibility complex class II (MHC II) and CD40 protein was investigated.

Specific method:
1. Preparation of a perfluoroheptanoic acid-modified chitosan-chicken ovalbumin composite: 0.9 mg of perfluoroheptanoic acid-modified chitosan was weighed and dissolved in 900 µL of ultrapure water with stirring. Under constant stirring, 100 µL (20 mg/mL) of chicken ovalbumin was added dropwise, and continuously stirred for one hour to obtain a perfluoroheptanoic acid-modified chitosan-chicken ovalbumin (FCS-OVA) composite.
2. 10 µL of the above-prepared composite was added to a 24-well plate, and 1 mL of a cell suspension containing 1 million dendritic cells was added. They were incubated for 24 h in a 37°C incubator, the dendritic cells were stained with FITC-CD11c, PE-MHC II and APC-CD40, the fluorescence signal of FITC was analyzed with flow cytometry, the dendritic cells were selected and then the fluorescence signals of PE and APC in the dendritic cells were analyzed, and the changes of histocompatibility complex class II (MHC II) and CD40 protein were determined. The results are shown in FIG. 5-2.

Experimental results: As shown in FIG. 5-2, under the stimulation of the perfluoroheptanoic acid-modified chitosan-chicken ovalbumin (FCS-OVA) composite, the expression levels of histocompatibility composite class II (MHC II) and CD40 protein in the dendritic cells increase significantly, indicating that the composite can effectively stimulate dendritic cells to present antigens.

III. A perfluoroheptanoic acid-modified chitosan-chicken ovalbumin composite was prepared with perfluoroheptanoic acid-modified chitosan as a carrier, and applied to the back of mice as patches, and the accumulation of the composite in the lymph nodes was investigated.

Specific method:
1. Preparation of cy5.5-labeled chicken ovalbumin: 10 mg of chicken ovalbumin was weighed, and dissolved in 1 mL of PBS solution, and 20 µL (20 mg/mL) cy5.5 was added and placed at 4°C overnight. Free cy5.5 was removed through G25-molecular sieve gel. Quantification was performed by BSA, and OVA was concentrated by ultrafiltration to 20 mg/mL.
2. Preparation of a perfluoroheptanoic acid-modified chitosan-chicken ovalbumin composite: 1 mg of perfluoroheptanoic acid-modified chitosan was weighed and dissolved in 450 µL PBS with stirring. Under constant stirring, 50 µL(20 mg/mL) PBS dissolved with cy5.5-labeled chicken ovalbumin was added dropwise, and continuously stirred for one hour to obtain a perfluoroheptanoic acid-modified chitosan-chicken ovalbumin (FCS-OVA-cy5.5) composite.
3. 12.5 µL of the above FCS-OVA-cy5.5 composite solution was mixed into 12.5 µg of an ointment to prepare an FCS-OVA-cy5.5 vaccine ointment, and the above ointment was applied to the back of C57 mice. Using small animal imaging, fluorescence distribution on the front of mice was photographed at 0h, 3h, 7h, 11h, 24h, and the aggregation of FCS-OVA-cy5.5 in the lymph nodes was observed. The results are shown in FIG. 5-3.
4. Experimental results: As shown in FIG. 5-3, fluorescence appears at 11 h and 24 h in the lymph nodes of the mice, which means that the perfluoroheptanoic acid-modified chitosan-chicken ovalbumin composite fixed on the back of the mice as patches can penetrate the skin into the lymph nodes of the mice.

IV. A perfluoroheptanoic acid-modified chitosan-chicken ovalbumin composite was prepared with perfluoroheptanoic acid-modified chitosan as a carrier, and applied to the skin with a patch as a vaccine implant, and three weeks later, B16-OVA tumor was implanted and the tumor growth was observed.

Specific method:
1. Preparation of a perfluoroheptanoic acid-modified chitosan-chicken ovalbumin composite: 1 mg of perfluoroheptanoic acid-modified chitosan was weighed and dissolved in 450 µL PBS with stirring. Under constant stirring, 50 µL (20 mg/mL) PBS dissolved with chicken ovalbumin was added dropwise, and continuously stirred for one hour to obtain a perfluoroheptanoic acid-modified chitosan-chicken ovalbumin (FCS-OVA) composite.
2. 12.5 µL of the above FCS-OVA composite solution was mixed into 12.5 µg of an ointment to prepare an FCS-OVA vaccine ointment, and 12.5 µL of the OVA (2 mg/mL) solution was mixed into 12.5 µg of an ointment to prepare an OVA vaccine ointment. The above ointments were separately applied to the back of C57 mice, and fixed with patches for 12 h. This operation was repeated twice a week for three weeks.
3. Each mouse was implanted with 1×10⁵ B16-OVA cells and the tumor growth was observed. The results are shown in FIG. 5-4.

Experimental results: As shown in FIG. 5-4, the tumor growth rate of mice implanted with perfluoroheptanoic acid-modified chitosan-chicken ovalbumin composite as a patch vaccine is slower than that of mice implanted with chicken ovalbumin alone, indicating that the perfluoroheptanoic acid-modified chitosan-chicken ovalbumin composite as a patch has the effect of being a vaccine.

The fluorinated chitosan in each of Examples 1-1 to 1-4 of the present invention can be used as a vaccine preparation, and is useful as various vaccination preparations.

Example 6-1: Taking the currently known small molecule drug UK5099 with a certain hair growth therapeutic effect as an example, fluorinated chitosan was used to encapsulate the drug, and the *in vitro* transdermal ability of perfluoroheptanoic acid-modified chitosan and the drug in different ratios was studied. Taking the small molecule drug metformin as an example, fluorinated chitosan was used to encapsulate the drug, and the actual hair growth effect in animals was studied.

Specific method:
1. In order to observe the membrane penetration effect of different proportions of perfluoroheptanoic acid-modified chitosan, an *in vitro* diffusion experiment was performed. The back skin of mice was removed of fat, and fixed on the Franz diffusion cell. The ratios of perfluoroheptanoic acid-modified chitosan to UK5099 were 1:1, 2.5:1, 5:1, and 10:1. In each group, samples were taken at 5 min, 30 min, 1 h, 2 h, 3 h, 4 h, 7 h, 10 h, and 24 h after drug application. The concentrations of UK5099 in the recipients at different time points were detected, the cumulative penetration percent was calculated, and the transmembrane effect was compared. The experimental results are shown in FIG. 6-1 and FIG. 6-2. Finally, the ratio of protein and perfluoroheptanoic acid-modified chitosan of 1:1 was selected for a series of animal experiments.
2. Preparation of a perfluoroheptanoic acid-modified chitosan-metformin hair growth liquid: first, perfluoroheptanoic acid-modified chitosan solid powder was dissolved, and a liquid dissolved with metformin was added dropwise under constant stirring, so that the final metformin concentration was 2 mg/mL, and the concentration of perfluoroheptanoic acid-modified chitosan was 2 mg/mL.
3. Balb/c mice at week 6 in the hair prohibition period were selected, and on the day before treatment, the mice in the perfluoroheptanoic acid-modified chitosan-metformin group, free metformin group, and blank control group were removed of the equivalent hair on the back and photographed as shown in the figure. Each mouse was uniformly sprayed with the drug according to its own treatment method, and the drug was given once every two days, 100 microliters each time. The photos on day 11, day 13, and day 17 after treatment are shown in FIG. 6-3 and Table 6-1.

Experimental results: It can be seen from the figure that although there are individual differences in mice, compared with the free drug group and the control group, the mice in the group with perfluoroheptanoic acid-modified chitosan have an obvious hair growth trend.

**Table 6-1**

| | Fluorinated chitosan-metformin group | | Metformin group | | Control group | |
|---|---|---|---|---|---|---|
| Day 0 | No hair | No hair | No hair | No hair | No hair | No hair |
| Day 11 | No hair | Slightly hairy | No hair | No hair | No hair | No hair |
| Day 13 | Slightly hairy | Obviously | No hair | No hair | No hair | No hair |
| Day 17 | Obviously | Obviously | No hair | No hair | No hair | No hair |

Example 6-2: A scar cream with perfluoroheptanoic acid-modified chitosan (FCS) and polyinosinic polycytidylic acid (poly (I:C)) as the main body was prepared, and the production of endogenous retinoic acid was induced, thereby improving scars with retinoic acid.

Specific method:
1. Preparation of a perfluoroheptanoic acid-modified chitosan-insulin mixture: perfluoroheptanoic acid-modified chitosan and poly (I: C) were separately dissolved in ultrapure water until uniformly dissolved. After both was mixed, the perfluoroheptanoic acid-modified chitosan and insulin bound together due to electrostatic adsorption, where they were mixed in a weight ratio of 1:1 to form stable nanoparticles.
2. Feasibility analysis on scar removal effect in animal experiments through an *in vitro* retinoic acid stimulation experiment with L929 (mouse fibroblast): L929 was plated in a 6-well plate for cell culture, and the medium volume per well was set to 3 mL. Poly(I:C), perfluoroheptanoic acid-modified chitosan and a mixture of poly(I:C) and perfluoroheptanoic acid-modified chitosan were added to the medium, so that the final concentrations of both poly(I:C) and perfluoroheptanoic acid-modified chitosan were 1 µg/mL. After L929 was cultured for 48 h, the culture medium and the cells were collected, and the supernatant was collected by centrifugation. The supernatant was uniformly mixed with a mixture of diethyl ether and acetone (ether: acetone = 1:8) in a ratio of 1:5, and the upper oil phase was collected. The oil phase was concentrated with nitrogen, and then HPLC (High Performance Liquid Chromatography) was used to detect the content of retinoic acid secreted by the cells.
3. Preparation of a scar cream of perfluoroheptanoic acid-modified chitosan and poly(I:C): poly(I:C) and perfluoroheptanoic acid-modified chitosan were pre-mixed at a ratio of 1:1, and then uniformly mixed with an equal volume of Aquaphor^{@} ointment to obtain a scar cream of perfluoroheptanoic acid-modified chitosan and poly(I:C).
4. Pre-judgment of scar removal effect of the perfluoroheptanoic acid-modified chitosan and poly(I:C) scar cream by morphological changes of mouse scars: Female Balb/c mice aged 10-12 weeks were anesthetized with isoflurane, 3 mm × 10 mm of the skin was cut off on the back and applied with iodophor, an open wound was made, the skin of the mice was healed with the scabs removed within one week, and the scar removal experiment was started two months later. The perfluoroheptanoic acid-modified chitosan and poly(I:C) scar cream were applied on the scars every day, and the morphology of the mouse scars was observed and evaluated on a weekly basis.

Experimental results: see FIG. 6-4. The left panel shows the efficiency of a mixture of perfluoroheptanoic acid-modified chitosan and poly(I:C) for the production of retinoic acid in mouse fibroblasts (the ordinate Mass of RA means the production efficiency of retinoic acid). The right panel shows the effect of the mixture of perfluoroheptanoic acid-modified chitosan and poly(I:C) when applied to the scars of mice.

As shown in FIG. 6-4, by HPLC detection and analysis, it is found that compared with the blank group, perfluoroheptanoic acid-modified chitosan alone and poly(I:C) alone, the mixture of perfluoroheptanoic acid-modified chitosan and poly(I:C) can significantly increase the production of retinoic acid in mouse fibroblasts *in vitro.* After 7 days of smearing, the scars of the mice are obviously smoothed. The above results collectively indicate that the delivery of poly(I:C) with perfluoroheptanoic acid-modified chitosan as a carrier can successfully achieve the production of retinoic acid in cells *in vitro,* and has obvious effects in living scar models and possesses transformational value.

Example 6-3: Taking tranexamic acid, a drug for treating melasma as an example, fluorinated chitosan was used to encapsulate tranexamic acid, and its transdermal ability was explored using a diffusion cell.

Specific method:
1. Preparation of a perfluoroheptanoic acid-modified chitosan-tranexamic acid solution: perfluoroheptanoic acid-modified chitosan solid powder was dissolved in PBS, and mixed with tranexamic acid dissolved in PBS at a ratio of 1: 1, and vortexed and shaken for 5 min, so that both were bound together by an electrostatic force. The final concentrations of perfluoroheptanoic acid-modified chitosan and tranexamic acid in the final solution were both 1 mg/mL.
2. *In vitro* diffusion cell construction: the abdomen skin of mice was removed, the fat layer was shaved off, and the skin was fixed on a diffusion cell. 7.5 mL PBS was added to the diffusion cell below the skin, and 0.75 mL perfluoroheptanoic acid-modified chitosan-tranexamic acid and the same concentration of pure tranexamic acid solution were respectively added to the diffusion cell above the skin.
3. Measurement of tranexamic acid penetration rate: 500 µL of PBS was removed from the bottom of the diffusion cell at 0, 1, and 3 h and the same volume of PBS was supplemented. The content of tranexamic acid was detected under the following HPLC conditions: methanol and 0.05 mol/L KH₂PO₄-0. 2% H₃PO₄ solution (volume ratio 5 : 95) as mobile phase, flow rate 1.0 mL/min, and detection wavelength 210 nm, and the cumulative penetration was calculated. The results are shown in the figure. The abscissa is time and the ordinate means the cumulative penetration.

Experimental results: As shown in FIG. 6-5, in the first three hours, tranexamic acid mixed with perfluoroheptanoic acid-modified chitosan can reach a cumulative penetration of 30%, while pure tranexamic acid substantially does not penetrate. It is proved that the perfluoroheptanoic acid-modified chitosan - tranexamic acid has better skin penetration ability and can be used for the treatment of skin disease melasma.

The above description of the disclosed examples enables those skilled in the art to implement or use the present invention. Various modifications to these examples will be apparent to those skilled in the art. The present invention will not be limited to the examples shown herein, but should conform to the widest scope consistent with the principles and features disclosed herein.

## Claims

1. A fluorinated chitosan derivative for use as a drug carrier, having the following structure: a fluorine-containing compound is covalently attached to the backbone of chitosan, wherein the chitosan has a molecular weight in the range of 1000-5000000, a degree of deacetylation of not less than 55%, and a viscosity in the range of 25-1000 cps; and the fluorine-containing compound is a fluorine-containing aliphatic chain shown by the following chemical formula (I) 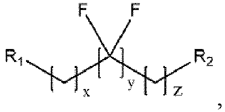 or an aromatic ring with functional groups shown by the following formula (II) 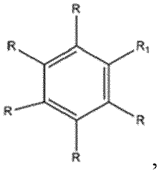 wherein Ri is an active group capable of reacting with a primary amino group, selected from halogen (fluorine, chlorine, bromine, iodine), a halogen-substituted alkane, cycloalkane, aldehyde group, carboxyl group, alkenyl group, alkynylgroup, hydroxyl group, sulfonyl chloride, sulfonic acid bond or mercapto group.

2. A fluorinated chitosan derivative for use as a drug carrier, having a molecular skeleton of chitosan which contains a primary amino group, as shown in formula (IV): 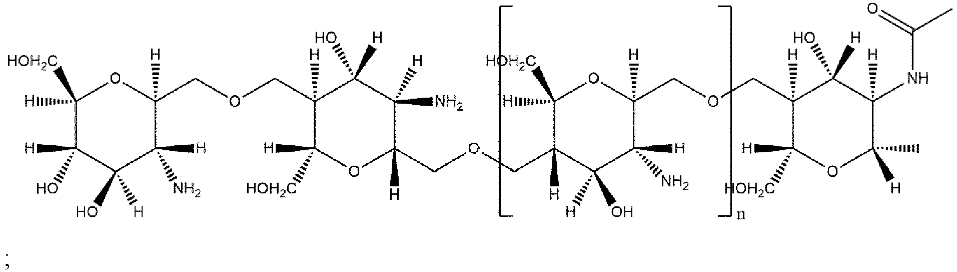 wherein a linking group formed between the primary amino group of the chitosan and a fluorine-containing functional group is: -NH-, -N=C-, -NHCH₂CH(OH)-, - NHCH₂CH(OH)CH₂O-, 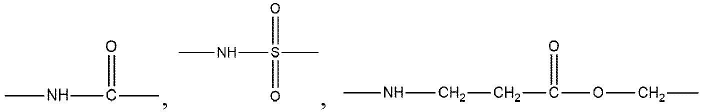 and a derivative group thereof; the chitosan has a molecular weight in the range of 1000-5000000, a degree of deacetylation of not less than 55%, and a viscosity in the range of 25-1000 cps;
the fluorine-containing functional group is a fluorine-containing aliphatic chain or an aromatic ring with functional groups.

3. The fluorinated chitosan derivative for use as a drug carrier according to claim 1, wherein: in the formula (I), x is an integer of 0-3, y is an integer of 0-20, z is an integer of 0-8, and R₂ is CF₃, CHF₂, CH₂F, or CH₃ (when y is not 0);
the fluorine-containing aliphatic chain compound refers to a fluorine-containing hydrocarbon compound and derivatives thereof, comprising trifluoroacetic acid, pentafluoropropionic acid, heptafluorobutyric acid, nonafluorovaleric acid, undecafluorohexanoic acid, tridecafluoroheptanoic acid, pentadecafluorooctanoic acid, heptadecafluorononanoic acid, nonadecafluorodecanoic acid, heptafluorobutyric anhydride, perfluoroheptanoic anhydride, nonadecafluorodecanoic anhydride, 2,2,3,3,4,4,4-heptafluorobutyl acrylate, 3-(1H, 1H, 5H octafluoropentyloxy)-1,2-epoxypropane, nonafluorobuanesulphonic anhydride and derivatives thereof.

4. The fluorinated chitosan derivative for use as a drug carrier according to claim 1, wherein in the formula (II), R is H, CH₃, OH, NO₂, O, CF₃, F, CH₂OH, CN, NCO, or (CF₂)ₐCF₃ (a is an integer of 1-20), or the like, and at least one R is F;
the fluorine-containing aromatic ring compound comprises 3-fluorobenzoic acid, 3,5-difluorobenzoic acid, 2,3,5,6-tetrafluoro-4-methylbenzoic acid, pentafluorobenzoic acid, 2-fluoro-3-(trifluoromethyl)benzoic acid and derivatives thereof.

5. The fluorinated chitosan derivative for use as a drug carrier according to claim 1, wherein: the chitosan and the fluorine-containing compound are covalently linked and the chitosan is surface-modified, to form a drug carrier having a structure as shown in formula (V) below, wherein b and c are both an integer of 20-500: 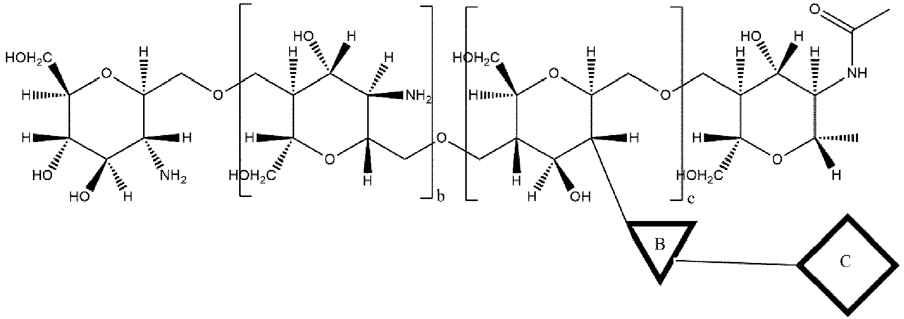 wherein B is a linking group comprising a fluorine-containing functional group and a primary amino group of the chitosan, and C is a fluorine-containing aliphatic chain or an aromatic ring of functional groups.

6. The fluorinated chitosan derivative for use as a drug carrier according to claim 1, wherein:
the fluorine-containing aliphatic chain is a class of fluorine-containing compounds with an active group capable of reacting with an amino group, and comprises those as shown in formula (VI): 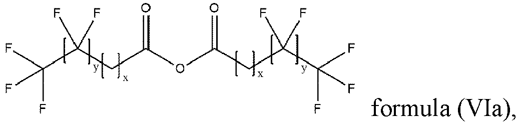 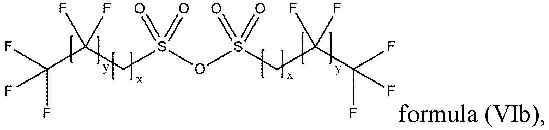 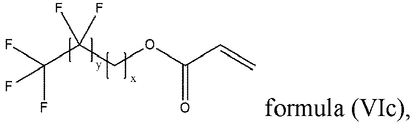 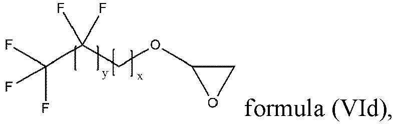 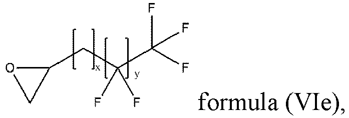 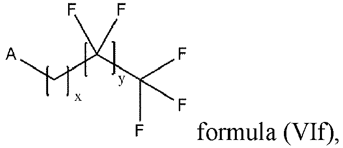 wherein A is -COOH or 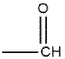 as an active group capable of reacting with a primary amino group, x is an integer of 0-3, and y is an integer of 0-8.

7. The fluorinated chitosan derivative for use as a drug carrier according to claim 1, wherein: the fluorine-containing aromatic ring compound is a class of fluorine-containing compounds with an active group capable of reacting with an amino group, and comprises those as shown in formula (VII):

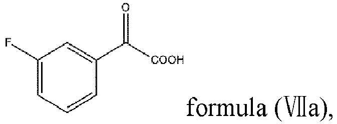

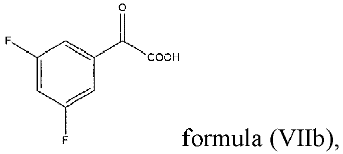

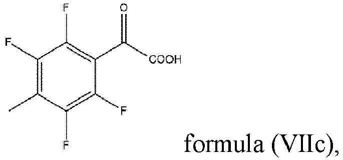

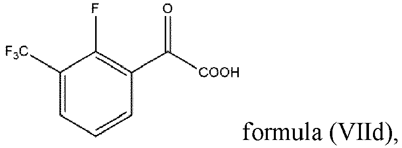

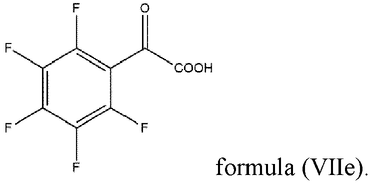

8. The fluorinated chitosan derivative for use as a drug carrier according to any one of claims 1 to 7, wherein: the fluorinated chitosan derivative serves as a drug carrier of a drug, and the drug is selected from a small molecule drug, a polypeptide, a protein drug, a combined drug of different drugs, and a combined drug of a drug and other pharmaceutical excipients.

9. Use of a fluorine-containing compound-modified chitosan as a drug carrier, wherein: the fluorinated chitosan derivative of any one of claims 1 to 7 is used as a drug carrier of a small molecule drug, a polypeptide, a protein drug, a combined drug of different drugs, and a combined drug of a drug and other pharmaceutical excipients.

10. A drug composite, comprising the fluorinated chitosan derivative for use as a drug carrier according to any one of claims 1 to 7 and a drug, wherein the drug is selected from a small molecule drug, a polypeptide, a protein drug, a combined drug of different drugs, and a combined drug of a drug and other pharmaceutical excipients.

11. A transdermal administration preparation prepared from the fluorinated chitosan derivative for use as a drug carrier according to any one of claims 1 to 7, comprising a transdermal preparation component (a), wherein the component (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps.

12. A transmucosal administration preparation prepared from the fluorinated chitosan derivative for use as a drug carrier according to any one of claims 1 to 7, comprising a transmucosal preparation component (a), wherein the component (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps; the mucosa comprises nasal mucosa, lung mucosa, vaginal mucosa, oral mucosa, and gastrointestinal mucosa.

13. An ocular barrier-penetrating administration preparation prepared from a fluorinated chitosan derivative for use as a drug carrier according to any one of claims 1 to 7, comprising an ocular barrier-penetrating preparation component (a), wherein the component (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps; the ocular barrier is a tear barrier, a corneal/conjunctival barrier, and a blood-aqueous barrier, or a blood-retinal barrier.

14. A transdermal vaccine carrier prepared from a fluorinated chitosan derivative for use as a drug carrier according to any one of claims 1 to 7, comprising a transdermal vaccine carrier (a), wherein the transdermal vaccine carrier (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps; the transdermal vaccine carrier has three antigen penetration pathways: transcellular penetration, paracellular penetration and transappendageal penetration.

15. A carrier for cosmetic and health care products prepared from a fluorinated chitosan derivative for use as a drug carrier according to any one of claims 1 to 7, comprising a carrier for cosmetic and health care products (a), wherein the carrier for cosmetic and health care products (a) is a fluorine-containing compound-modified cationic polymer; the fluorine-containing compound-modified cationic polymer is a fluorinated chitosan; the fluorine-containing compound is covalently attached to the backbone of the chitosan; the chitosan has a molecular weight in the range of 1000-5000000, a degree of deacetylation of not less than 55%, and a viscosity in the range of 25-1000 cps; the carrier for cosmetic and health care products is suitable for hair growth drugs and hair care drugs, cosmetic drugs, and health care drugs.
